# EUROPEAN PATENT APPLICATION

(11) **EP 3 875 458 A1**
(43) Date of publication of application: **08.09.2021**
(21) Application number: 19878365.6
(22) Date of filing: 25.10.2019
(51) Int. Cl.: C07D 471/04, A61P 35/00, A61K 31/437

(54) **DIPHENYL-LIKE COMPOUND, INTERMEDIATE THEREOF, PREPARATION METHOD THEREFOR, PHARMACEUTICAL COMPOSITION THEREOF AND USES THEREOF**

(30) Priority: 02.11.2018 CN 201811301041
(71) Applicant: Shanghai Maxinovel Pharmaceuticals Co., Ltd., Shanghai 201210 (CN)
(72) Inventor: WANG, Yuguang, Shanghai 201210 (CN); ZHANG, Nong, Shanghai 201210 (CN); WU, Tianzhi, Shanghai 201210 (CN); HE, Min, Shanghai 201210 (CN)
(74) Representative: Mewburn Ellis LLP
(86) International application number: PCT/CN2019/113209
(87) International publication number: WO 2020/088357

(57) **Abstract**

The present invention provides a diphenyl-like compound, and intermediate thereof, a preparation method therefor, a pharmaceutical composition thereof and the uses thereof. According to the invention, the diphenyl-like compound has a significant inhibitory effect on PD-1 and/or PD-L1, and can effectively alleviate or treat related diseases such as cancer.

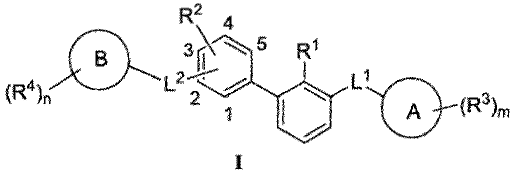

## Description

The present application claims the priority of Chinese patent application No. 201811301041.9 filed on November 2, 2018. The entirety of the above Chinese patent application is incorporated herein by reference.

### Technical Field

The present disclosure relates to a diphenyl-like compound, and intermediate thereof, a preparation method therefor, a pharmaceutical composition thereof and the uses thereof.

### Background

PD-1 (programmed death 1) programmed death receptor 1 is an important immunosuppressive molecule. It is a member of CD28 super family, and initially cloned from apoptotic mouse T cell hybridoma 2B4.11. Immunoregulation targeting PD-1 is of great significance in the anti-tumor, anti-infection, anti-autoimmune diseases and survival of organ transplantation. Its ligand PD-L1 can also be used as a target, and the corresponding antibody can also play the same role.

PD-1/PD-L1 plays a negative immunoregulatory role. When PD-1 on the surface of cell is coupled with PD-L1, phosphorylation of Tyr in the domain of Immunoreceptor Tyrosine-based Switch Motifs (ITSM) in the cytoplasmic region of T cell is caused, and then the phosphorylated Tyr can recruit phosphatase protein tyrosinase 2 and protein tyrosinase 1, which not only may retard the activation of extracellular signal-regulated kinase, but also may block the activation of phosphatidylinositol 3-kinase (PI3K) and serine-threonine protein kinase (Akt), ultimately inhibiting the proliferation of T lymphocyte and the secretion of the relevant cytokines. PD-1/PD-L1 signal may inhibit the activation and proliferation of T cell, meanwhile the secretion of cytokines interleukin 2 (IL2), interferon γ and IL-10 is reduced (Eur. J. Immunol., 2002, 32 (3), 634-643.). Additionally, the immune function of PD-1/PD-L1 signal on B cell is also similar to T cell. When PD-1 is crosslinked with B cell antigen receptor, the cytoplasmic region of PD-1 act on the tyrosinase with protein tyrosinase 2 binding site, ultimately retarding the activation of B cell. The role of negative immunoregulatory molecule PD-1/PD-L1 in tumor immune escape has attracted more and more attention. Many studies confirm that PD-L1 on the surface of tumor cell in tumor microenvironment is increased and meanwhile is binded with PD-1 on the activated T cell, which transmits negative regulatory signal and causes the apoptosis or immune anergy of tumor antigen specific T cell, thereby inhibiting immune response, in turn promoting the escape of tumor cell.

PD-1/PD-L1 antibody inhibitors currently appeared on the market comprise nivolumab from BMS (2014), lambrolizumab from Merck (2014) and atezolizumab from Roche (2016). PD-1/PD-L1 antibody inhibitors being studied comprise Pidilizumab from Cure Tech, AMP-224 from GSK and MEDI-4736 from AstraZeneca. All of the above are biomacromolecules, whereas small molecule inhibitors of PD-1/PD-L1 currently are in the early stage of research and development, peptide-like small molecule inhibitor AC-170 of PD-L1 from Curis (WO 2012168944, WO 2015033299, WO 2015033301, WO 2015036927, WO 2015044900) is just entering clinical stage I, benzyl phenyl ether small molecule inhibitor of PD-1/PD-L1 from BMS (WO 2015034820, WO 2015160641, WO 2017066227, WO 2018009505, WO 2018044963, WO 2018118848) is still in the pre-clinical study stage, a series of small molecule inhibitors of PD-1/PD-L1 made by Incyte (WO 2017070089, WO 2017087777, WO 2017106634, WO 2017112730, WO 2017192961, WO 2017205464, WO 2017222976, WO 2018013789, WO 2018044783, WO 2018119221, WO 2018119224, WO 2018119263, WO 2018219266, WO 2018119286) are still in the pre-clinical study. Compared with biomacromolecules, small molecule compounds are capable of crossing the cell membrane and acting on intracellular targets, thus having a wide range of applications. Secondly, small molecules after chemically modified generally have good bioavailability and compliance, effectively avoiding the decomposition inactivation by enzymes in the digestive tract. Finally, studies on small molecules are quite mature in many aspects, such as production process, design of dosage form and administration mode.

Currently in the prior art, diphenyl-like compounds have not been reported as small molecule inhibitors of PD-1/PD-L1 successfully appeared on the market, which situation urgently needs to be addressed.

### Content of the invention

An object of the present disclosure is to provide a diphenyl-like compound totally different from the prior art, and intermediate thereof, a preparation method therefor, a pharmaceutical composition thereof and the uses thereof. The diphenyl-like compound of the present disclosure has a significant inhibitory effect on PD-1 and/or PD-L1, and can effectively alleviate or treat the related diseases such as cancer.

The present disclosure provides a diphenyl-like compound of general formula I, a pharmaceutically acceptable salt, tautomer, mesomer, racemate, stereoisomer, or prodrug thereof: 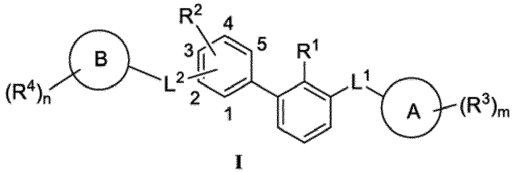 wherein,
ring A and ring B are independently aromatic ring or heteroaromatic ring;
L¹ and L² are independently chemical bond, alkynyl, -C(R⁵)=C(R⁶)- or -CR⁷R⁸-CR⁹R¹⁰-, substituted or unsubstituted cycloalkyl, substituted or unsubstituted heterocycloalkyl, substituted or unsubstituted aryl or substituted or unsubstituted heteroaryl;
R⁵, R⁶, R⁷, R⁸, R⁹ and R¹⁰ are independently hydrogen, deuterium, halogen, cyano, or substituted or unsubstituted alkyl respectively;
R¹ and R² are independently deuterium, halogen, cyano, or substituted or unsubstituted alkyl;
each R³ and each R⁴ are independently hydrogen, deuterium, hydroxyl, -SR¹¹, - NR¹²R¹³, halogen, cyano, substituted or unsubstituted alkyl, substituted or unsubstituted alkoxy, -CONH₂, -COR¹⁴, -COOR¹⁵ or -OCOR¹⁶; R¹¹, R¹² and R¹³ are independently hydrogen, C₁-C₄ alkyl, substituted C₁-C₄ alkyl or -COR^{a}, R^{a} is hydrogen, hydroxyl, C₁-C₄ alkyl or C₁-C₄ alkoxy;
R¹⁴, R¹⁵ and R¹⁶ are independently hydrogen, C₁-C₄ alkyl or substituted C₁-C₄ alkyl;
among R¹¹, R¹², R¹³, R¹⁴, R¹⁵ and R¹⁶, the substituted in the substituted C₁-C₄ alkyl refers to being substituted with one or more of C₆-C₁₄ aryl, substituted C₆-C₁₄ aryl, C₁-C₁₀ heteroaryl and substituted C₁-C₁₀ heteroaryl;
substituents in the substituted cycloalkyl, the substituted heterocycloalkyl, the substituted aryl, the substituted heteroaryl in L¹ and L², the substituted alkyl in R¹ and R², the substituted alkyl or the substituted alkoxy in each R³ and each R⁴ are selected from one or more of halogen, cyano, C₁-C₄ alkyl, hydroxyl, 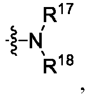 C₆-C₁₄ aryl, substituted C₆-C₁₄ aryl, C₁-C₁₀ heteroaryl, substituted C₁-C₁₀ heteroaryl, C₁-C₄ alkoxy, C₁-C₄ carboxyl, C₁-C₄ ester group and C₁-C₄ amido; in 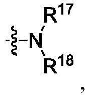 R¹⁷ and R¹⁸ are independently hydrogen, substituted or unsubstituted C₁-C₄ alkyl, substituted or unsubstituted C₆-C₁₄ aryl, substituted or unsubstituted C₃-C₆ cycloalkyl, or substituted or unsubstituted C₁-C₄ alkoxy; or R¹⁷, R¹⁸ together with the nitrogen atom to which they are attached form a substituted or unsubstituted 5 to 7-membered heterocycle; in the heterocycle, heteroatom is N, or N and O, the number of heteroatom is 1 to 4; each R¹⁷ and each R¹⁸ are identical or different;
substituents in the substituted C₁-C₄ alkyl, the substituted C₆-C₁₄ aryl, the substituted C₃-C₆ cycloalkyl, the substituted C₁-C₄ alkoxy and the substituted 5 to 7-membered heterocycle in R¹⁷ and R¹⁸ are selected from one or more of halogen, cyano, C₁-C₄ alkyl, substituted C₁-C₄ alkyl, C₆-C₁₄ aryl, substituted C₆-C₁₄ aryl, C₁-C₁₀heteroaryl, substituted C₁-C₁₀ heteroaryl, hydroxyl, 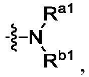 C₁-C₄ alkoxy, C₁-C₄ carboxyl, C₁-C₄ ester group and C₁-C₄ amido;
in R¹⁷ and R¹⁸, when substituents in the substituted C₁-C₄ alkyl, the substituted C₆-C₁₄ aryl, the substituted C₃-C₆ cycloalkyl, the substituted C₁-C₄ alkoxy and the substituted 5 to 7-membered heterocycle are substituted C₁-C₄ alkyl, in the substituents, substituents in the substituted C₁-C₄ alkyl are selected from one or more of halogen, cyano, C₁-C₄ alkyl, C₆-C₁₄ aryl, substituted C₆-C₁₄ aryl, C₁-C_{1 0}heteroaryl, substituted C₁-C₁₀ heteroaryl, hydroxyl, 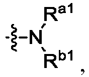 C₁-C₄ alkoxy, C₁-C₄ carboxyl, C₁-C₄ ester group and C₁-C₄ amido; in 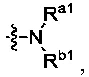 R^{a1} and R^{b1} are independently hydrogen, C₁-C₄ alkyl or 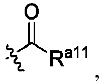 R^{a11} is C₁-C₄ alkyl;
all the above C₁-C₁₀ heteroaryl refer to C₁-C₁₀ heteroaryl in which heteroatom is selected from N, O and S and the number of heteroatom is 1 to 4;
substituents in all the above substituted C₆-C₁₄ aryl and substituted C₁-C₁₀ heteroaryl are selected from one or more of cyano, halogen, hydroxyl, C₁-C₄ alkyl and C₁-C₄ alkoxy;
when substituents are more than one, the substituents are identical or different;
m is 1, 2 or 3;
n is 1, 2 or 3.

In the present disclosure, all the term aromatic ring refers to any stable monocyclic or bicyclic carbocycle with up to 7 atoms in each ring, wherein at least one ring is aromatic ring. All the term aromatic ring is preferably C₆-C₂₀ aromatic ring, more preferably C₆-C₁₄ aromatic ring, most preferably C₆-C₁₀ aromatic ring. Examples of aromatic ring include but not limited to benzene, naphthalene, tetrahydronaphthalene, 2,3-dihydroindene, diphenyl, phenanthrene, anthracene or acenaphthene.

In the present disclosure, all the term heteroaromatic ring refers to stable monocyclic or bicyclic ring with up to 7 atoms in each ring, wherein at least one ring is aromatic ring and contains 1 to 4 heteroatoms selected from O, N and S. "Heteroaromatic ring" in the present disclosure preferably refers to C₁-C₁₀ heteroaromatic ring in which heteroatom is selected from O, N and S and the number of heteroatom is 1, 2, 3 or 4, further preferably C₁-C₈ heteroaromatic ring in which heteroatom is selected from O, N and S and the number of heteroatom is 1, 2, 3 or 4, more preferably C₁-C₆ heteroaromatic ring in which heteroatom is selected from O, N and S and the number of heteroatom is 1, 2, 3 or 4. Examples of heteroaromatic ring include but not limited to: acridine, carbazole, cinnoline, carboline, quinoxaline, imidazole, pyrazole, pyrrole, indole, indoline, benzotriazole, benzimidazole, furan, thiophene, isothiazole, benzothiophene, dihydrobenzothiophene, benzofuran, isobenzofuran, benzoxazole, benzofurazan, benzopyrazole, quinoline, isoindoline, isoquinoline, oxazole, oxadiazole, isoxazole, indole, pyrazine, pyridopyridine, tetrazolopyridine, pyridazine, pyridine, naphthyridine, pyrimidine, pyrrole, tetrazole, thiadiazole, thiazole, thiophene, triazole, quinazoline, tetrahydroquinoline, dihydrobenzimidazole, dihydrobenzofuran, dihydrobenzoxazole and dihydroquinoline.

In the present disclosure, all the term cycloalkyl is preferably C₃-C₂₀ cycloalkyl, more preferably C₃-C₁₀ cycloalkyl, most preferably C₃-C₆ cycloalkyl. Examples of cycloalkyl include but not limited to: cyclopropyl, cyclobutyl, cyclopentyl, cyclohexyl, cycloheptyl, cyclooctyl, cyclodecyl and cyclododecyl and cyclohexenyl.

In the present disclosure, all the term heterocycloalkyl refers to C₂-C₁₀ non-aromatic ring in which heteroatom is selected from O, N and S and the number of heteroatom is 1, 2, 3 or 4. In the present disclosure, heterocycloalkyl is preferably C₂-C₈ heterocycloalkyl in which heteroatom is selected from O, N and S and the number of heteroatom is 1, 2, 3 or 4, further preferably C₂-C₆ heterocycloalkyl in which heteroatom is selected from O, N and S and the number of heteroatom is 1, 2, 3 or 4. Examples of heteroalkyl include but not limited to: tetrahydropyranyl, azetidinyl, 1,4-dioxanyl, piperazinyl, piperidinyl, pyrrolidinyl, morpholinyl, thiomorpholinyl, dihydrofuryl, dihydroimidazolyl, indolinyl, dihydroisoxazolyl, dihydroisothiazolyl, dihydrooxadiazolyl, dihydrooxazolyl, dihydropyrazinyl, dihydropyrazolyl, dihydropyridyl, dihydropyrimidinyl, dihydropyrrolyl, dihydroquinolyl, dihydrotetrazolyl, dihydrothiadiazolyl, dihydrothiazolyl, dihydrothiophenyl, dihydrotriazolyl, dihydroazetidinyl, methylenedioxybenzoyl, tetrahydrofuryl, tetrahydrothiophenyl and N-oxides thereof.

In the present disclosure, all the term aryl is preferably C₆-C₂₀ aryl, more preferably C₆-C₁₄ aryl, most preferably C₆-C₁₀ aryl. Examples of aryl include but not limited to phenyl, naphthyl, tetrahydronaphthyl, 2,3-dihydroindenyl, xenyl, phenanthryl, anthryl and acenaphthyl.

In the present disclosure, all the term heteroaryl is preferably C₁-C₁₀ heteroaryl in which heteroatom is selected from O, N and S and the number of heteroatom is 1, 2, 3 or 4, further preferably C₁-C₈ heteroaryl in which heteroatom is selected from O, N and S and the number of heteroatom is 1, 2, 3 or 4, more preferably C₁-C₆ heteroaryl in which heteroatom is selected from O, N and S and the number of heteroatom is 1, 2, 3 or 4. Examples of heteroaryl include but not limited to benzimidazolyl, benzofuryl, benzofurazanyl, benzopyrazolyl, benzotriazolyl, benzothiophenyl, benzoxazolyl, carbazolyl, carbolinyl, cinnolinyl, furyl, imidazolyl, indolinyl, indolyl, indazolyl, isobenzofuryl, isoindolinyl, isoquinolyl, isothiazolyl, isooxazolyl, naphthyridinyl, oxadiazolyl, oxazolyl, oxazoline, isoxazoline, oxetanyl, pyranyl, pyrazinyl, pyrazolyl, pyridazinyl, pyridopyridyl, pyridazinyl, pyridyl, pyrimidinyl, pyrrolyl, quinazolyl, quinolyl, quinoxalinyl, tetrazolyl, tetrazolopyridyl, thiadiazolyl, thiazolyl, thiophenyl and triazolyl.

In the present disclosure, all the term halogen is preferably fluorine, chlorine, bromine or iodine.

In the present disclosure, all the term alkyl comprises branched and linear saturated aliphatic hydrocarbonyl of 1 to 20 carbon atoms, preferably 1 to 10 carbon atoms, more preferably 1 to 8 carbon atoms. Examples of alkyl include but not limited to methyl, ethyl, n-propyl, isopropyl, n-butyl, tert-butyl, isobutyl, pentyl, hexyl, heptyl, octyl, nonyl, decyl, 4,4-dimethylpentyl, 2,2,4-trimethylpentyl, undecyl, dodecyl and various isomers thereof. Alkyl in the present disclosure is preferably C₁-C₄ alkyl, more preferably methyl, ethyl, n-propyl, isopropyl, n-butyl, isobutyl or tert-butyl.

In the present disclosure, all the term alkoxy represents cyclic or non-cyclic alkyl linked via oxygen bridge with the recited carbon atom number. Thus, alkoxy comprises the definitions of the above alkyl and cycloalkyl. Alkoxy in the present disclosure is preferably C₁-C₄ alkoxy, more preferably methoxy, ethoxy, n-propoxy, isopropoxy or tert-butoxy.

In the present disclosure, all the term 5 to 7-membered heterocycle refers to 5 to 7-membered heterocycle in which heteroatom is selected from O, N and S, the number of heteroatom is 1, 2, 3 or 4 and the number of carbon atom is 1, 2, 3, 4, 5 or 6. The number of ring atoms in the 5 to 7-membered heterocycle is 5, 6 or 7. In the present disclosure, examples of the 5 to 7-membered heterocycle include but not limited to: azetidinyl, piperazinyl, piperidinyl, pyrrolidinyl, morpholinyl, thiomorpholinyl, dihydroimidazolyl, indolinyl, dihydroisoxazolyl, dihydroisothiazolyl, dihydrooxadiazolyl, dihydrooxazolyl, dihydropyrazinyl, dihydropyrazolyl, dihydropyridyl, dihydropyrimidinyl, dihydropyrrolyl, dihydroquinolyl, dihydrotetrazolyl, dihydrothiadiazolyl, dihydrothiazolyl, dihydrotriazolyl and dihydroazetidinyl.

In a preferred embodiment, L¹ is alkynyl, -C(R⁵)=C(R⁶)-, -CR⁷R⁸-CR⁹R¹⁰-, substituted or unsubstituted cycloalkyl, substituted or unsubstituted heterocycloalkyl, substituted or unsubstituted aryl, or substituted or unsubstituted heteroaryl, preferably alkynyl, -C(R⁵)=C(R⁶)- or -CR⁷R⁸-CR⁹R¹⁰-, more preferably -C(R⁵)=C(R⁶)-, most preferably -CH=CH-.

In a preferred embodiment, L² is alkynyl, -C(R⁵)=C(R⁶)-, -CR⁷R⁸-CR⁹R¹⁰-, substituted or unsubstituted cycloalkyl, substituted or unsubstituted heterocycloalkyl, substituted or unsubstituted aryl, or substituted or unsubstituted heteroaryl, preferably alkynyl, -C(R⁵)=C(R⁶)- or -CR⁷R⁸-CR⁹R¹⁰-, more preferably -C(R⁵)=C(R⁶)-, most preferably -CH=CH-.

In a preferred embodiment, L² is absent.

In a preferred embodiment, R⁵, R⁶, R⁷, R⁸, R⁹ and R¹⁰ are independently hydrogen or deuterium respectively.

In a preferred embodiment, R¹ is halogen, for example, F, Cl, Br or I.

In a preferred embodiment, R¹ is cyano.

In a preferred embodiment, R¹ is alkyl, preferably C₁-C₄ alkyl, more preferably methyl.

In a preferred embodiment, R¹ is substituted alkyl. Substituents in the substituted alkyl are preferably halogen or hydroxy. R¹ is preferably alkyl substituted with halogen, the alkyl substituted with halogen is preferably C₁-C₄ alkyl substituted with one or more of F, Cl, Br and I, more preferably -CH₂F, -CHF₂ or -CF₃.

In a preferred embodiment, R² is deuterium.

In a preferred embodiment, R² is halogen, for example, F, Cl, Br or I.

In a preferred embodiment, R² is cyano.

In a preferred embodiment, R² is alkyl, preferably C₁-C₄ alkyl, more preferably methyl, ethyl, n-propyl, isopropyl, n-butyl, isobutyl or tert-butyl.

In a preferred embodiment, R² is substituted alkyl, preferably substituted C₁-C₄ alkyl. Substituents in the substituted alkyl are preferably one or more of halogen, cyano, C₁-C₄ alkyl, hydroxyl, C₁-C₄ alkoxy, C₁-C₄ carboxyl, C₁-C₄ ester group and C₁-C₄ amido, when substituents are more than one, the substituents are identical or different. The alkyl substituted with halogen is preferably C₁-C₄ alkyl substituted with one or more of F, Cl, Br and I, more preferably -CH₂F, -CHF₂ or -CF₃.

In a preferred embodiment, R² is at position 1 of benzene ring.

In a preferred embodiment, R³ and R⁴ preferably are independently deuterium, halogen, cyano, -SR¹¹, -NR¹²R¹³, substituted or unsubstituted alkyl, or substituted or unsubstituted alkoxy.

In a preferred embodiment, R³ and R⁴ preferably are independently deuterium, halogen, cyano, -SR¹¹, substituted or unsubstituted alkyl, or substituted or unsubstituted alkoxy.

In a preferred embodiment, R³ and R⁴ preferably are -SR¹¹, R¹¹ is substituted C₁-C₄ alkyl.

In a preferred embodiment, R³ and R⁴ preferably are halogen.

In a preferred embodiment, R³ and R⁴ preferably are substituted or unsubstituted alkyl. Substituents in the substituted alkyl are preferably substituted with one or more of halogen, cyano, hydroxyl, 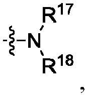 C₆-C₁₄ aryl, substituted C₆-C₁₄ aryl, C₁-C₁₀ heteroaryl, substituted C₁-C₁₀heteroaryl, C₁-C₄ alkoxy and C₁-C₄ carboxyl. When substituents are more than one, the substituents are identical or different.

In a preferred embodiment, R³ and R⁴ preferably are substituted alkyl. Substituents in the substituted alkyl are preferably substituted with one or more of halogen, 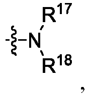 substituted C₆-C₁₄ aryl and substituted C₁-C₁₀ heteroaryl. When substituents are more than one, the substituents are identical or different.

In a preferred embodiment, R³ and R⁴ preferably are alkyl substituted with halogen. The alkyl substituted with halogen is preferably C₁-C₄ alkyl substituted with one or more of F, Cl, Br and I, preferably -CF₃.

In a preferred embodiment, R³ and R⁴ preferably are alkyl substituted with 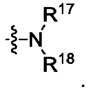 The alkyl substituted with 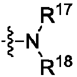 preferably is C₁-C₄ alkyl substituted with 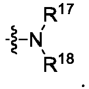 The C₁-C₄ alkyl substituted with 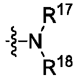 preferably is 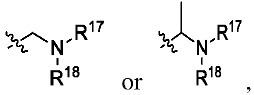 wherein one of R¹⁷ and R¹⁸ is H, the other one is alkyl substituted with hydroxy and/or carboxyl.

In a preferred embodiment, one of R¹⁷ and R¹⁸ is H, the other one is alkyl substituted with one or more of C₁-C₄ alkoxy, hydroxy and carboxyl.

In a preferred embodiment, when R³ and R⁴ are alkyl substituted with 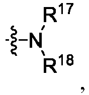 the alkyl substituted with 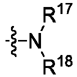 preferably is

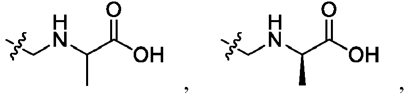

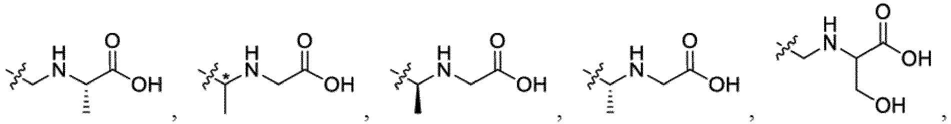

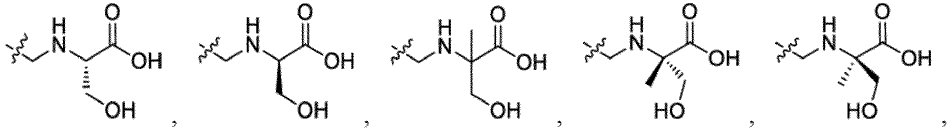

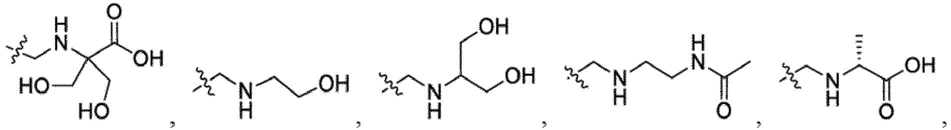

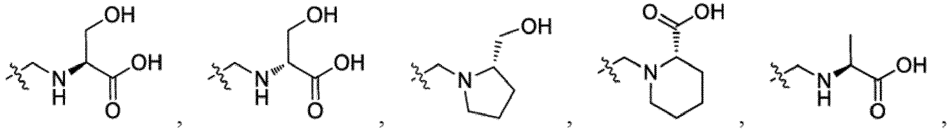

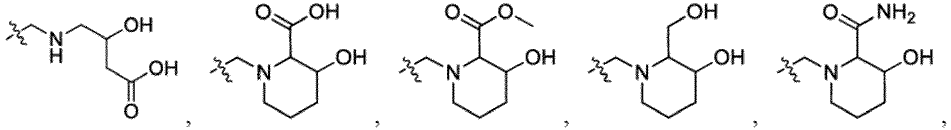

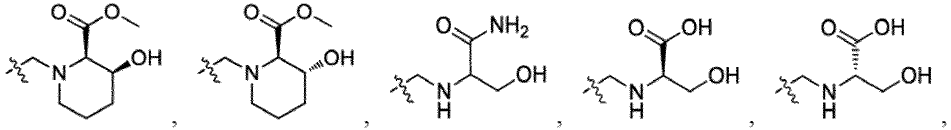

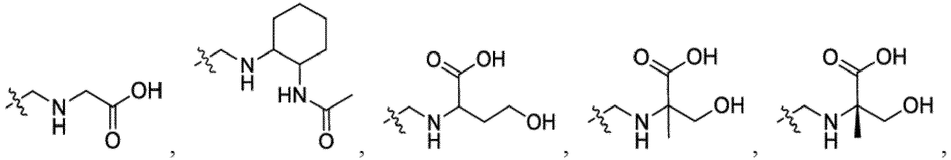

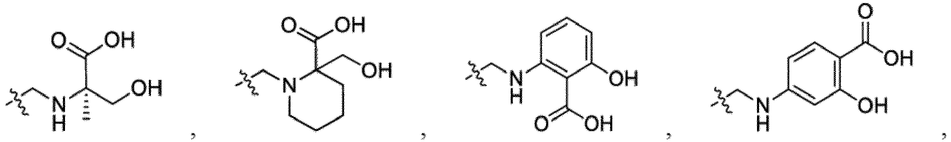

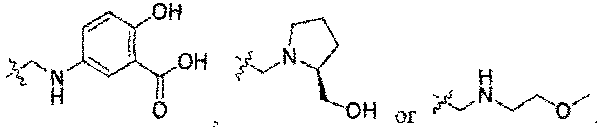

In a preferred embodiment, R³ or R⁴ preferably is alkyl substituted with substituted C₆-C₁₄ aryl, more preferably

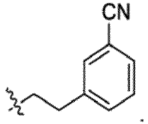

In a preferred embodiment, R³ or R⁴ preferably is alkyl substituted with substituted C₁-C₁₀heteroaryl, more preferably

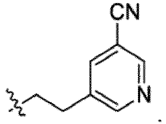

In a preferred embodiment, when R³ is substituted or unsubstituted alkyl (alkyl substituted with 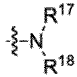 ), R³ is at meta-position or para-position of the atom linked with L¹ on ring A.

In a preferred embodiment, when R⁴ is substituted or unsubstituted alkyl (for example, alkyl substituted with 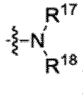 ), R⁴ is at meta-position or para-position of the atom linked with L² on ring B.

In a preferred embodiment, when R³ is substituted or unsubstituted alkyl (for example, alkyl substituted with 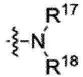 ), 0, 1 or 2 additional substituents can be present on ring A. When 1 additional substituent is present, the additional substituent is at para-position, meta-position or ortho-position of substituted or unsubstituted alkyl (for example, alkyl substituted with 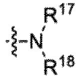 ).

In a preferred embodiment, when R⁴ is substituted or unsubstituted alkyl (for example, alkyl substituted with 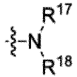 ), 0, 1 or 2 additional substituents can be present on ring B. When 1 additional substituent is present, the additional substituent is at para-position, meta-position or ortho-position of substituted or unsubstituted alkyl (for example, alkyl substituted with 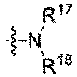 ).

In a preferred embodiment, R³ and R⁴ are substituted or unsubstituted alkoxy. Substituents in the substituted alkoxy are preferably substituted with one or more of halogen, cyano, hydroxyl, 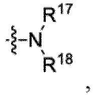 C₆-C₁₄ aryl, substituted C₆-C₁₄ aryl, C₁-C₁₀ heteroaryl and substituted C₁-C₁₀ heteroaryl. When substituents are more than one, the substituents are identical or different.

In a preferred embodiment, R³ and R⁴ are substituted or unsubstituted alkoxy. Substituents in the substituted alkoxy are preferably substituted with one or more of halogen, cyano, hydroxyl, 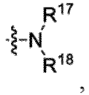 C₆-C₁₄ aryl, substituted C₆-C₁₄ aryl, C₁-C₁₀ heteroaryl, substituted C₁-C₁₀ heteroaryl and C₁-C₄ alkoxy. When substituents are more than one, the substituents are identical or different.

In a preferred embodiment, R³ and R⁴ are substituted alkoxy, substituents in the substituted alkoxy preferably are substituted with one or more of C₁-C₁₀ heteroaryl and substituted C₁-C₁₀ heteroaryl, when substituents are more than one, the substituents are identical or different. The substituted alkoxy preferably is

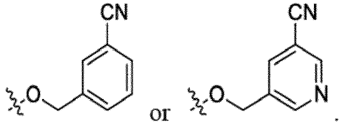

In a preferred embodiment, R³ and R⁴ preferably are substituted alkoxy, substituents in the substituted alkoxy preferably are substituted with C₁-C₄ alkoxy. The substituted alkoxy preferably is

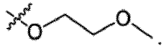

In a preferred embodiment, when R³ is substituted or unsubstituted alkoxy, R³ is at ortho-position or meta-position of the atom linked with L¹ on ring A.

In a preferred embodiment, when R⁴ is substituted or unsubstituted alkoxy, R⁴ is at ortho-position or meta-position of the atom linked with L² on ring B.

In a preferred embodiment, group 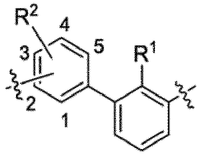 is preferably 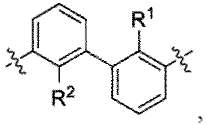 wherein, definitions of R¹ and R² are the same as before.

In a preferred embodiment, group 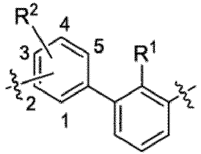 preferably is

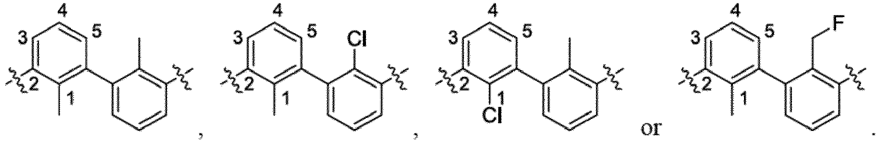

In a preferred embodiment, 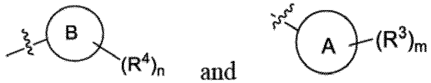 are independently 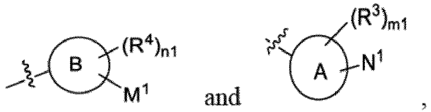 wherein M¹ and N¹ are alkyl substituted with 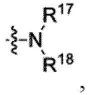 or one of M¹ and N¹ is alkyl substituted with 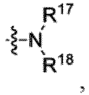 the other one is substituted alkoxy; wherein in M¹ and N¹, the definition of the alkyl substituted with 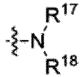 and the definition of alkoxy are the same as those of the corresponding groups in the above R³ and R⁴; the definitions of R¹⁷ , R¹⁸, R³ and R⁴ are the same as defined described previously, n1 and m1 are independently 0, 1 or 2.

Preferably, M¹ and N¹ are 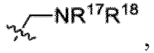 or one of M¹ and N¹ is 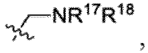 the other one is alkoxy substituted with one or more of C₁-C₄ alkoxy, C₁-C₁₀ heteroaryl and substituted C₁-C₁₀ heteroaryl; R³ and R⁴ preferably are hydrogen, halogen, alkyl, alkyl substituted with halogen, alkoxy or substituted alkoxy, substituents in the substituted alkoxy preferably are substituted with one or more of C₁-C₄ alkoxy, C₁-C₁₀ heteroaryl and substituted C₁-C₁₀ heteroaryl; the definitions of R¹⁷ and R¹⁸ are the same as defined described previously.

More preferably, M¹ and N¹ are 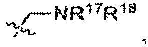 or one of M¹ and N¹ is 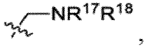 the other one is alkoxy substituted with C₁-C₄ alkoxy; R³ and R⁴ preferably are halogen, alkyl, alkyl substituted with halogen, alkoxy or alkoxy substituted with C₁-C₄ alkoxy; the definitions of R¹⁷ and R¹⁸ are the same as defined described previously.

In a preferred embodiment, 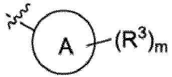 preferably is 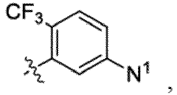 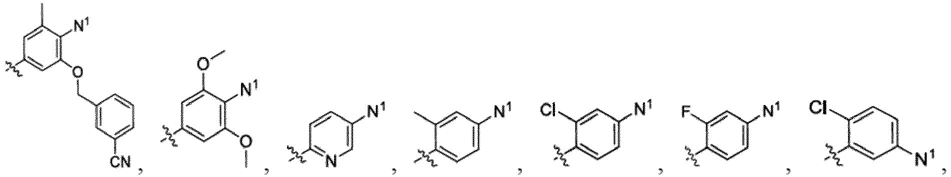 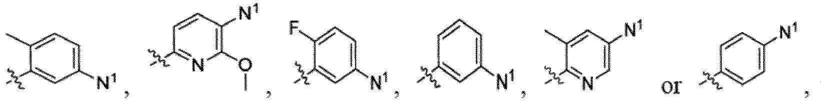 wherein the definitions of N¹, R¹⁷ and R¹⁸ are the same as defined described previously.

In a preferred embodiment, 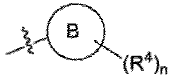 preferably is 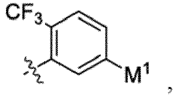 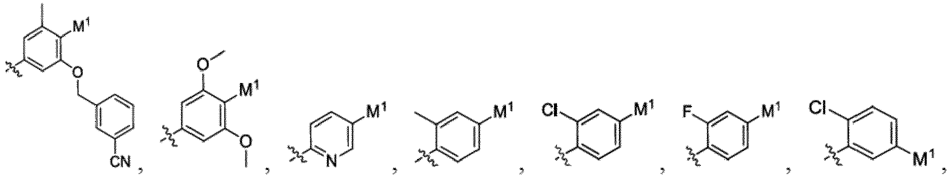 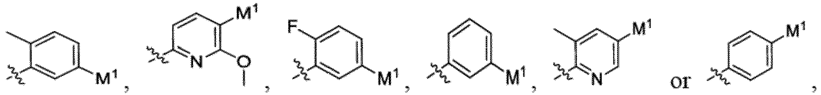 wherein the definitions of M¹, R¹⁷ and R¹⁸ are the same as defined described previously.

In a preferred embodiment, 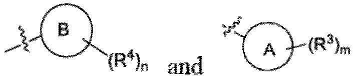 independently are preferably

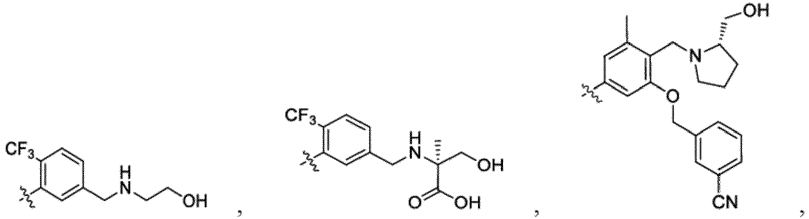

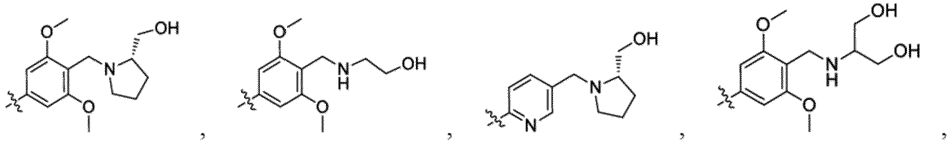

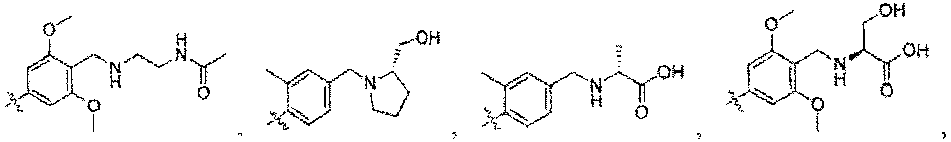

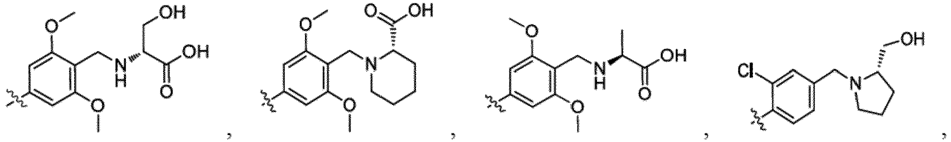

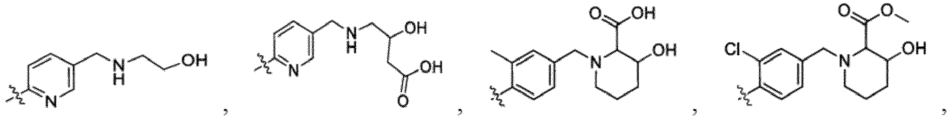

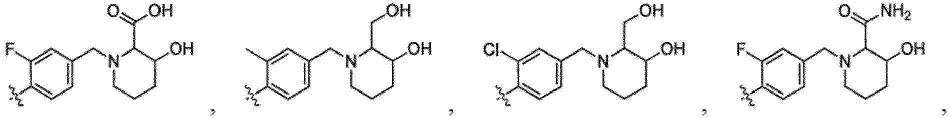

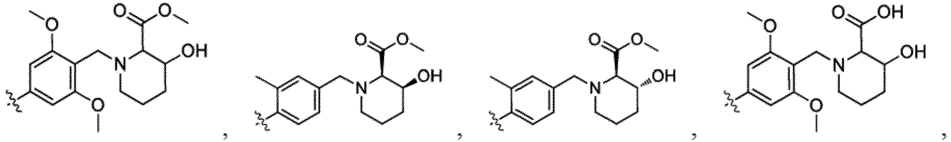

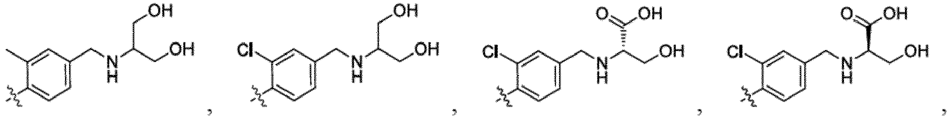

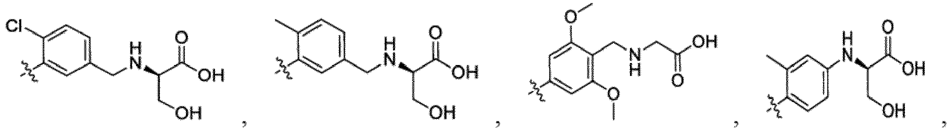

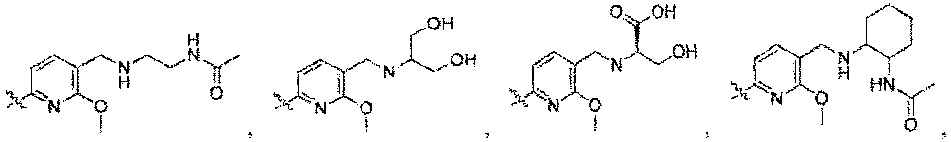

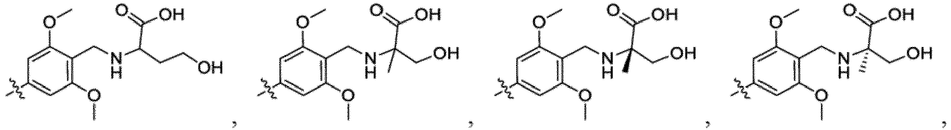

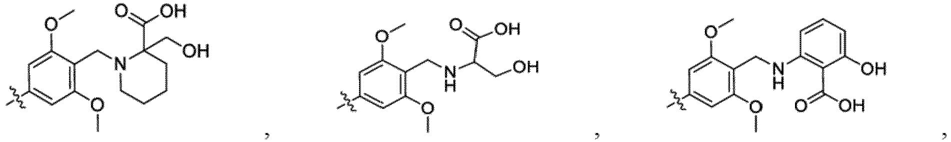

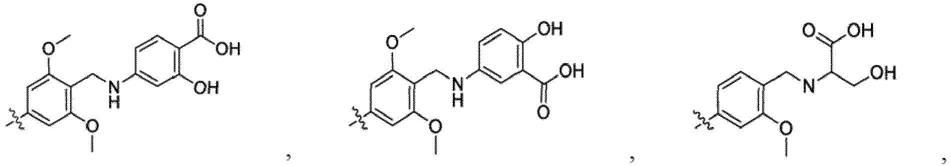

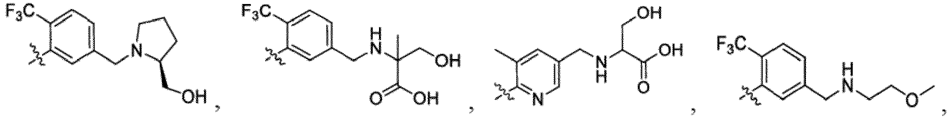

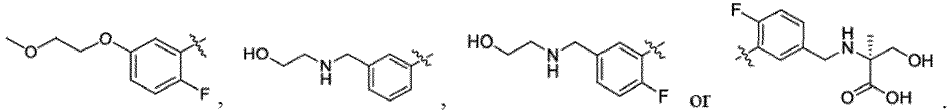

In a preferred embodiment, 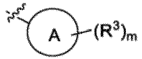 preferably is

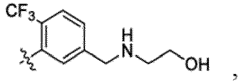

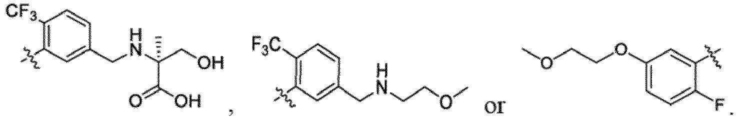

In a preferred embodiment, 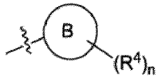 preferably is

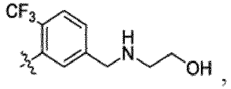

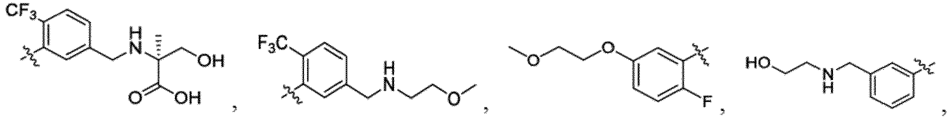

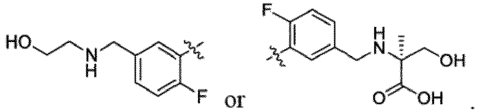

In a preferred embodiment, 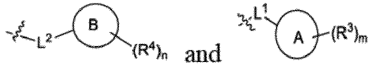 independently are preferably 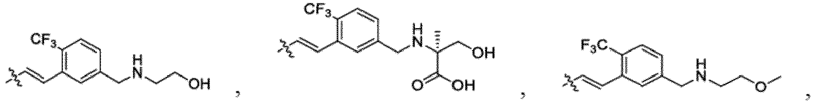 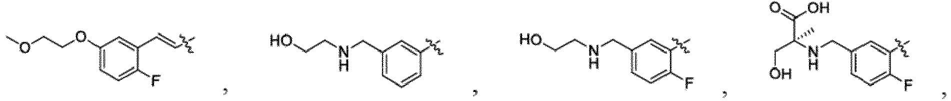 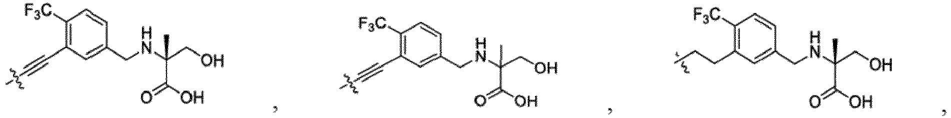 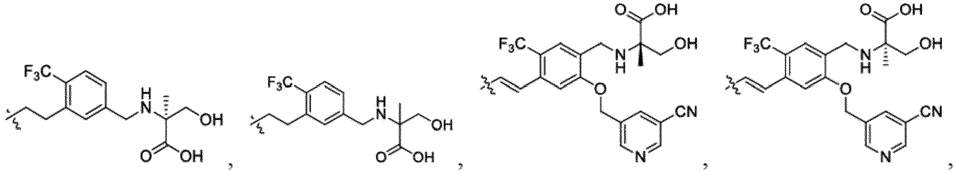 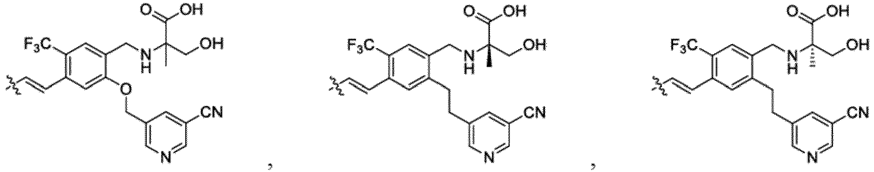 or

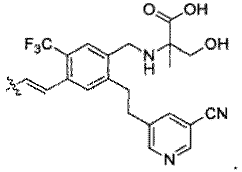

In a preferred embodiment,
L¹ is alkynyl, -C(R⁵)=C(R⁶)- or -CR⁷R⁸-CR⁹R¹⁰-,
L² is alkynyl, -C(R⁵)=C(R⁶)-, -CR⁷R⁸-CR⁹R¹⁰- or absent,
R⁵, R⁶, R⁷, R⁸, R⁹ and R¹⁰ are independently hydrogen or deuterium respectively,
R¹ is halogen, or substituted or unsubstituted alkyl,
R² is halogen, or substituted or unsubstituted alkyl, and
R³ and R⁴ are independently deuterium, halogen, cyano, -SR¹¹, -NR¹²R¹³, substituted or unsubstituted alkyl, or substituted or unsubstituted alkoxy.

In a preferred embodiment,
L¹ is alkynyl, -C(R⁵)=C(R⁶)- or -CR⁷R⁸-CR⁹R¹⁰-,
L² is alkynyl, -C(R⁵)=C(R⁶)-, -CR⁷R⁸-CR⁹R¹⁰- or absent,
R⁵, R⁶, R⁷, R⁸, R⁹ and R¹⁰ are independently hydrogen or deuterium respectively,
R¹ is halogen, or substituted or unsubstituted alkyl,
R² is halogen, or substituted or unsubstituted alkyl, and
R³ and R⁴ are independently halogen, -SR¹¹, or substituted or unsubstituted alkoxy; R¹¹ is substituted C₁-C₄ alkyl; substituents in the substituted alkyl are substituted with one or more of halogen, cyano, hydroxyl, 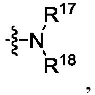 C₆-C₁₄ aryl, substituted C₆-C₁₄ aryl, C₁-C₁₀ heteroaryl, substituted C₁-C₁₀ heteroaryl, C₁-C₄ alkoxy and C₁-C₄ carboxyl; substituents in the substituted alkoxy are substituted with one or more of halogen, cyano, hydroxyl, 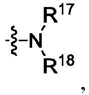 C₆-C₁₄ aryl, substituted C₆-C₁₄ aryl, C₁-C₁₀ heteroaryl and substituted C₁-C₁₀ heteroaryl; when substituents are more than one, the substituents are identical or different.

In a preferred embodiment,
L¹ is alkynyl, -C(R⁵)=C(R⁶)- or -CR⁷R⁸-CR⁹R¹⁰-,
L² is alkynyl, -C(R⁵)=C(R⁶)-, -CR⁷R⁸-CR⁹R¹⁰- or absent,
R⁵, R⁶, R⁷, R⁸, R⁹ and R¹⁰ are independently hydrogen or deuterium respectively;
R¹ is halogen, or substituted or unsubstituted alkyl;
R² is halogen or alkyl,
R³ and R⁴ are independently halogen, -SR¹¹, substituted or unsubstituted alkyl, or substituted or unsubstituted alkoxy; R¹¹ is substituted C₁-C₄ alkyl; substituents in the substituted alkyl are substituted with one or more of halogen, 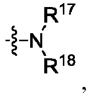 substituted C₆-C₁₄ aryl and substituted C₁-C₁₀heteroaryl; substituents in the substituted alkoxy preferably are substituted with one or more of C₁-C₄ alkoxy, C₁-C₁₀heteroaryl and substituted C₁-C₁₀ heteroaryl; when substituents are more than one, the substituents are identical or different.

In a preferred embodiment,
L¹ is -C(R⁵)=C(R⁶)- (preferably -CH=CH-),
L² is -C(R⁵)=C(R⁶)- or absent (preferably -CH=CH-),
R⁵ and R⁶ are independently hydrogen or deuterium,
R¹ is halogen, alkyl (preferably C₁-C₄ alkyl, more preferably methyl), or alkyl substituted with halogen,
R² is halogen or alkyl (preferably C₁-C₄ alkyl, more preferably methyl, ethyl, n-propyl, isopropyl, n-butyl, isobutyl or tert-butyl),
R³ and R⁴ are independently halogen, -SR¹¹, substituted or unsubstituted alkyl, or substituted or unsubstituted alkoxy; R¹¹ is substituted C₁-C₄ alkyl; substituents in the substituted alkyl are preferably substituted with one or more of halogen, 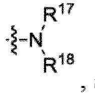 substituted C₆-C₁₄ aryl and substituted C₁-C₁₀ heteroaryl (further, R³ and R⁴ are defined as follows: (1) R³ and R⁴ are preferably -SR¹¹, R¹¹ is substituted C₁-C₄ alkyl; (2) R³ and R⁴ are preferably alkyl substituted with halogen, the alkyl substituted with halogen is preferably C₁-C₄ alkyl substituted with one or more of F, Cl, Br and I, preferably -CF₃; (3) R³ and R⁴ are preferably alkyl substituted with 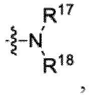 the alkyl substituted with 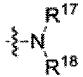 is preferably C₁-C₄ alkyl substituted with 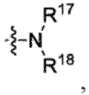 the C₁-C₄ alkyl substituted with 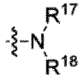 is preferably 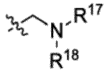 or 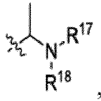 wherein, one of R¹⁷ and R¹⁸ is H, the other one is alkyl substituted with one or more of C₁-C₄ alkoxy, hydroxy and carboxyl; when R³ and R⁴ are alkyl substituted with 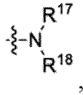 the alkyl substituted with 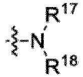 is preferably 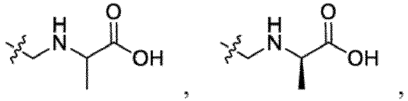 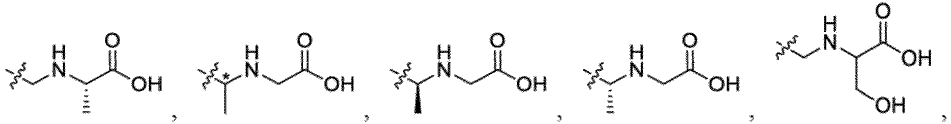 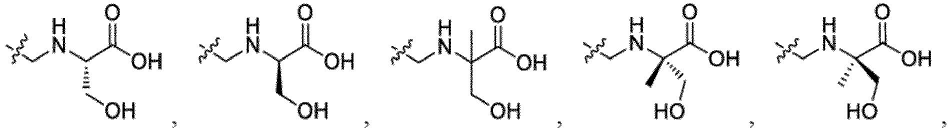 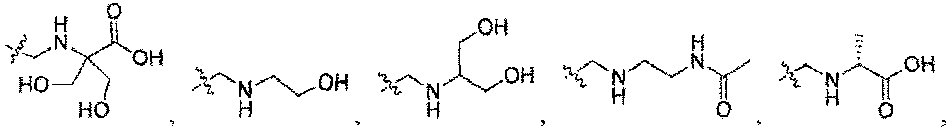 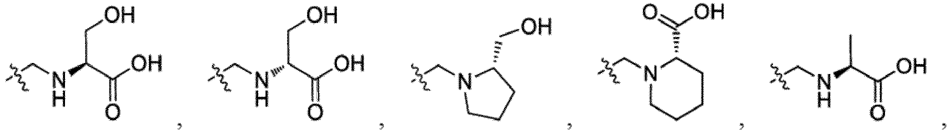 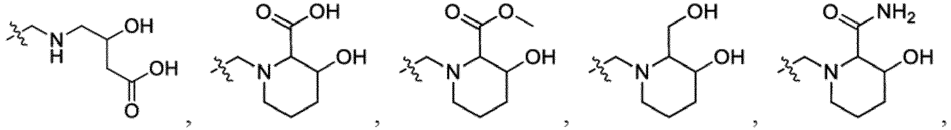 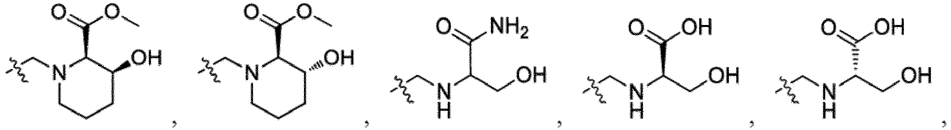 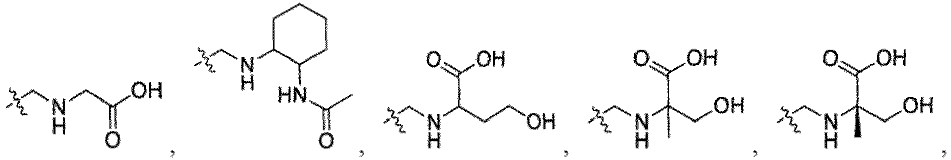 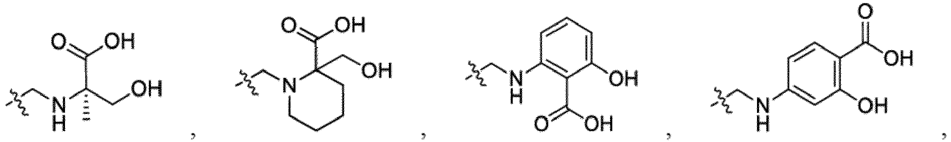 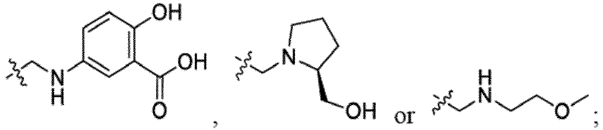 (4) R³ and R⁴ are preferably alkyl substituted with substituted C₆-C₁₄ aryl, more preferably 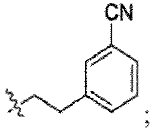 (5) R³ and R⁴ are preferably alkyl substituted with substituted C₁-C₁₀ heteroaryl, more preferably 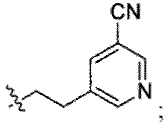 (6) R³ and R⁴ are preferably substituted alkoxy, substituents in the substituted alkoxy is preferably substituted with one or more of C₁-C₄ alkoxy, C₁-C₁₀ heteroaryl and substituted C₁-C₁₀ heteroaryl, the substituted alkoxy is preferably

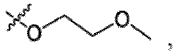

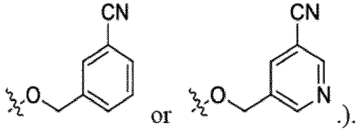

The diphenyl-like compound represented by the general formula **I** in the present disclosure is preferably selected from any one compound of: 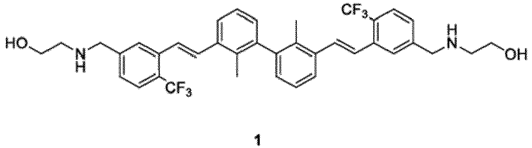 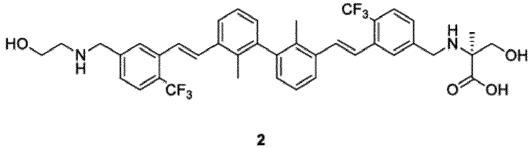 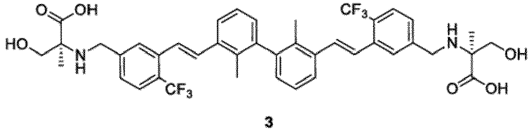 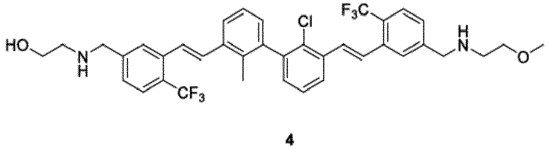 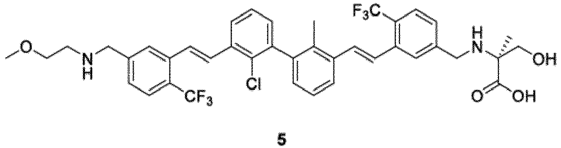 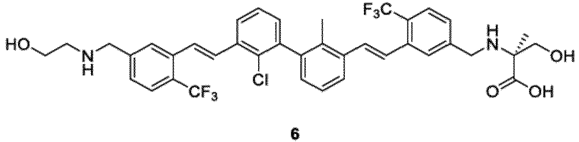 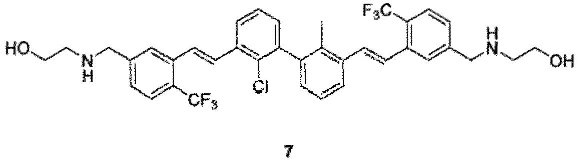 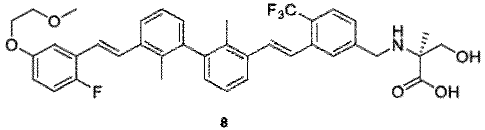 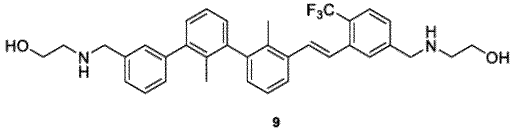 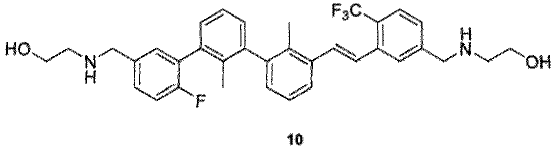 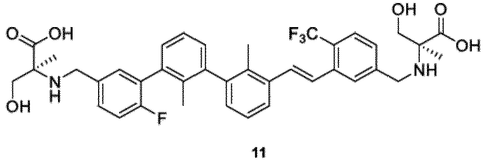 and

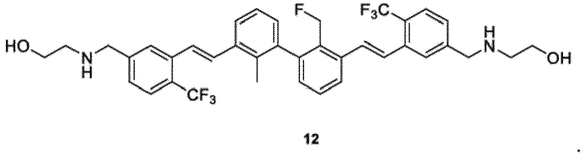

In a preferred embodiment, the diphenyl-like compound represented by the general formula I is preferably a diphenyl-like compound represented by general formula **I-A** or **II:**

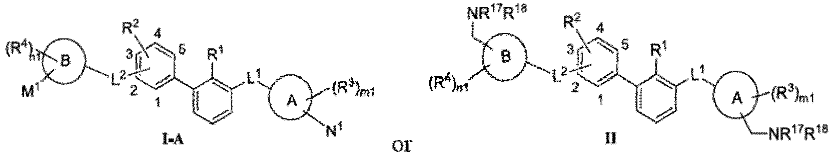

wherein, the definitions of ring A, ring B, L₁, L₂, R¹, R², R³, R⁴, M¹, N¹, R¹⁷ and R¹⁸ are the same as defined described previously, n1 is 0, 1 or 2, m1 is 0, 1 or 2.

In the present disclosure, 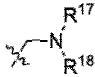 in ring A and 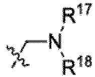 in ring B of a diphenyl-like compound represented by general formula II may be identical or different.

The present disclosure also provides a preparation method of a diphenyl-like compound represented by general formula **I-A** or **II,**

in the compound represented by general formula **I-A,** when -NH- or -COOH is contained in M¹ and N¹, it is prepared by using a method comprising a step of: deprotecting a compound represented by general formula **II-F** as shown below, to obtain the diphenyl-like compound represented by general formula **I-A,**

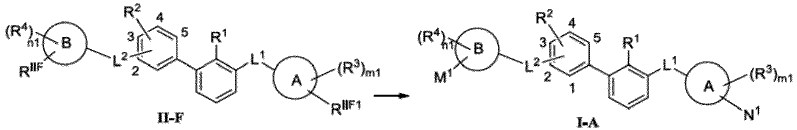

wherein the definitions of ring A, ring B, L¹, L², R¹, R², R³, R⁴, M¹ and N¹ are the same as defined described previously, n1 is 0, 1 or 2, m1 is 0, 1 or 2, R^{IIF} is a group containing amino or carboxyl protecting group corresponding to M¹, R^{IIF1} is identical to N¹; or R^{IIF} is identical to M¹, R^{IIF1} is a group containing amino or carboxyl protecting group corresponding to N¹; or R^{IIF} is a group containing amino or carboxyl protecting group corresponding to M¹, R^{IIF1} is a group containing amino or carboxyl protecting group corresponding to N¹;

the preparation method of the diphenyl-like compound of general formula **II** employs any one method of:
(1) method 1 comprising a step of: reacting a compound represented by general formula **II-A** with a compound **II-A1** as shown below, to obtain the diphenyl-like compound represented by general formula **II**, 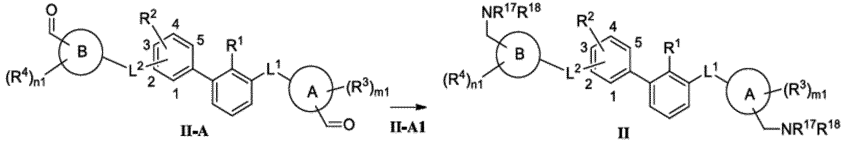
   wherein the structure of the compound **II-A1** is as follows: 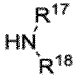 or an acid salt thereof,
   the definitions of ring A, ring B, L¹, L², R¹, R², R³, R⁴, R¹⁷ and R¹⁸ are the same as defined described previously, n1 is 0, 1 or 2, m1 is 0, 1 or 2; in this method, 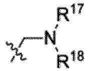 in ring A and ring B are identical;
(2) method 2 comprising a step of: reacting a compound represented by general formula **II-B** with a compound **II-B1** as shown below, to obtain the diphenyl-like compound represented by general formula **II,** 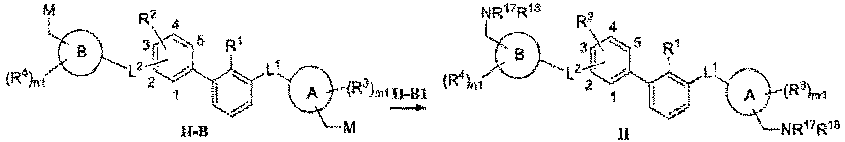
   wherein the structure of the compound **II-B1** is as follows: 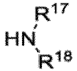 or an acid salt thereof,
   the definitions of ring A, ring B, L¹, L², R¹, R², R³, R⁴, R¹⁷ and R¹⁸ are the same as defined described previously, n1 is 0, 1 or 2, m1 is 0, 1 or 2, M is halogen; in this method, 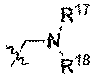 in ring A and ring B are identical;
(3) method 3 comprising a step of: reacting a compound represented by general formula **II-C** with a compound **II-C1** as shown below, to obtain the diphenyl-like compound represented by general formula **II,** 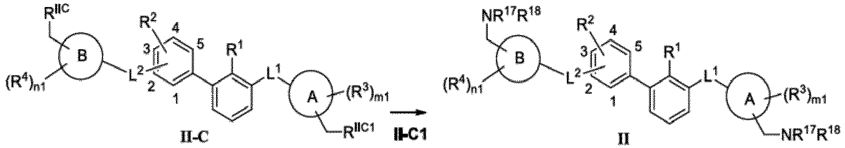
   wherein the structure of the compound **II-C1** is as follows: 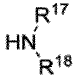 or an acid salt thereof,
   the definitions of ring A, ring B, L¹, L², R¹, R², R³, R⁴, R¹⁷ and R¹⁸ are the same as defined described previously, n1 is 0, 1 or 2, m1 is 0, 1 or 2; one of R^{IIC} and R^{IIC1} is 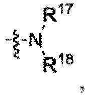 the other one is 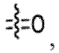 in this method, 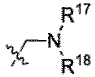 in ring A and ring B are identical or different;
(4) method 4 comprising a step of: reacting a compound represented by general formula **II-D** with a compound **II-D1** as shown below, to obtain the diphenyl-like compound represented by general formula II**,** 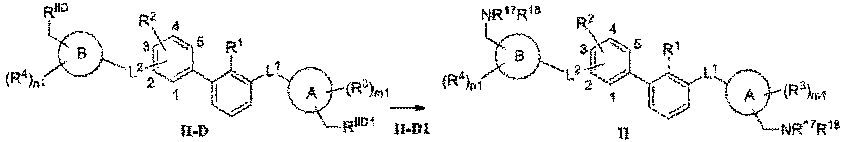
   wherein the structure of the compound **II-D1** is as follows: 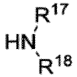 or an acid salt thereof,
   the definitions of ring A, ring B, L¹, L², R¹, R², R³, R⁴, R¹⁷ and R¹⁸ are the same as defined described previously, n1 is 0, 1 or 2, m1 is 0, 1 or 2, one of R^{IID} and R^{IID1} is 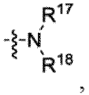 the other one is halogen, in this method, 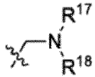 in ring A and ring B are identical or different;
(5) method 5 comprising a step of: deprotecting a compound represented by general formula **II-E** as shown below, to obtain the diphenyl-like compound represented by general formula **II,** R¹⁷ or R¹⁸ in the compound represented by general formula **II** containing carboxyl; 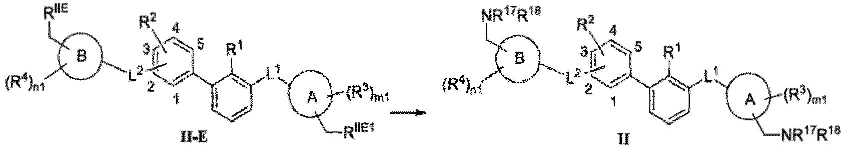 wherein the definitions of ring A, ring B, L¹, L², R¹, R², R³, R⁴, R¹⁷ and R¹⁸ are the same as defined described previously, n1 is 0, 1 or 2, m1 is 0, 1 or 2, R^{IIE} and R^{IIE1} are 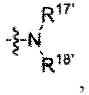 each R¹⁷ and each R¹⁸ are identical or different, and at least one contains carboxyl protecting group, R¹⁷ and R¹⁸ free of carboxyl protecting group are the same as the corresponding R¹⁷ and R¹⁸ in general formula **II** respectively; in this method, 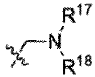 in ring A and ring B are identical or different.

The present disclosure also provides a compound represented by general formula **II-A, II-B**, **II-C, II-D, II-E** and **II-F**: 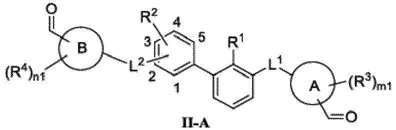 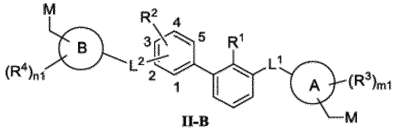 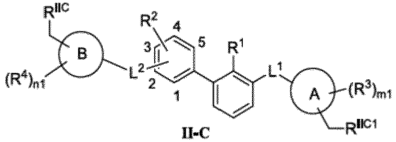 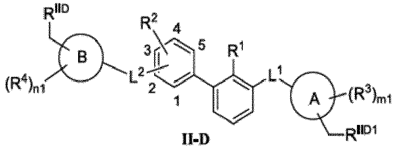 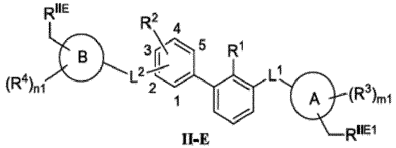 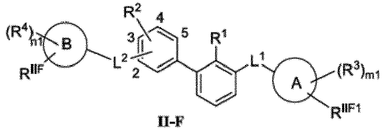 the definitions of ring A, ring B, L¹, L², R¹, R², R³, R⁴, M¹, N¹, R¹⁷ and R¹⁸ are the same as defined described previously, n1 is 0, 1 or 2, m1 is 0, 1 or 2; M is halogen, one of R^{IIC} and R^{IIC1} is 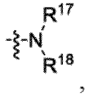 the other one is 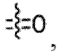 one of R^{IID} and R^{IID1} is 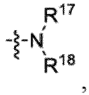 the other one is halogen, R^{IIE} and R^{IIE1} are 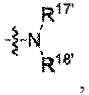 each R^{17'} and each R^{18'} are identical or different, and at least one contains carboxyl protecting group, R^{17'}and R^{18'} free of carboxyl protecting group are the same as the corresponding R¹⁷ and R¹⁸ in general formula **II** respectively; R^{IIF} is a group containing amino or carboxyl protecting group corresponding to M¹, R^{IIF1} is identical to N¹; or R^{IIF} is identical to M¹, R^{IIF1} is a group containing amino or carboxyl protecting group corresponding to N¹; or R^{IIF} is a group containing amino or carboxyl protecting group corresponding to M¹, R^{IIF1} is a group containing amino or carboxyl protecting group corresponding to N¹.

The present disclosure also provides compounds as shown below:

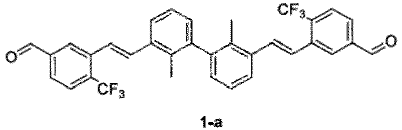

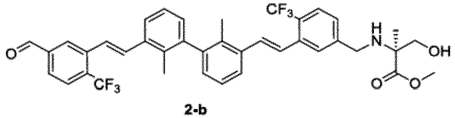

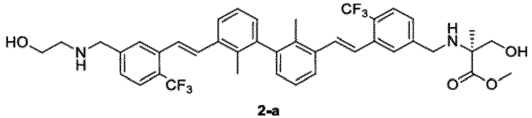

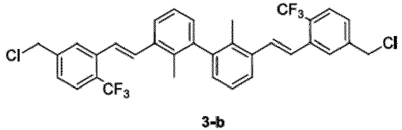

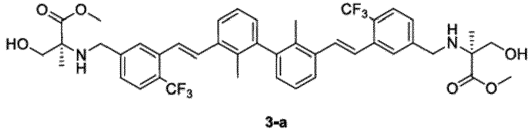

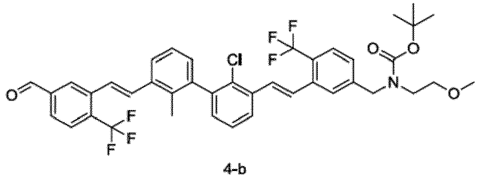

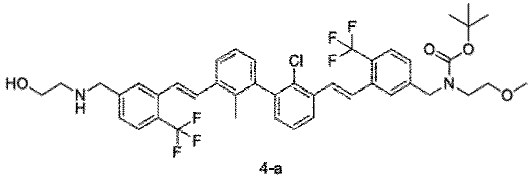

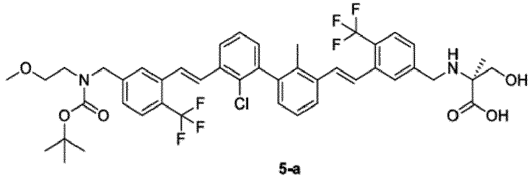

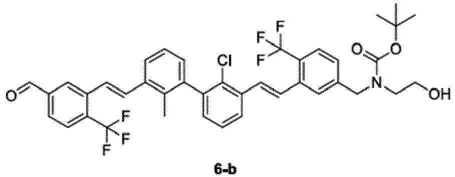

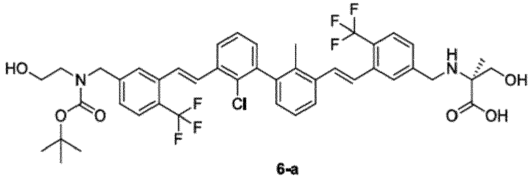

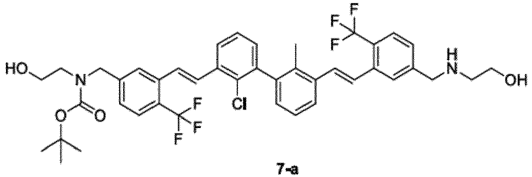

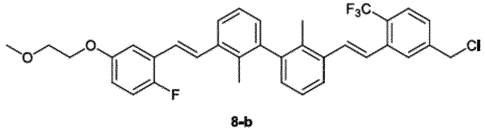

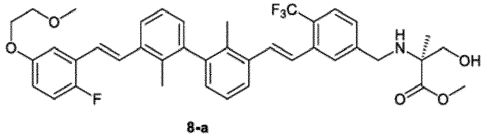

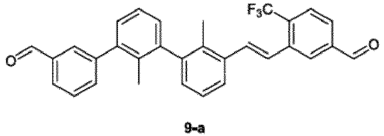

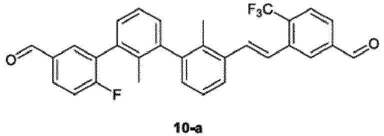

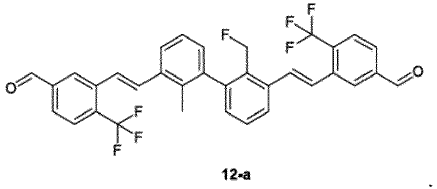

The present disclosure also provides use of the diphenyl-like compound of general formula **I,** a pharmaceutically acceptable salt, tautomer, mesomer, racemate, stereoisomer, or prodrug thereof in the preparation of a PD-1 inhibitor and/or PD-L1 inhibitor.

The present disclosure also provides use of one or more of the diphenyl-like compound of general formula **I,** a pharmaceutically acceptable salt, tautomer, mesomer, racemate, stereoisomer, metabolite, metabolic precursor and prodrug thereof in the preparation of a medicament for preventing, alleviating or treating cancer, infection, autoimmune disease or related diseases thereof.

The cancer is preferably one or more of lung cancer, esophageal cancer, gastric cancer, colorectal cancer, liver cancer, nasopharyngeal cancer, brain tumor, breast cancer, cervical cancer, blood cancer and bone cancer.

The present disclosue also provides a pharmaceutical composition comprising a therapeutically and/or prophylactically effective amount of the diphenyl-like compound of general formula **I,** a pharmaceutically acceptable salt, tautomer, mesomer, racemate, stereoisomer, metabolite, metabolic precursor or prodrug thereof, and a pharmaceutically acceptable carrier and/or diluent.

In the present disclosure, according to therapeutic purpose, the pharmaceutical composition can be formulated into a variety of unit dosage form for administration, such as, tablet, pill, powder, liquid, suspension, emulsion, granule, capsule, suppository and injection (solution and suspension) and the like, preferably liquid, suspension, emulsion, suppository and injection (solution and suspension) and the like.

Any excipient known and widely used in the art can be used in order to shape the pharmaceutical composition in the form of tablet. For example, carriers, such as lactose, white sugar, sodium chloride, glucose, urea, starch, calcium carbonate, kaolin, crystalline cellulose and silicic acid, etc; adhesives, such as water, ethanol, propanol, common syrup, glucose solution, starch solution, gelatin solution, carboxymethyl cellulose, lac, methyl cellulose and potassium phosphate, polyvinylpyrrolidone, etc; disintegrants, such as dry starch, sodium alginate, agar powder and kelp powder, sodium bicarbonate, calcium carbonate, fatty acid ester of polyethylene sorbitan, sodium dodecyl sulfate, glycerol monostearate, starch and lactose, etc; disintegration inhibitors, such as white sugar, glycerol tristearate, coconut oil and hydrogenated oil; adsorptive promoters, such as quaternary ammonium base and sodium dodecyl sulfate, etc; wetting agents, such as glycerol, starch, etc; adsorbents, such as starch, lactose, kaolin, bentonite and colloidal silicic acid, etc; and lubricants, such as pure talc, stearate, boric acid powder and polyethylene glycol, etc. The common coating materials can also be used as needed to make sugar coated tablets, gelatin coated tablets, enteric coated tablets, coated tablets, double-layer coated tablets and multi-layer coated tablets.

In order to shape the pharmaceutical composition in the form of pill, any excipient known and widely used in the art can be used, for example, carriers, such as lactose, starch, coconut oil, hardening vegetable oil, kaolin and talc, etc; adhesives, such as arabic gum powder, gum tragacanth powder, gelatin and ethanol, etc; disintegrants, such as agar and kelp powder, etc.

In order to shape the pharmaceutical composition in the form of suppository, any excipient known and widely used in the art can be used, for example, polyethylene glycol, coconut oil, higher alcohols, esters of higher alcohols, gelatin and semisynthetic glycerides, etc.

In order to prepare the pharmaceutical composition in the form of injection, injection isotonic with blood is made after disinfecting solution or suspension (it is better to add an appropriate amount of sodium chloride, glucose or glycerol, etc). Any common carrier in the art can also be used while preparing injection. For example, water, ethanol, propylene glycol, ethoxylated isostearyl alcohol, polyoxy isostearyl alcohol and fatty acid ester of polyethylene sorbitan, etc. Additionally, common solvents, buffers and analgetic, etc can also be added.

The diluent in the pharmaceutical composition may be a conventional diluent in the art.

The pharmaceutical composition may be in the oral form, also may be in the form of sterile injection aqueous solution, and the oral or injection composition can be prepared according to any method for preparing a pharmaceutical composition known in the art.

Unless otherwise stated, all of the following terms appear in the specification and claims of the present disclosure have the following meanings:

All the term cycloalkyl (including when used alone or contained in other groups) comprises a saturated or partially-unsaturated (containing 1 or 2 double bonds) cyclic hydrocarbon group containing 1 to 3 rings, which comprises monocyclic alkyl, bicyclic alkyl and tricyclic alkyl.

All the term alkoxy represents cyclic or non-cyclic alkyl linked via oxygen bridge with the recited carbon atom number. Thus, alkoxy comprises the definitions of the above alkyl and cycloalkyl.

All the term alkenyl refers to a linear, branched or cyclic non-aromatic hydrocarbonyl containing specified number of carbon atom and at least one carbon-carbon double bond. There is preferably one carbon-carbon double bond, and there may be up to four non-aromatic carbon-carbon double bonds. Alkenyl is preferably C₂₋₁₂ alkenyl, further preferably C₂₋₆ alkenyl. Thus, C₂₋₁₂ alkenyl refers to alkenyl having 2 to 12 carbon atoms. C₂₋₆ alkenyl refers to alkenyl having 2 to 6 carbon atoms, comprising ethenyl, propenyl, butenyl, 2-methylbutenyl and cyclohexenyl. The linear chain, branched chain or ring moiety of alkenyl may contain a double bond, and if indicated as substituted alkenyl, it may be substituted.

All the term alkynyl refers to a linear, branched or cyclic hydrocarbonyl containing specified number of carbon atom and at least one carbon-carbon triple bond. There can be up to three carbon-carbon triple bonds. Alkynyl is preferably C₂₋₁₂ alkynyl, further preferably C₂₋₆ alkynyl. Thus, C₂₋₁₂ alkynyl refers to alkynyl having 2 to 12 carbon atoms. C₂₋₆ alkynyl refers to alkynyl having 2 to 6 carbon atoms, comprising ethynyl, propynyl, butynyl and 3-methylbutynyl and the like.

All the term hydroxy represents

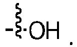

All the term amino represents

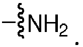

All the term cyano represents -CN.

All the term carboxyl represents -COOH, wherein C₁-C₄ carboxyl refers to - (CH₂)ₙCOOH, n is 0, 1, 2 or 3. All the term C₁-C₄ carboxyl is preferably 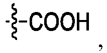 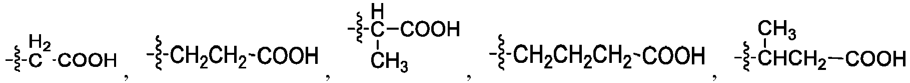 or

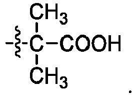

All the term ester group represents -COO-, wherein C₁-C₄ ester group refers to - COOR^{x}, R^{x} is C₁-C₄ alkyl.

All the term amido represents "-CONR^{x1}R^{x2}" or "-NR^{x3}COR^{x4}", R^{x1}, R^{x2}, R^{x3} and R^{x4} are independently H or C₁-C₄ alkyl.

All the term heteroaryl should be understood to comprise N-oxide derivatives of any heteroaromatic ring containing nitrogen. In the case where the heteroaryl substituent is a dicyclic substituent and one ring is a non-aromatic ring or contains no heteroatom, it can be understood that linking occurs through an aromatic ring or heteroatom containing ring respectively.

All the term therapeutically effective amount refers to an amount of a compound sufficient to effectively treat diseases or disorders described herein when administered to a subject. Although an amount of a compound constituting "therapeutically effective amount" will vary depending on the compound, disorders and severity thereof, and age of the subject to be treated, it can be determined in a conventional manner by a person skilled in the art.

When the specific salt, pharmaceutical composition, composition, excipient and the like are mentioned to be "pharmaceutically acceptable", it means that the salt, pharmaceutical composition, composition, excipient and the like are generally non-toxic, safe, and suitable for use by a subject, preferably a mammal subject, more preferably a human subject.

All the term pharmaceutically acceptable salts refer to pharmaceutically acceptable organic or inorganic salts of the compounds of the present disclosure. Exemplary salts include but are not limited to: sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate and pamoate (i.e., 1-1-methylene-bis (2-hydroxy-3-naphthoate)).

All the term prodrugs refer to derivatives of a compound containing a biologically reactive functional group such that under biological conditions (in vitro or in vivo), the biologically reactive functional group can be cleaved from the compound or otherwise react to provide the compound. In general, prodrugs are inactive, or at least less active than the compound itself, such that the compound does not exhibit its activity until it is cleaved from the biologically reactive functional group. A biologically reactive functional group may be hydrolyzed or oxidized under biological conditions to provide the compound. For example, prodrugs may contain a biologically hydrolyzable group. Examples of a biologically hydrolyzable group include but not limited to biologically hydrolyzable phosphate, biologically hydrolyzable amide, biologically hydrolyzable carbonate, biologically hydrolyzable carbamate and biologically hydrolyzable ureide.

A compound of the present disclosure may have one or more asymmetric center ("stereoisomer"). As used herein, all the term "stereoisomer" refers to cis- or trans-isomer, R- or S-enantiomer and diastereoisomer. These stereoisomers can be prepared by asymmetric synthesis or chiral separation (e.g., separation, crystallization, thin layer chromatography, column chromatography, gas chromatography, high performance liquid chromatography). These stereoisomers may also be obtained by diastereoisomer-derivatization in which a mixture of enantiomers or racemates reacts with a suitable chiral compound, and then by crystallization or any other suitable conventional method.

All the term subject refers to any animal that will receive or has received administration of the compound or pharmaceutical composition, preferably mammal, most preferably human. As use herein, all the term "mammal" comprises any mammal. Examples of mammal include but not limited to cattle, horse, sheep, pig, cat, dog, mouse, rat, rabbit, guinea pig, monkey, human and the like, most preferably human.

In certain examples, treatment or being treated refers to amelioration, prevention or reversal of a disease or disorder or at least one identifiable symptom thereof. In some other examples, treatment or being treated refers to amelioration, prevention or reversal of at least one measurable body parameter of a disease or disorder being treated which may have not been identified in mammal. However, in another example, treatment or being treated refers to slowing of progression of a disease or disorder, either physically, for example stabilization of an identifiable symptom, or physiologically, for example stabilization of a body parameter, or both. In some other examples, treatment or being treated refers to delaying of the onset of a disease or disorder.

In certain examples, a compound of the present disclosure may be administered as precaution. As used herein, "prevention" or "preventing" refers to reduction of a risk of suffering from the given disease or disorder. In a preferred mode of examples, the specified compound is administered as precaution to a subject, preferably a subject having a family history or trend of cancer or autoimmune disease.

On the basis of not violating the common knowledge in the art, each of the above preferred conditions can be arbitrarily combined to obtain the more preferred examples of the present disclosure.

The reagents and raw materials used in the present disclosure are commercially available.

The positive and progressive effects of the present disclosure lie in: the diphenyl-like compound of the present disclosure has a significant inhibitory effect on PD-1 and PD-L1, and can effectively alleviate or treat the related diseases such as cancer.

### Detailed description of the embodiment

The present disclosure is further illustrated by way of examples below, but is not therefore limited to the scope of those examples. The experimental methods in the examples below of which the specific conditions are not indicated are selected according to the conventional methods and conditions, or according to the commodity instructions.

In the examples described below, room temperature refers to 10 °C to 30 °C; reflux refers to the refluxing temperature of a solvent; overnight refers to 8 to 24 hours, preferably 12 to 18 hours.

The structure of a compound is determined by nuclear magnetic reasonance (NMR) or mass spectrometry (MS), the nuclear magnetic reasonance spectra are obtained by Bruker Avance-500 instrument, using deuterated dimethyl sulfoxide, deuterated chloroform and deuterated methanol and the like as solvent, and tetramethylsilane (TMS) as internal standard. The mass spectrum is obtained by liquid chromatography-mass spectrometry (LC-MS) Agilent Technologies 6110 with ESI ion source.

The microwave reaction is carried out in the Explorer automatic microwave synthesizer produced by CEM company, USA, the magnetron frequency is 2450 MHz and the continuous microwave output power is 300 W.

The instrument used for high performance liquid chromatography preparation is Gilson 281, and the used preparation column is Shimadazu Shim-Pack, PRC-ODS, 20 x 250 mm, 15 µm.

### Example 1

2-[({3-[(E)-2-(3-{3-[(*E*)-2-(5-{[(2-hydroxyethyl)amino]methyl} -2-(trifluoromethyl)phenyl)ethenyl]-2-methylphenyl}-2-methylphenyl)ethenyl]-4-(trifluoromethyl)phenyl}methyl)amino]-1-ethanol **1**

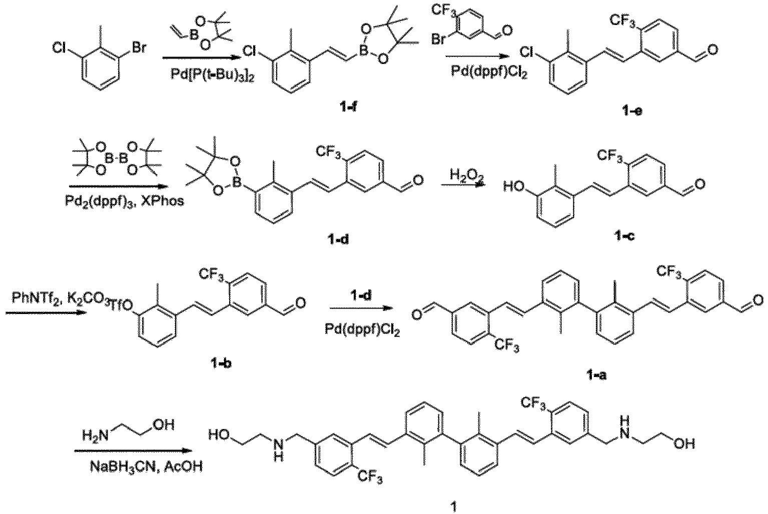

### Synthesis of compound 1-f

At room temperature, to a solution of 2-bromo-6-chlorotoluene (15.67 g, 76.26 mmol) and pinacol ethenylborate (14.30 g, 91.51 mmol) in toluene (300 mL) were added bis(tri-tert-butylphosphine)palladium (2.73 g, 5.34 mmol) and triethylamine (61.74 g, 610.08 mmol), the reaction mixture was heated to 80 °C, and reacted overnight with stirring under nitrogen. After the reaction was completed, the reaction mixture was diluted by adding ethyl acetate (100 mL), and washed with water (100 mL) and saturated brine (100 mL). The resulting organic phase was dried over anhydrous sodium sulfate, and concentrated under reduced pressure, and the residue was purified through silica gel column chromatography (petroleum ether : ethyl acetate = 10 : 1) to obtain compound **1-f** (10.5 g, yield: 49.4%).

¹H NMR (500 MHz, CDCl₃): δ 7.65-7.62 (d, *J* = 18.5 Hz, 1H), 7.42-7.41 (d, *J* = 7.5 Hz,lH), 7.31-7.30 (d, *J* = 7.5 Hz,1H), 7.12-7.09 (t, 1H), 6.06-6.02 (d, *J* = 18.0 Hz,1H), 2.45 (s, 3H), 1.32 (s, 12H).

### Synthesis of compound 1-e

At room temperature, to a mixed solution of 3-bromo-4-trifluoromethyl benzaldehyde (4.16 g, 16.45 mmol) and compound **1-f** (5.5 g, 19.74 mmol) in 1,4-dioxane (40 mL) and water (2 mL) were added [1,1'-bis(diphenylphosphino)ferrocene]dichloropalladium (1.423 g, 1.645 mmol) and sodium carbonate (4.36 g, 41.13 mmol), the reaction mixture was heated to 80 °C, and stirred under nitrogen for 16 hours. After the reaction was completed, the reaction mixture was diluted by adding ethyl acetate (50 mL), and washed with water (50 mL) and saturated brine (50 mL) successively. The resulting organic phase was dried over anhydrous sodium sulfate, and concentrated under reduced pressure, and the residue was purified through silica gel column chromatography (petroleum ether : ethyl acetate = 40 : 1) to obtain compound **1-e** (4.16 g, yield: 78%).

¹H NMR (500 MHz, CDCl₃): δ 10.15 (s, 1H), 8.25 (s, 1H), 7.87 (s, 2H), 7.47-7.40 (m, 2H), 7.37-7.36 (d, *J* = 7.0 Hz, 1H), 7.32-7.29 (m, 1H), 7.20-7.17 (m, 1H), 2.50 (s, 3H).

### Synthesis of compound 1-d

To a 100 milliliter of reaction flask were added compound **1-e** (3.24 g, 10 mmol), bis(pinacolato)diboron (3.05 g, 12 mmol), tris(dibenzylideneacetone)dipalladium (458 mg, 0.5 mmol), 2-dicyclohexylphosphino-2',4',6'-triisopropylbiphenyl (952 mg, 2.0 mmol), potassium acetate (3.0 g, 112 mmol) and toluene (80 mL). The mixture was reacted at 90 °C under nitrogen protection for 16 hours. The mixture was cooled to room temperature and filtered, the filtrate was concentrated under reduced pressure, and the resulting residue was purified through silica gel column chromatography (petroleum ether : ethyl acetate = 25 : 1) to obtain compound **1-d** (3.06 g, yield: 82%).

¹H NMR (500 MHz, CDCl₃): δ 10.15 (s, 1H), 8.28 (s, 1H), 7.85 (s, 2H), 7.73-7.76 (m, 1H), 7.62 (d, *J* = 7.5 Hz, 1H), 7.50(d, *J* = 18 Hz, 1H), 7.26-7.28 (m, 1H), 7.23 (d, *J* = 7.5 Hz, 1H), 2.65 (s, 3H), 1.37 (s, 12H).

### Synthesis of compound 1-c

At room temperature, to a solution of compound **1-d** (416.24 mg, 1.0 mmol) in tetrahydrofuran (20 mL) was added hydrogen peroxide (30%, 1 mL), and the reaction mixture was stirred at ambient temperature for 16 hours. The reaction mixture was concentrated under reduced pressure, the residue was dissolved with ethyl acetate (20 mL), and then washed with saturated sodium thiosulfate aqueous solution (20 mL) twice and saturated brine (20 mL) once. The organic phase was dried over anhydrous sodium sulfate, and concentrated under reduced pressure, and the residue was purified through silica gel column chromatography (petroleum ether : ethyl acetate = 10 : 1) to obtain compound **1-c** (220 mg, yield: 71.9%).

¹H NMR (400 MHz, CDCl₃): δ 10.17 (s, 1H), 8.29 (s, 1H), 7.89 (s, 2H), 7.48-7.45 (d, *J* = 16.0 Hz, 1H), 7.37-7.33 (m, 1H), 7.23-7.21 (d, *J* = 7.5 Hz, 1H), 7.16-7.13 (t, 1H), 6.82-6.80 (d, *J =* 8.0 Hz, 1H), 4.90 (s, 1H), 2.38 (s, 3H).

### Synthesis of compound 1-b

At room temperature, to a solution of compound **1-c** (529 mg, 1.73 mmol) and phenylbis(trifluoromethanesulfonyl)imide (618 mg, 1.73 mmol) in acetone (20 mL) was added potassium carbonate (358 mg, 2.59 mmol), and the reaction mixture was stirred at ambient temperature for 16 hours. The reaction mixture was concentrated under reduced pressure, the residue was dissolved with ethyl acetate (20 mL), and then washed with saturated sodium thiosulfate aqueous solution (20 mL) twice and saturated brine (20 mL) once. The organic phase was dried over anhydrous sodium sulfate, and concentrated under reduced pressure, and the resulting residue was purified through silica gel column chromatography (petroleum ether : ethyl acetate = 40 : 1 to 20 : 1) to obtain compound **1-b** (391 mg, yield: 51.7%).

¹H NMR (400 MHz, CDCl₃): δ 10.08 (s, 1H), 8.18 (s, 1H), 7.85-7.81 (m, 2H), 7.54-7.52 (d, *J* = 7.5 Hz, 1H), 7.30 (s, 2H), 7.28-7.24 (m, 1H), 7.19-7.18 (m, 1H), 2.39 (t, 3H).

### Synthesis of compound 1-a

Under nitrogen protection, to a mixed solution of compound **1-b** (391 mg, 0.892 mmol) and compound **1-d** (445.4 mg, 1.070 mmol) in 1,4-dioxane (20 mL) and water (1 mL) were added [1,1'-bis(diphenylphosphino)ferrocene]dichloropalladium (77.2 mg, 0.0892 mmol) and sodium carbonate (236.4 mg, 2.23 mmol). The reaction mixture was heated to 80 °C, and stirred for 16 hours. The reaction mixture was cooled to room temperature, diluted by adding ethyl acetate solution (20 mL), and then washed with water (20 mL) three times and saturated brine (20 mL) once. The organic phase was dried over anhydrous sodium sulfate, and concentrated under reduced pressure, and the residue was purified through silica gel thin layer chromatography preparation plate (petroleum ether: ethyl acetate = 10 : 1) to obtain compound **1-a** (116 mg, yield: 22.5%).

¹H NMR (400 MHz, CDCl₃): δ 10.07 (s, 2H), 8.22 (s, 2H), 7.80 (s, 4H), 7.57-7.55 (d, *J=* 7.5 Hz, 2H), 7.46-7.43 (d, *J=* 15.5 Hz, 2H), 7.36-7.31 (m, 2H), 7.26- 7.21 (m, 2H), 7.08-7.07 (d, *J=* 7.0 Hz, 2H), 2.11 (s, 6H).

### Synthesis of compound 1

At room temperature, to a mixed solution of compound **1-a** (100 mg, 0.27 mmol) and aminoethanol (64.3 mg, 0.54 mmol) in methanol (10 mL) and dichloromethane (10 mL) was added glacial acetic acid (32.4 mg, 0.54 mmol), and the reaction mixture was stirred at room temperature for 1 hour. Then, sodium cyanoborohydride (84.8 mg, 1.35 mmol) was added and stirred for 16 hours. The reaction mixture was concentrated under reduced pressure, the residue was dissolved with ethyl acetate (20 mL), and then washed with water (20 mL) and saturated brine (20 mL) once respectively. The organic phase was dried over anhydrous sodium sulfate, and concentrated under reduced pressure, and the residue was purified through silica gel thin layer chromatography preparation plate (dichloromethane : methanol = 10 : 1) to obtain compound **1** (24 mg, yield: 18.9%). LC-MS (ESI): m/z = 669.0 [M+H]⁺.

¹H NMR (400 MHz, CD₃OD): δ 8.15 (s, 2H), 7.84-7.83 (d, *J*= 8.0 Hz, 2H), 7.67-7.59 (m, 6H), 7.41-7.33 (m, 4H), 7.14-7.13 (d, *J =* 6.5 Hz, 2H), 4.41 (s, 4H), 3.88- 3.86 (m, 4H), 3.25-3.23 (m, 4H), 2.20 (s, 6H).

### Example 2

(2*S*)-3-hydroxy-2-[({3-[(*E*)-2-(3-{3-[(*E*)-2-(5-{[(2-hydroxyethyl)amino]methyl}-2-(trifluoromethyl)phenyl)ethenyl]-2-methylphenyl}-2-methylphenyl)ethenyl]-4-(trifluoromethyl)phenyl}methyl)amino]-2-methylpropanoic acid **2**

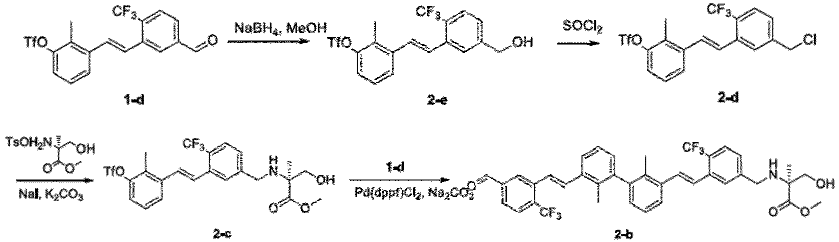

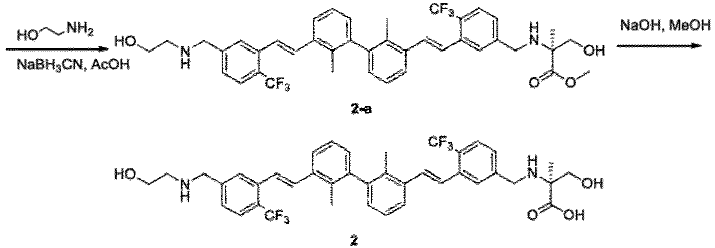

### Synthesis of compound 2-e

At room temperature, to a mixed solution of compound **1-b** (559 mg, 1.275 mmol) in tetrahydrofuran (10 mL) and methanol (10 mL) was added sodium borohydride (96.5 mg, 2.551 mmol), and the reaction mixture was stirred for 16 hours. The reaction mixture was concentrated under reduced pressure, the residue was dissolved with ethyl acetate (20 mL), and then washed with water (20 mL) and saturated brine (20 mL) once respectively. The organic phase was dried over anhydrous sodium sulfate, and concentrated under reduced pressure, and the residue was purified through silica gel column chromatography (petroleum ether : ethyl acetate = 10 : 1) to obtain compound **2-e** (517 mg, yield: 92.5%).

### Synthesis of compound 2-d

At room temperature, to a solution of compound **2-e** (517 mg, 1.174 mmol) in dichloromethane (10 mL) were added thionyl chloride (698.4 mg, 5.87 mmol) and 2 drops of *N*,*N*-dimethylformamide, and the reaction mixture was stirred for 16 hours. The reaction mixture was concentrated under reduced pressure, and the resulting oil was dissolved with ethyl acetate (20 mL), washed with saturated sodium bicarbonate aqueous solution (20 mL) twice, saturated brine (20 mL) once, and the resulting organic phase was dried over anhydrous sodium sulfate, concentrated under reduced pressure to obtain compound **2-d** (500 mg, yield: 92.9%) which was directly used in the next reaction.

¹H NMR (400 MHz, CDCl₃): δ 7.78 (s, 1H), 7.73-7.71 (d, *J*= 8.0 Hz, 1H), 7.62-7.60 (d, *J=* 8.0 Hz, 1H), 7.47-7.45 (d, *J=* 8.0 Hz, 1H), 7.40-7.32 (m, 2H), 7.29- 7.25 (m, 2H), 4.69 (s, 2H), 2.47 (m, 3H).

### Synthesis of compound 2-c

To a solution of compound **2-d** (500 mg, 1.09 mmol) and (*S*)-2-methylserine methyl ester p-toluenesulfonate (332.8 mg, 1.09 mmol) in acetonitrile (20 mL) were added sodium iodide (32.7 mg, 0.218 mmol) and potassium carbonate (753.2 mg, 5.45 mmol), the reaction mixture was heated to 80 °C and stirred under nitrogen protection for 16 hours. The reaction mixture was cooled to room temperature, diluted by adding ethyl acetate (20 mL), and then washed with water (20 mL) and saturated sodium thiosulfate aqueous solution (20 mL) twice and saturated brine (20 mL) once. The organic phase was dried over anhydrous sodium sulfate, and concentrated under reduced pressure, and the resulting residue was purified through silica gel thin layer chromatography preparation plate (petroleum ether : ethyl acetate = 5 : 1) to obtain compound **2-c** (471 mg, yield: 77.7%).

¹H NMR (400 MHz, CDCl₃): δ 7.74 (s, 1H), 7.68-7.67 (d, *J* = 8.5 Hz, 1H), 7.61-7.60 (d, *J =* 7.5 Hz, 1H), 7.44-7.42 (d, *J* = 8.0 Hz, 1H), 7.40-7.37 (m, 1H), 7.35- 7.31 (t, 1H), 7.28-7.24 (m, 2H), 3.84-3.83 (d, *J* = 2.0 Hz, 2H), 3.81 (s, 3H), 3.79-3.77 (d, *J* = 11.0 Hz, 1H), 3.68-3.66 (d, *J* = 11.0 Hz, 1H), 2.47 (s, 3H), 1.42 (s, 3H).

### Synthesis of compound 2-b

To a mixed solution of compound **2-c** (471 mg, 0.848 mmol) and compound **1-d** (423.3 mg, 1.017 mmol) in 1,4-dioxane (20 mL) and water (1 mL) were added [1,1'-bis(diphenylphosphino)ferrocene]dichloropalladium (73.4 mg, 0.0848 mmol) and sodium carbonate (224.7 mg, 2.12 mmol), the reaction mixture was heated to 80 °C and stirred under nitrogen protection for 16 hours. The reaction mixture was cooled to room temperature, diluted by adding ethyl acetate solution (20 mL), and then washed with water (20 mL) three times and saturated brine (20 mL) once. The organic phase was dried over anhydrous sodium sulfate, and concentrated under reduced pressure, and the residue was purified through silica gel thin layer chromatography preparation plate (petroleum ether : ethyl acetate = 1 : 1) to obtain compound **2-b** (459 mg, yield: 77.9%). LC-MS (ESI): m/z = 696.0 [M+H]⁺.

### Synthesis of compound 2-a

At room temperature, to a mixed solution of compound **2-b** (459 mg, 0.66 mmol) and aminoethanol (80.6 mg, 1.32 mmol) in methanol (10 mL) and dichloromethane (10 mL) was added glacial acetic acid (79.3 mg, 1.32 mmol), the reaction mixture was stirred for 1 hour, then sodium cyanoborohydride (207.4 mg, 3.30 mmol) was added thereto and stirred for 16 hours. The reaction mixture was concentrated under reduced pressure, the residue was dissolved with ethyl acetate (20 mL), and then washed with water (20 mL) and saturated brine (20 mL) once respectively. The organic phase was dried over anhydrous sodium sulfate, and concentrated under reduced pressure, and the residue was purified through silica gel thin layer chromatography preparation plate (dichloromethane : methanol = 10 : 1) to obtain compound **2-a** (32 mg, yield: 6.6%). LC-MS (ESI): m/z = 741.0 [M+H]⁺.

### Synthesis of compound 2

At room temperature, to a mixed solution of compound **2-a** (100 mg, 0.27 mmol) in methanol (10 mL) and water (10 mL) was added sodium hydroxide (32.4 mg, 0.54 mmol), and stirring was continued for 16 hours. The reaction mixture was concentrated under reduced pressure, the residue was diluted with water (20 mL), and pH was adjusted to 4 to 5 using diluted hydrochloric acid (1.0 M), with white precipitate being separated out. The precipitate was filtered, and the filter cake was washed with water (5 mL), then dried in a vacuum to obtain compound **2** (30 mg, yield: 98%). LC-MS (ESI): m/z = 727.0 [M+H]⁺.

¹H NMR (400 MHz, CD₃OD): δ 8.20-8.16 (d, *J* = 19.0 Hz, 2H), 7.85-7.81 (m, 2H), 7.69-7.59 (m, 6H), 7.42-7.33 (m, 4H), 7.15-7.12 (m, 2H), 4.41 (s, 2H), 4.39-4.30 (m, 2H), 4.05-4.02 (d, *J=* 12.5 Hz, 1H), 3.88-3.85 (m, 3H), 3.25-3.23 (m, 2H), 2.21- 2.20 (d, *J*= 4.0 Hz, 6H), 1.59 (s, 3H).

### Example 3

(2*S*)-2-[({3-[(E)-2-(3-{3-[(E)-2-[5-({[(1*S*)-1-carboxyl-2-hydroxy-1-methylethyl]amino}methyl)-2-(trifluoromethyl)phenyl]ethenyl]-2-methylphenyl}-2-methylphenyl)ethenyl]-4-(trifluoroinethyl)phenyl}methyl)amino]-3-hydroxy-2-methylpropanoic acid **3**

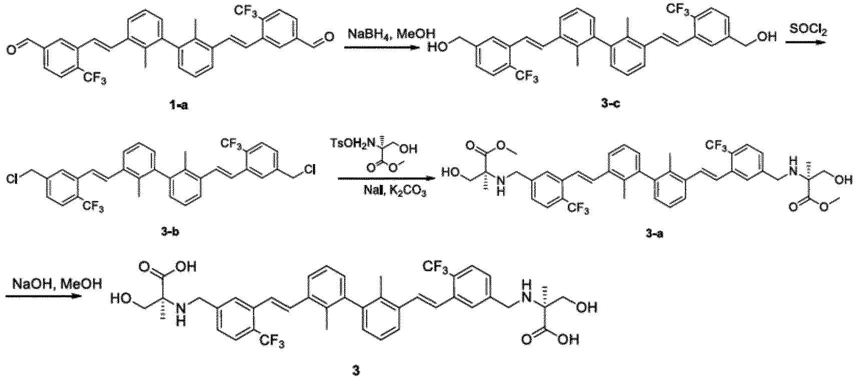

### Synthesis of compound 3-c

At room temperature, to a mixed solution of compound **1-a** (381 mg, 0.659 mmol) in tetrahydrofuran (10 mL) and methanol (10 mL) was added sodium borohydride (124.6 mg, 3.293 mmol), and the reaction mixture was stirred at room temperature for 16 hours. The reaction mixture was concentrated under reduced pressure, the residue was dissolved with ethyl acetate (20 mL), and then washed with water (20 mL) and saturated brine (20 mL) once respectively. The organic phase was dried over anhydrous sodium sulfate, and concentrated under reduced pressure, and the residue was purified through silica gel column chromatography (petroleum ether : ethyl acetate = 10 : 1 to 5 : 1) to obtain compound **3-c** (367 mg, yield: 96.3%).

### Synthesis of compound 3-b

At room temperature, to a solution of compound **3-b** (367 mg, 0.63 mmol) in dichloromethane (20 mL) were added thionyl chloride (374.7 mg, 3.15 mmol) and 2 drops of N,N-dimethylformamide, and the reaction mixture was stirred for 16 hours. The reaction mixture was concentrated under reduced pressure, and the resulting oil was dissolved with ethyl acetate (20 mL), washed with saturated sodium bicarbonate aqueous solution (20 mL) twice, saturated brine (20 mL) once, and the resulting organic phase was dried over anhydrous sodium sulfate, concentrated under reduced pressure to obtain compound **3-b** (333 mg, yield: 85.4%) which was directly used in the next reaction.

### Synthesis of compound 3-a

To a solution of compound **3-b** (333 mg, 0.538 mmol) and (*S*)-2-methylserine methyl ester p-toluenesulfonate (328.3 mg, 1.075 mmol) in acetonitrile (20 mL) were added potassium carbonate (743.6 mg, 5.38 mmol) and sodium iodide (40.3 mg, 0.269 mmol), the reaction mixture was heated to 80 °C and stirred under nitrogen protection for 16 hours. The reaction mixture was cooled to room temperature, diluted by adding ethyl acetate (20 mL), and then washed with water (20 mL) and saturated sodium thiosulfate aqueous solution (20 mL × 2) and with saturated brine (20 mL) once. The organic phase was dried over anhydrous sodium sulfate, and concentrated under reduced pressure, and the resulting residue was purified through silica gel thin layer chromatography preparation plate (dichloromethane : methanol = 15 : 1) to obtain compound **3-a** (360 mg, yield: 82.6%). LC-MS (ESI): m/z = 813.0 [M+H]⁺.

### Synthesis of compound 3

At room temperature, to a mixed solution of compound **3-a** (360 mg, 0.443 mmol) in methanol (20 mL) and water (2 mL) was added sodium hydroxide (88.6 mg, 2.215 mmol), and stirring was continued for 16 hours. The reaction mixture was concentrated under reduced pressure, the residue was diluted with water (20 mL), and pH was adjusted to 4 to 5 using diluted hydrochloric acid (1.0 M), with white precipitate being separated out. The precipitate was filtered, and the filter cake was washed with water (5 mL), then dried in a vacuum to obtain compound **3** (264 mg, yield: 74.8%). LC-MS (ESI): m/z = 785.0 [M+H]⁺.

¹H NMR (400 MHz, CD₃OD): δ 8.19 (s, 2H), 7.82-7.81 (d, *J*= 8.5 Hz, 2H), 7.69-7.61 (m, 6H), 7.41-7.38 (m, 2H), 7.35-7.32 (m, 2H), 7.14-7.13 (d, *J* = 7.5 Hz, 2H), 4.39-4.31 (m, 4H), 4.06-4.03 (d, *J=* 12.5 Hz, 2H), 3.88-3.86 (d, *J=* 12.5 Hz, 2H), 2.20 (s, 6H), 1.60 (s, 6H).

### Example 4

2-[({3-[(*E*)-2-(3-{3-[(*E*)-2-(5-{[(2-methoxyethyl)amino]methyl}-2-(trifluoromethyl)phenyl)ethenyl]-2-chlorophenyl}-2-methylphenyl)ethenyl]-4-(trifluoromethyl)phenyl}methyl)amino]-1-ethanol **4**

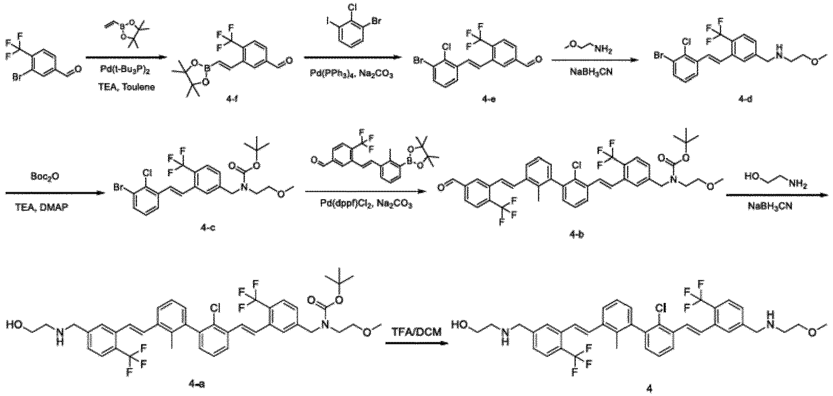

### Synthesis of compound 4-f

3-bromo-4-trifluoromethylbenzaldehyde (4.0 g, 15.8 mmol) and ethene borate (2.92 g, 18.9 mmol) were dissolved in toluene (100 mL), and bis(tri-tert-butylphosphine)palladium (807 mg, 1.58 mmol) and triethylamine (4.78 g, 47.4 mmol) were added to the solution. The reaction mixture was stirred at 80°C under nitrogen protection for 5 hours, and thin layer chromatography was used to monitor the complete reaction of the raw material. The reaction was concentrated under reduced pressure, and the residue was purified through silica gel column chromatography (petroleum ether: ethyl acetate = 50 to 10: 1), to obtain brown viscous substance **4-f** (300 mg, yield: 58%).

### Synthesis of compound 4-e

Compound 1-bromo-2-chloro-3-iodobenzene (2.0 g, 8.2 mmol) and compound **4-f** (3.0 g, 9 mmol) were dissolved in a mixed solution of dioxane and water (50 mL/5 mL), tetrakis(triphenylphosphine) palladium (947 mg, 0.82 mmol) and sodium carbonate (2.60 g, 24.6 mmol) were added to the solution. The reaction mixture was stirred at 70 °C under nitrogen protection for 12 hours, and thin layer chromatography was used to monitor the complete reaction of the raw material. The reaction was concentrated under reduced pressure, and the residue was purified through silica gel column chromatography (petroleum ether : ethyl acetate = 100 to 10 : 1), to obtain white solid **4-e** (1.15 g, yield: 36%).

### Synthesis of compound 4-d

Compound **4-e** (760 mg, 2.0 mmol) and 2-methoxyethylamine (450 mg, 6.0 mmol) were dissolved in a mixed solution of dichloromethane (30 ml) and MeOH (10 ml), glacial acetic acid (360 mg, 6.0 mmol) was added to the reaction mixture at ambient temperature, after the reaction mixture was stirred for 2 hours, sodium cyanoborohydride (152 mg, 4.0 mmol) was added to the reaction mixture, stirring was continued for 18 hours, and thin layer chromatography was used to monitor the complete reaction of the raw material. The reaction was concentrated under reduced pressure, the residue was dissolved in dichloromethane (80 mL), washed with water (50 mL) and saturated brine (30 mL), and dried over anhydrous sodium sulfate. The reaction was concentrated under reduced pressure, and the residue was purified through silica gel column chromatography (petroleum ether : ethyl acetate = 20 to 5 : 1) to obtain brown solid **4-d** (700 mg, yield: 78%).

¹H NMR (400 MHz, CD₃OD) δ: 7.79 (s, 1H), 7.66-7.60 (m, 3H), 7.49 (s, 1H), 7.42-7.40 (m, 2H), 7.19 (t, *J* = 6.0 Hz, 1H), 3.94 (s, 2H), 3.56 (t, *J* = 4.0 Hz, 2H), 3.39 (s, 3H), 2.86 (t, *J* = 4.0 Hz, 2H).

### Synthesis of compound 4-c

Compound **4-d** (350 mg, 0.78 mmol) and di-tert-butyl dicarbonate (340 mg, 1.56 mmol) were dissolved in dichloromethane (30 ml), triethylamine (315.1 mg, 3.12 mmol) and 4-dimethylaminopyridine (9.5 mg, 0.078 mmol) were added successively, and the reaction mixture was stirred overnight at ambient temperature. The reaction system was diluted by adding dichloromethane (60 mL), then washed with water (50 ml) and saturated brine (40 ml) successively, and dried over anhydrous sodium sulfate. The reaction was concentrated under reduced pressure, and the residue was purified through silica gel column chromatography (petroleum ether : ethyl acetate = 20 to 10 : 1), to obtain yellow oil **4-c** (400 mg, yield: 93%).

¹H NMR (400 MHz, CDsOD): δ 7.69-7.60 (m, 4H), 7.50-7.46 (m, 1H), 7.48-7.42 (m, 1H), 7.32-7.27 (m, 1H), 7.19 (t, *J* = 6.4 Hz, 1H), 4.62 (d, *J* = 9.2 Hz, 2H), 3.57-3.40 (m, 4H), 3.34 (s, 3H), 1.54-1.39 (m, 9H).

### Synthesis of compound 4-b

Compound **4-c** (165 mg, 0.3 mmol) and compound **1-d** (137 mg, 0.33 mmol) were dissolved in a mixed solution of dioxane and water (40 mL/4 mL), [1,1'-bis(diphenylphosphino)ferrocene]dichloropalladium (44 mg, 0.06 mmol) and sodium carbonate (127 mg, 1.2 mmol) were added to the solution. The reaction mixture was stirred at 85 °C under nitrogen protection for 12 hours. The reaction was concentrated under reduced pressure, and the residue was purified through silica gel column chromatography (petroleum ether : ethyl acetate = 20 to 5 : 1), to obtain yellow solid **4-b** (150 mg, yield: 66%).

¹H NMR (400 MHz, CDsOD): δ 10.17(s, 1H), 8.31(s, 1H), 7.90 (s, 1H), 7.89 (s, 1H), 7.76-7.65 (m, 4H), 7.60-7.34 (m, 6H), 7.25-7.18 (m, 3H), 4.62 (d, *J* = 8.4 Hz, 2H), 3.56-3.39 (m, 4H), 3.33 (s, 3H), 2.24 (s, 3H), 1.45-1.39 (m, 9H).

### Synthesis of compound 4-a

Compound **4-b** (130 mg, 0.17 mmol) and ethanolamine (52 mg, 0.85 mmol) were dissolved in a mixed solution of dichloromethane (10 mL) and methanol (3 mL), and 1 drop of glacial acetic acid was added to the reaction mixture. After the reaction mixture was stirred at room temperature for 2 hours, sodium cyanoborohydride (21 mg, 0.33 mmol) was added to the reaction mixture, and then the reaction was continued for 12 hours at room temperature. The reaction was concentrated under reduced pressure, and the residue was purified through silica gel column chromatography (dichloromethane : methanol = 20 to 10 : 1), to obtain yellow oil **4-a** (130 mg, yield: 94%).

### Synthesis of compound 4

Compound **4-a** (130 mg, 0.16 mmol) was dissolved in dichloromethane (20 mL), trifluoroacetic acid (1 mL) was added dropwise, the temperature was kept at 30 °C, and the reaction was stirred for 5 hours. The reaction was concentrated under reduced pressure, and the residue was purified through high performance liquid chromatography preparation to obtain white solid **4** (65 mg, yield: 58%). LC-MS (ESI): m/z = 703.2[M-1]⁺.

¹H NMR (400 MHz, CDsOD): δ 8.14 (d, *J* = 4.8 Hz, 1H), 7.87-7.74 (m, 4H), 7.66-7.47 (m, 6H), 7.42-7.34 (m, 2H), 7.30 (d, *J* = 10.6 Hz, 1H), 7.16 (d, *J* = 6.4 Hz, 1H), 4.40 (s, 2H), 4.39 (s, 2H), 3.87-3.85 (m, 2H), 3.71-3.69 (m, 2H),3.43 (s, 3H), 3.33-3.30 (m, 2H), 3.24-3.22 (m, 2H), 2.23 (s, 3H).

### Example 5

(2S)-3-hydroxy-2-[({3-[(E)-2-(3-{3-[(E)-2-(5-{[(2-methoxyethyl)amino]methyl}-2-(trifluoromethyl)phenyl)ethenyl]-2-chlorophenyl}-2-methylphenyl)ethenyl]-4-(trifluoromethyl)phenyl}methyl)amino]-2-methylpropanoic acid **5**

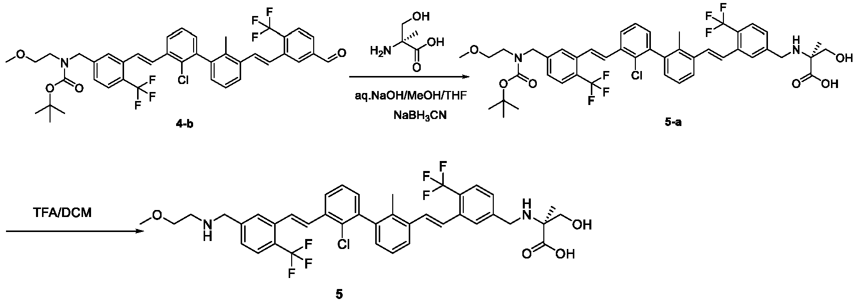

### Synthesis of compound 5-a

Compound (S)-2-amino-3-hydroxy-2-methylpropanoic acid (72 mg, 0.60 mmol) was added to methanol (20 ml), stirred at room temperature, sodium hydroxide (24 mg, 0.6 mmol) aqueous solution (5 mL) was added slowly, and the reaction system gradually became clear. After being continuously stirred for 1 hour, a solution of compound **4-b** (150 mg, 0.2 mmol) in THF (5 ml) was added to the reaction system. After completing the dropwise-addition, stirring was continued overnight. Sodium cyanoborohydride (25.2 mg, 0.4 mmol) was added to the system and stirred for 2 hours. The reaction was concentrated under reduced pressure, and the residue was washed with water (3 mL) to obtain crude product **5-a** (160 mg). It was directly used for the next reaction. LC-MS (ESI): m/z = 861[M+H]⁺.

### Synthesis of compound 5

Compound **5-a** (160 mg) was dissolved in dichloromethane (15 mL), trifluoroacetic acid (1 mL) was added dropwise, and stirred at 30 °C for 5 hours. TCL was used to monitor the complete reaction of the raw material. The reaction was concentrated under reduced pressure, and the residue was purified through high performance liquid chromatography preparation to obtain white solid **5** (31 mg, yield: 20%). LC-MS (ESI): m/z = 761.2[M+H]⁺.

¹H NMR (400 MHz, CDsOD): δ 8.17 (s, 1H), 7.97 (s, 1H), 7.80-7.61 (m, 7H), 7.53-7.33 (m, 5H), 7.27 (d, *J* = 6.0 Hz, 1H), 7.15 (d, *J* = 5.6 Hz, 1H), 4.30-4.22 (q, 2H), 3.98-3.96 (m, 3H), 3.82 (d, *J* = 9.6 Hz, 1H), 3.56 (t, *J* = 4.4 Hz, 2H), 3.37 (s, 3H), 2.85 (t, *J* = 4.4 Hz, 2H), 2.23 (s, 3H), 1.53(s, 3H).

### Example 6

(2*S*)-3-hydroxy-2-[({3-[(*E*)-2-(3-{3-[(*E*)-2-(5-{[(2-hydroxyethyl)amino]methyl}-2-(trifluoromethyl)phenyl)ethenyl]-2-chlorophenyl}-2-methylphenyl)ethenyl]-4-(trifluoromethyl)phenyl}methyl)amino]-2-methylpropanoic acid **6**

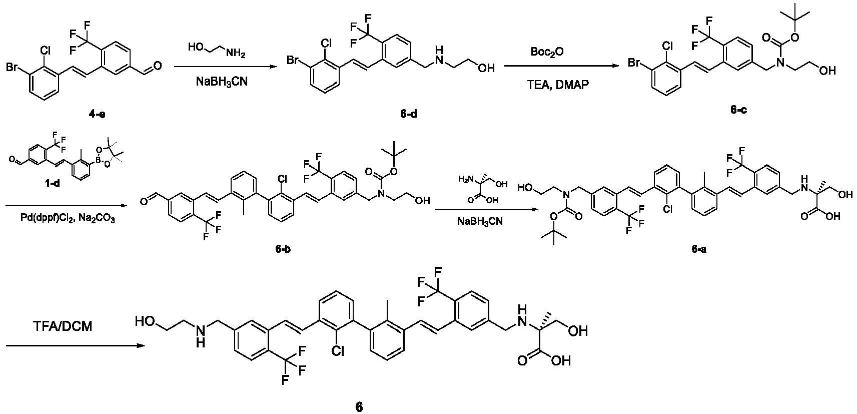

### Synthesis of compound 6-d

Compound **4-e** (450 mg, 1.15 mmol) and 2-aminoethanol (211 mg, 3.47 mmol) were dissolved in a mixed solution of dichloromethane (30 mL) and methanol (10 mL), glacial acetic acid (0.1 mL) was added, the reaction mixture was stirred at room temperature for 2 hours, then sodium cyanoborohydride (114 mg, 2.30 mmol) was added, and the reaction was continued to be stirred for 18 hours after addition. The reaction was concentrated under reduced pressure, the residue was dissolved in dichloromethane (50 mL), washed with water (50 mL) and saturated brine (30 mL) successively, and dried over anhydrous sodium sulfate. The reaction was concentrated under reduced pressure, and the residue was purified through silica gel column chromatography (methanol : ethyl acetate = 1 to 20 : 100), to obtain white solid **6-d** (430 mg, yield: 86%).

¹H NMR (400 MHz, CDsOD): δ 7.77 (s, 1H), 7.67-7.60 (m, 3H), 7.52-7.38 (m, 3H), 7.19 (t, *J* = 6.4 Hz, 1H), 3.95 (s, 2H), 3.74 (t, *J* = 4.0 Hz, 2H), 2.88 (t, *J* = 4.0 Hz, 2H).

### Synthesis of compound 6-c

Compound **6-d** (430 mg, 1.0 mmol) and di-tert-butyl dicarbonate (436 mg, 2.0 mmol) were dissolved in dichloromethane (40 mL), triethylamine (404 mg, 4.0 mmol) and 4-N,N-dimethylpyridine (122 mg, 1.0 mmol) were added successively, and the reaction mixture was stirred overnight at room temperature. The reaction system was diluted by adding dichloromethane (60 mL), then washed with water (50 mL) and saturated brine (30 mL) successively, and dried over anhydrous sodium sulfate. The reaction was concentrated under reduced pressure, and the residue was purified through silica gel column chromatography (petroleum ether : ethyl acetate = 50 to 15 : 1), to obtain yellow oil **6-c** (100 mg, yield: 18%). LC-MS (ESI): m/z = 536.0 [M+H]⁺.

### Synthesis of compound 6-b

Compound **6-c** (100 mg, 0.2 mmol) and **1-d** (100 mg, 0.24 mmol) were dissolved in a mixed solution of dioxane and water (20 mL/2 mL), then [1,1'-bis(diphenylphosphino)ferrocene]dichloropalladium (29.2 mg, 0.04 mmol) and sodium carbonate (63.6 mg, 0.6 mmol) were added to the solution. The reaction mixture was stirred at 85 °C for 10 hours. The reaction was concentrated under reduced pressure, and the residue was purified through silica gel column chromatography (petroleum ether : ethyl acetate = 10 to 2 : 1), to obtain transparent oil **6-b** (100 mg, yield: 66%). LC-MS (ESI): m/z = 744.1[M+H]⁺.

### Synthesis of compound 6-a

Compound (S)-2-amino-3-hydroxy-2-methylpropanoic acid (47.6 mg, 0.40 mmol) was added to methanol (10 ml), stirred at room temperature, sodium hydroxide (16 mg, 0.4 mmol) aqueous solution (3 ml) was added slowly, the reaction system gradually became clear, stirring was continued for 1 hour, and then a solution of **6-b** (100 mg, 0.13 mmol) in THF (4 mL) was added slowly. After completing the dropwise-addition, stirring was continued overnight. Sodium cyanoborohydride (12.6 mg, 0.2 mmol) was added to the system and stirred for 2 hours. The reaction was concentrated under reduced pressure, and the resulting residue was washed with water (3 mL) to obtain crude product **6-a** (160 mg). It was directly used for the next reaction. LC-MS (ESI): m/z = 861.0[M+H]⁺.

### Synthesis of compound 6

Compound **6-a** (160 mg) was dissolved in dichloromethane (10 mL), trifluoroacetic acid (1 mL) was added dropwise, and stirred at 30 °C for 5 hours. Thin layer chromatography was used to monitor the complete reaction of the raw material. The reaction was concentrated under reduced pressure, and the residue was purified through high performance liquid chromatography preparation to obtain white solid product **6** (9 mg, yield: 10%). LC-MS (ESI): m/z = 374.2[M/2+H]⁺.

¹H NMR (400 MHz, CDsOD): δ 8.17 (s, 1H), 8.13 (s, 1H), 7.87-7.74 (m, 4H), 7.67-7.63 (m, 4H), 7.55-7.34 (m, 4H), 7.30 (d, *J* = 6.4 Hz, 1H), 7.1 (d, *J* = 5.6 Hz, 1H), 4.42-4.38 (m, 4H), 4.10 (d, *J* = 9.2 Hz, 1H), 3.90 (d, *J* = 8.8 Hz, 1H), 3.87-3.85 (m, 2H), 3.24-3.22 (m, 2H), 2.23 (s, 3H), 1.64(s, 3H).

### Example 7

2-[({3-[(*E*)-2-(3-{3-[(*E*)-2-(5-{[(2-hydroxyethyl)amino]methyl}-2-(trifluoromethyl)phenyl)ethenyl]-2-chlorophenyl}-2-methylphenyl)ethenyl]-4-(trifluoromethyl)phenyl}methyl)amino]-1-ethanol **7**

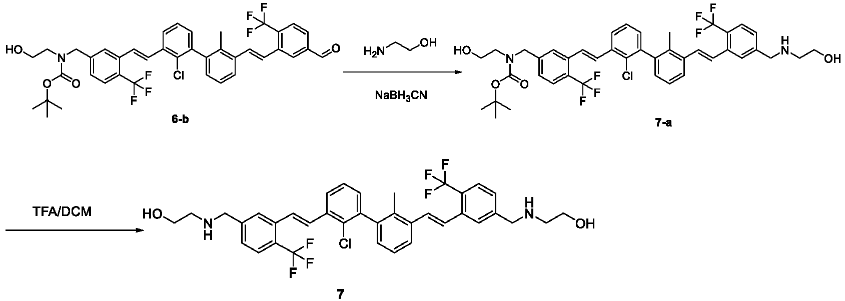

### Synthesis of compound 7-a

Compound **6-b** (168 mg, 0.2 mmol) and 2-aminoethanol (35 mg, 0.6 mmol) were dissolved in a mixed solution of dichloromethane (20 ml) and MeOH (5 ml), glacial acetic acid (0.1 mL) was added to the reaction mixture, stirred at room temperature for 2 hours, then sodium cyanoborohydride (25 mg, 0.4 mmol) was added, and the reaction was continued to be stirred for 18 hours after addition. The reaction was concentrated under reduced pressure, the residue was dissolved in dichloromethane (50 mL), washed with water (50 mL) and saturated brine (30 mL) successively, and dried over anhydrous sodium sulfate. The reaction was concentrated under reduced pressure, and the residue was purified through silica gel column chromatography (methanol: ethyl acetate = 1 to 20 : 100), to obtain colorless viscous substance **7-a** (120 mg, yield: 67%). LC-MS (ESI): m/z = 789.2[M+H]⁺.

### Synthesis of compound 6

Compound **7-a** (120 mg, 0.13 mmol) was dissolved in dichloromethane (10 mL), trifluoroacetic acid (1 mL) was added dropwise, and stirred at 30 °C for 5 hours. The reaction was concentrated under reduced pressure, and the resulting residue was purified through high performance liquid chromatography preparation to obtain white solid product **7** (40 mg, yield: 43%). LC-MS (ESI): m/z = 689.3[M+H]⁺.

¹H NMR (400 MHz, CDsOD): δ 7.98 (s, 1H), 7.96 (s, 1H), 7.76 (d, *J* = 4.2 Hz, 1H), 7.72-7.31 (m, 8H), 7.53-7.33 (m, 5H), 7.27 (d, *J* = 6.4 Hz, 1H), 7.13 (d, *J* 6.0 Hz, 1H), 3.93 (s, 4H), 3.72-3.70 (m, 4H), 2.79-2.70 (m, 4H), 2.22 (s, 3H).

### Example 8

(2S)-3-hydroxy-2-[({3-[(*E*)-2-[3-(3-(*E*)-{2-[2-fluoro-5-(2-methoxyethoxy)phenyl]ethenyl}-2-methylphenyl)-2-methylphenyl]ethenyl]-4-(trifluoromethyl)phenyl}methyl)amino]-2-methylpropanoic acid **8**

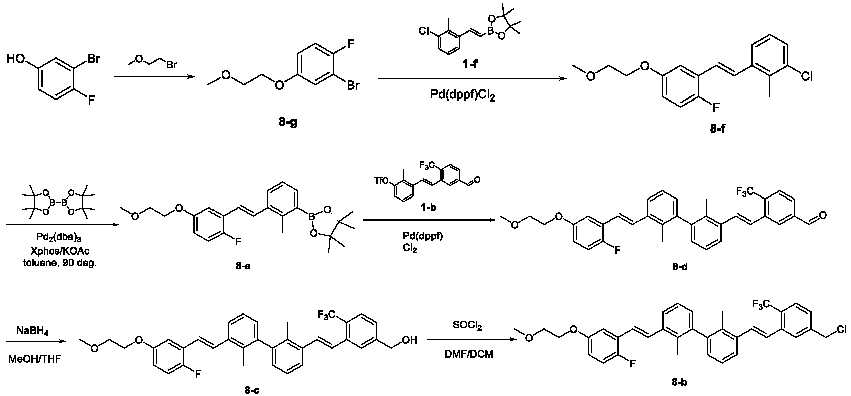

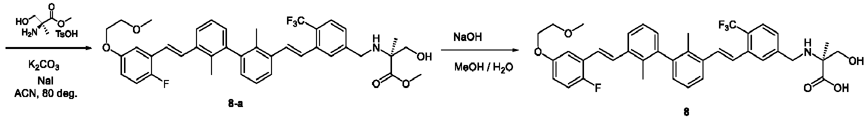

### Synthesis of compound 8-g

At ambient temperature, to a solution of 3-bromo-4-fluorophenol (1.0 g, 5.24 mmol) and 1-bromo-2-methoxyethane (800 mg, 5.76 mmol) in DMF (20 mL) were added sodium iodide (157 mg, 1.05 mmol) and potassium carbonate (3.62 g, 26.2 mmol), and the reaction mixture was stirred overnight at 80 °C. After the reaction was completed, the reaction mixture was diluted with ethyl acetate, washed with water (100 mL) and saturated brine (100 mL). The resulting organic phase was dried over anhydrous sodium sulfate, and concentrated under reduced pressure, and the residue was purified through silica gel column chromatography (petroleum ether : ethyl acetate = 50 : 1 to 10 : 1) to obtain compound **8-g** (1.263 g, yield: 96.9%).

¹H NMR (500 MHz, CDCl3): δ 7.14-7.12 (1H, dd, *J* = .0 Hz, *J* = .5 Hz), 7.07-7.03 (1H, dd, *J* = .5 Hz, *J* = 9.5 Hz), 6.88-6.85 (1H, m), 4.10-4.08 (2H, m), 3.76-3.75 (2H, m), 3.47 (3H, s).

### Synthesis of compound 8-f

To a mixed solution of **8-g** (1.263 g, 5.07 mmol) and **1-f** (1.694 g, 6.08 mmol) in 1,4-dioxane (20 mL) and water (1 mL) were added [1,1'-bis(diphenylphosphino)ferrocene]dichloropalladium (438 mg, 0.507 mmol) and sodium carbonate (1.344 g, 12.67 mmol), the reaction mixture was heated to 80 °C and reacted overnight with stirring under nitrogen protection. The reaction was concentrated under reduced pressure, and the residue was purified through silica gel column (PE : EA = 50 : 1 to 10 : 1) to obtain **8-f** (1.20 g, yield: 73.9%).

### Synthesis of compound 8-e

To a solution of **8-g** (1.20 g, 3.74 mmol) and bis(pinacolato)diboron (1.14 g, 4.49 mmol) in toluene (20 mL) were added tris(dibenzylideneacetone)dipalladium (171 mg, 0.187 mmol), 2-dicyclohexylphosphino-2',4',6'-triisopropylbiphenyl (356.6 mg, 0.748 mmol) and potassium acetate (1.101 g, 11.22 mmol), the reaction mixture was heated to 90 °C, and reacted overnight with stirring under nitrogen protection. The reaction was concentrated under reduced pressure, and the residue was purified through silica gel column chromatography (petroleum ether : ethyl acetate = 50 : 1 to 10 : 1) to obtain **8-e** (336 mg, yield: 21.7%).

¹H NMR (500 MHz, CDCl₃): δ 7.74-7.72 (d, *J* = .5 Hz, 1H), 7.68-7.67 (d, *J* = .5 Hz, 1H), 7.49-7.45 (d, *J* = 6.0 Hz, 1H), 7.25-7.20 (m, 2H), 7.08-7.05 (d, *J* = 16.5 Hz, 1H), 7.02-6.99 (t, *J* = 9.0 Hz, 1H), 6.81-6.79 (m, 1H), 4.17-4.14 (m, 2H), 3.80-3.78 (m, 2H), 3.50 (s, 3H), 2.64 (3H, s), 1.39 (s, 12H).

### Synthesis of compound 8-d

To a solution of **8-e** (336 mg, 0.815 mmol) and **1-b** (297 mg, 0.679 mmol) in ethylene glycol dimethyl ether (10 mL) were added [1,1'-bis(diphenylphosphino)ferrocene]dichloropalladium (58 mg, 0.068 mmol) and cesium fluoride (258 mg, 1.70 mmol), and the reaction mixture was stirred overnight at 80 °C under nitrogen protection. The reaction was concentrated under reduced pressure, and the residue was purified through silica gel column chromatography (petroleum ether : ethyl acetate = 20 : 1 to 5 : 1) to obtain **8-d** (100 mg, yield: 25.6%).

### Synthesis of compound 8-c

To a mixed solution of **8-d** (100 mg, 0.174 mmol) in tetrahydrofuran (5 mL) and methanol (5 mL) was added sodium borohydride (13 mg, 0.348 mmol), and the reaction mixture was stirred overnight at room temperature. The reaction was concentrated under reduced pressure, and the residue was purified through silica gel column chromatography (petroleum ether : ethyl acetate = 10 : 1 to 3 : 1) to obtain **8-c** (80 mg, yield: 80%).

¹H NMR (500 MHz, CDCl₃): δ 7.71 (s, 1H), 7.59-7.52 (m, 3H), 7.39-7.36 (d, *J* = 16.0 Hz, 1H), 7.32 (s, 2H), 7.28-7.27 (d, *J* = 8.0 Hz, 1H), 7.22-7.17 (m, 2H), 7.11-7.00 (m, 4H), 6.93-6.89 (t, *J* = 9.0 H, 1 Hz), 6.72-6.70 (m, 1H), 4.72 (s, 2H), 4.06-4.03 (m, 2H), 3.69-3.67 (m, 2H), 3.38 (s, 3H), 2.07 (s, 3H), 2.06 (s, 3H).

### Synthesis of compound 8-b

To a solution of **8-c** (80 mg, 0.139 mmol) in dichloromethane (50 mL) was added thionyl chloride (82 mg, 0.694 mmol), and the reaction mixture was stirred overnight. After the completion of the reaction, the organic solvent was dried by spin drying, and the residue was further dried with an oil pump to obtain **8-b** (82 mg, yield: 99%), which was directly used for the next reaction.

### Synthesis of compound 8-a

To a solution of **8-b** (82 mg, 0.138 mmol) and (*S*)-2-methylserine methyl ester p-toluenesulfonate (84 mg, 0.28 mmol) in acetonitrile (10 mL) were added sodium iodide (10 mg, 0.068 mmol) and K₂CO₃ (191 mg, 1.38 mmol), and the reaction mixture was stirred overnight at 80 °C under nitrogen protection. After the completion of the reaction, the solvent was dried by spin drying, the residue was dissolved with ethyl acetate (100 mL) and washed with saturated sodium thiosulfate aqueous solution (100 mL × 2) and saturated brine (100 mL × 1). The resulting organic phase was dried over anhydrous sodium sulfate and dried by spin drying to obtain a crude compound, which was purified through preparation plate (petroleum ether : ethyl acetate = 2 : 1) to obtain target compound **8-a** (23 mg, yield: 24.2%). LC-MS (ESI): m/z = 692.0 [M+H]⁺.

### Synthesis of compound 8

To a mixed solution of **8-a** (23 mg, 0.033 mmol) in methanol (10 mL) and water (1 mL) was added sodium hydroxide (6 mg, 0.15 mmol), and stirred overnight at room temperature. After the completion of the reaction, the solvent was dried by spin drying, the residue was diluted by adding water, then pH of the solution was adjusted to 4 to 5 using diluted hydrochloric acid (1 M), and a white solid was precipitated. The precipitate was filtered, and dried to obtain **8** (20 mg). LC-MS (ESI): m/z = 678.0 [M+H]⁺.

¹H NMR (500 MHz, CDsOD): δ 8.22 (1H, s), 7.84-7.82 (d, *J*= 8.5 Hz, 1H), 7.70-7.56 (m, 5H), 7.41-7.25 (m, 4H), 7.17-7.03 (m, 4H), 6.89-6.87 (m, 1H), 4.43 (s, 2H), 4.16-4.10 (m, 4H), 3.77-3.76 (m, 2H), 3.45 (s, 3H), 2.19 (s, 3H), 2.15 (s, 3H), 1.69 (s, 3H).

### Example 9

2-({[(3-(*E*)-2-{3-[3-(3-{[(2-hydroxyethyl)amino]methyl}phenyl)-2-methylphenyl]-2-methylphenyl}ethenyl)-4-(trifluoromethyl)phenyl]methyl}amino)-1-ethanol **9**

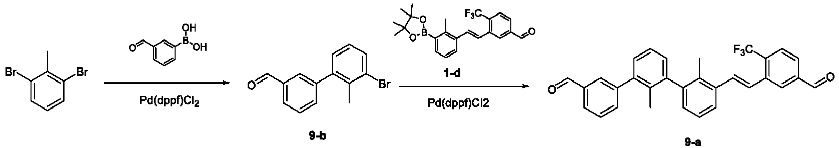

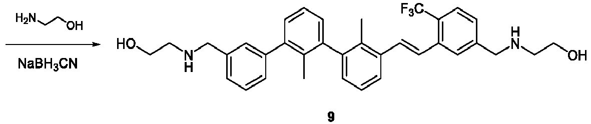

### Synthesis of compound 9-b

To a mixed solution of 3-formylphenylboric acid (1.0 g, 6.67 mmol) and 2,6-dibromotoluene (2.5 g, 10.0 mmol) in 1,4-dioxane (30 mL) and water (1.5 mL) were added [1,1'-bis(diphenylphosphino)ferrocene]dichloropalladium (577 mg, 0.667 mmol) and sodium carbonate (1.768 g, 16.675 mmol), the reaction mixture was heated to 80 °C under nitrogen protection and stirred overnight. The reaction was concentrated under reduced pressure, and the residue was purified through silica gel column chromatography (petroleum ether : ethyl acetate = 50 : 1 to 10 : 1) to obtain target compound **9-b** (1.106 g, yield: 60.1%).

¹H NMR (500 MHz, CDCl₃): δ 10.0 (s, 1H), 7.83-7.81 (m, 1H), 7.73 (m, 1H), 7.55-7.47 (m, 3H), 7.11-7.09 (dd, *J* = 1.0 Hz, *J* = 7.5 Hz, 1H), 7.06-7.03 (t, *J* = 8.0 Hz, 1H), 2.23 (s, 3H).

### Synthesis of compound 9-a

To a solution of **9-b** (1.106 g, 4.02 mmol) and **1-d** (2.00 g, 4.8 mmol) in ethylene glycol dimethyl ether (20 mL) were added [1,1'-bis(diphenylphosphino)ferrocene]dichloropalladium (348 mg, 0.402 mmol) and cesium fluoride (1.527 g, 10.05 mmol), and the reaction mixture was stirred overnight at 80 °C under nitrogen protection. The reaction was concentrated under reduced pressure, and the residue was purified through silica gel column chromatography (petroleum ether : ethyl acetate = 10 : 1 to 3 : 1) to obtain **9-a** (1.69 g, yield: 86.7%).

¹H NMR (500 MHz, CDCl₃): δ10.07 (s, 1H), 10.01 (s, 1H), 8.21 (s, 1H), 7.83-7.79 (m, 4H), 7.60-7.52 (m, 3H), 7.46-7.43 (d, *J* = 15.5 Hz, 1H), 7.35-7.31 (m, 1H), 7.27-7.23 (m, 2H), 7.19-7.17 (m, 1H), 7.11-7.10 (m, 2H), 2.14 (s, 3H), 1.87 (s, 3H).

### Synthesis of compound 9

At ambient temperature, to a mixed solution of **9-a** (100 mg, 0.206 mmol) and ethanolamine (25 mg, 0.41 mmol) in methanol (10 mL) and dichloromethane (10 mL) was added glacial acetic acid (25 mg, 0.41 mmol), the reaction mixture was stirred for 1 hour, then sodium cyanoborohydride (63 mg, 1.0 mmol) was added thereto and stirred overnight at room temperature. The solvent was dried by spin drying, and the residue was purified through high performance liquid chromatography preparation to obtain target compound **9** (54 mg, yield: 45%). LC-MS (ESI): m/z = 575.0 [M+H]⁺.

¹H NMR (500 MHz, CD₃OD): δ8.14 (s, 1H), 7.85-7.83 (d, *J* = 8.0 Hz, 1H), 7.66-7.47 (m, 7H), 7.41-7.33 (m, 3H), 7.28-7.26 (dd, *J* = 1.5 Hz, *J* = 7.5 Hz, 1H), 7.17-7.14 (m, 2H), 4.41 (s, 2H), 4.34 (s, 2H), 3.88-3.84 (m, 4H), 3.24-3.22 (m, 2H), 3.20-3.18 (m, 2H), 2.22 (s, 3H), 1.96 (s, 3H).

### Example 10

2-({[(3-(*E*)-2-{3-[3-(5-{[(2-hydroxyethyl)amino]methyl}-2-fluoro-phenyl)-2-methylphenyl]-2-methylphenyl}ethenyl)-4-(trifluoromethyl)phenyl]methyl}amino)-1-ethanol 10

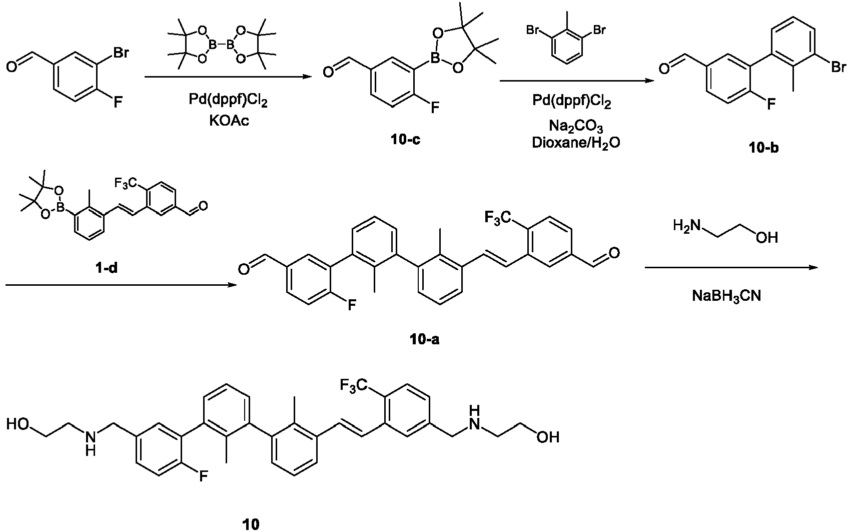

### Synthesis of compound 10-c

To a solution of 3-bromo-4-fluorobenzaldehyde (1.0 g, 4.93 mmol) and bis(pinacolato)diboron (1.5 g, 5.91 mmol) in toluene (30 mL) were added [1,1'-bis(diphenylphosphino)ferrocene]dichloropalladium (426 mg, 0.493 mmol) and potassium acetate (1.452 g, 14.79 mmol), and the reaction mixture was stirred overnight at 80 °C under nitrogen protection. The reaction was concentrated under reduced pressure, and the residue was purified through silica gel column chromatography (petroleum ether : ethyl acetate = 50 : 1 to 10 : 1) to obtain **10-c** (1.1 g, yield: 90.2%).

¹H NMR (500 MHz, CDCl₃): δ 10.00 (s, 1H), 8.32-8.31 (m, 1H), 8.04-8.01 (m, 1H), 7.22-7.19 (t, *J* = 9.0 Hz, 1H), 1.40 (s, 12H).

### Synthesis of compound 10-b

To a mixed solution of **10-c** (1.11 g, 4.439 mmol) and 2,6-dibromotoluene (1.664 g, 6.658 mmol) in 1,4-dioxane (30 mL) and water (1.5 mL) were added [1,1'-bis(diphenylphosphino)ferrocene]dichloropalladium (384 mg, 0.444 mmol) and sodium carbonate (1.177 g, 11.1 mmol), the reaction mixture was heated to 80 °C and reacted overnight with stirring under nitrogen protection. The reaction was concentrated under reduced pressure, and the residue was purified through silica gel column chromatography (petroleum ether : ethyl acetate = 50 : 1 to 10 : 1) to obtain target compound **10-b** (605 mg, yield: 46.5%).

¹H NMR (500 MHz, CDCl₃): δ 9.94 (s, 1H), 7.89-7.85 (m, 1H), 7.74-7.73 (m, 1H), 7.58-7.56 (dd, *J* = 2.0 Hz, *J* = 7.5 Hz, 1H), 7.26-7.23 (t, *J* = 9.0 Hz, 1H), 7.11-7.05 (m, 2H), 2.18 (s, 3H).

### Synthesis of compound 10-a

To a solution of **10-b** (0.605 g, 2.064 mmol) and **1-d** (1.031 g, 2.477 mmol) in ethylene glycol dimethyl ether (20 mL) were added [1,1'-bis(diphenylphosphino)ferrocene]dichloropalladium (178.2 mg, 0.206 mmol) and cesium fluoride (784 mg, 5.16 mmol), and the reaction mixture was heated to 80 °C and reacted overnight with stirring under nitrogen protection. The reaction was concentrated under reduced pressure, and the residue was purified through silica gel column chromatography (petroleum ether : ethyl acetate = 20 : 1 to 3 : 1) to obtain target compound **10-a** (0.587 g, yield: 56.4%).

¹H NMR (500 MHz, CDCl₃): δ 10.08 (s, 1H), 9.96 (s, 1H), 8.21 (s, 1H), 7.88-7.80 (m, 4H), 7.56-7.55 (d, *J* = 7.5 Hz, 1H), 7.46-7.43 (d, *J* = 16.5 Hz, 1H), 7.35-7.31 (m, 1H), 7.29-7.23 (m, 3H), 7.16-7.11 (m, 3H), 2.13 (s, 3H), 1.82 (s, 3H).

### Synthesis of compound 10

To a mixed solution of **10-a** (100 mg, 0.199 mmol) and ethanolamine (24.3 mg, 0.398 mmol) in methanol (10 mL) and dichloromethane (10 mL) was added glacial acetic acid (23.9 mg, 0.398 mmol), the reaction mixture was stirred for 1 hour, then sodium cyanoborohydride (62.5 mg, 0.995 mmol) was added thereto and stirred overnight. After the completion of the reaction, the solvent was dried by spin drying, and the residue was purified through high performance liquid chromatography preparation to obtain target compound **10** (112 mg, yield: 95%). LC-MS (ESI): m/z = 593.0 [M+H]⁺.

¹H NMR (500 MHz, CDsOD): δ8.15 (s, 1H), 7.84-7.83 (d, *J*= 8.0 Hz, 1H), 7.67-7.55 (m, 5H), 7.41-7.27 (m, 5H), 7.22-7.15 (m, 2H), 4.41 (s, 2H), 4.32 (s, 2H), 3.88-3.84 (m, 4H), 3.24-3.19 (m, 4H), 2.22 (s, 3H), 1.89 (s, 3H).

### Example 11

(2*S*)-2-[([3-[(*E*)-2-(3-[3-[5-([[(1*S*)-1-carboxyl-2-hydroxy-1-methylethyl]amino}methyl)-2-fluorophenyl]-2-methylphenyl}-2-methylphenyl)ethenyl]-4-(trifluoromethyl)phenyl}methyl)amino]-3-hydroxy-2-methylpropanoic acid **11**

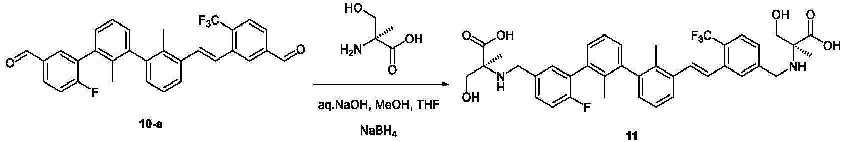

### Synthesis of compound 11

(*S*)-2-methylserine (189.6 mg, 1.592 mmol) was suspended in methanol (5 mL), and a solution of sodium hydroxide (63.7 mg, 1.592 mmol) dissolved in 5 mL of water was added dropwise slowly. The reaction mixture was stirred until it was clear, then a solution of **10-a** (100 mg, 0.199 mmol) in tetrahydrofuran (5 mL) was added, the reaction was stirred overnight, and then sodium borohydride (3 mg, 1.0 mmol) was added and the stirring was continued for half an hour. LC/MS was used to monitor the reaction process, after the completion of the reaction, the solvent was dried by spin drying, and the residue was purified through high performance liquid chromatography preparation to obtain compound **11** (48 mg, yield: 34.1%). LC-MS (ESI): m/z = 709.0 [M+H]⁺.

¹H NMR (500 MHz, CDsOD): δ8.18 (s, 1H), 7.85-7.83 (d, *J* = 8.5 Hz, 1H), 7.67-7.57 (m, 5H), 7.42-7.29 (m, 5H), 7.22-7.16 (m, 2H), 4.44-4.38 (dd, *J* = 12.5 Hz, *J* = 4.5 Hz, 2H), 4.37-4.31 (dd, *J* = 13.0 Hz, *J* = 4.5 Hz, 2H), 4.15-4.10 (t, *J* = 12.0 Hz, 2H), 3.94-3.90 (m, 2H), 2.22 (s, 3H), 1.91 (s, 3H), 1.68 (s, 3H), 1.66 (s, 3H).

### Example 12

2-[({3-[(*E*)-2-(3-{3-[(*E*)-2-(5-{[(2-hydroxyethyl)amino]methyl}-2-(trifluoromethyl)phenyl)ethenyl]-2-(fluoromethyl)phenyl}-2-methylphenyl)ethenyl]-4-(trifluoromethyl)phenyl}methyl)amino]-1-ethanol **12**

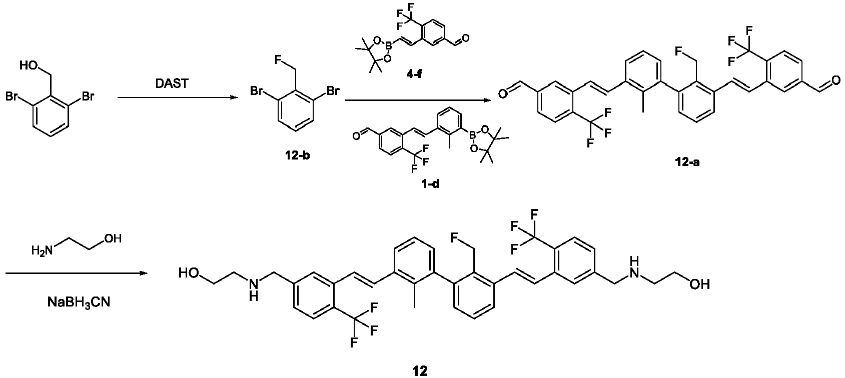

### Synthesis of compound 12-b

2,6-Dibromobenzyl alcohol (2.40 g, 9.06 mmol) was suspended in dry dichloromethane (60 mL), diethylaminosulfur trifluoride (1.90 mg, 11.8 mmol) was added dropwise at -78 °C slowly, and then continued to be stirred at -78 °C for 1 hour after the addition. TLC was used to monitor the completion of reaction. The reaction mixture was added slowly into saturated sodium bicarbonate aqueous solution (150 mL), the organic phase was separated, and the aqueous phase was extracted with dichloromethane (30 mL × 2). The organic phase was combined, washed with saturated brine (30 mL), and dried over anhydrous sodium sulfate. The reaction was concentrated under reduced pressure, and the residue was purified through silica gel column chromatography (eluent: petroleum ether) to obtain white solid product **12-b** (1.86 g, yield: 78%).

### Synthesis of compound 12-a

To 100 mL of three-necked flask were added **12-b** (268 mg, 1.0 mmol), **4-f** (391 mg, 1.2 mmol), **1-d** (541 mg, 1.3 mmol), cesium fluoride (600 mg, 4.0 mmol), potassium phosphate (848 mg, 4.0 mmol), [1,1'-bis(diphenylphosphino)ferrocene]dichloropalladium (120 mg, 0.16 mmol) and toluene (25 mL). The reaction mixture was reacted under nitrogen protection with stirring at 80 °C for 6 hours. The reaction was concentrated under reduced pressure, and the residue was purified through silica gel column chromatography (petroleum ether : ethyl acetate = 100 : 1 to 8 : 1) to obtain yellowish solid **12-a** (306 mg, yield: 44%).

### Synthesis of compound 12

To a solution of **12-a** (150 mg, 0.25 mmol) in dichloromethane (10 mL) were added ethanolamine (61 mg, 1.0 mmol), methanol (10 mL), and glacial acetic acid (20 mg, 0.34 mmol). The reaction mixture was stirred for 1 hour, and then sodium cyanoborohydride (63 mg, 1.0 mmol) was added. After the addition, the stirring was continued for 16 hours. The solvent was removed by rotary evaporation under reduced pressure, and the residue was purified through silica gel column chromatography (dichloromethane : 7N solution of ammonia in methanol = 15 : 1) to obtain off-white solid **12** (54 mg, yield: 31%). LC-MS (ESI): m/z = 687 [M+H]⁺.

¹H NMR (400 MHz, CDsOD): δ 7.98 (s, 1H), 7.97 (s, 1H), 7.75 (d, *J* = 7.6 Hz, 1H), 7.69-7.72(m, 3H), 7.61-7.65 (m, 2H), 7.52-7.57 (m, 2H), 7.45 to 7.48 (m, 2H), 7.37 to 7.42(m, 1H), 7.31 to 7.35(m, 1H), 7.22(d, *J* = 7.6 Hz, 1H), 7.15(d, *J* = 7.2 Hz, 1H), 5.36 to 5.45 (m, 1H), 5.24 to 5.33 (m, 1H), 3.93 (s, 2H), 3.92 (s, 2H), 3.71 (d, *J* = 5.2 Hz, 4H), 2.76-2.79 (m, 4H), 2.19 (s, 3H).

### Effect examples

The binding ability of the compounds of the present disclosure to PD-1/PD-L1 was detected using Homogeneous Time-Resolved Fluorescence (HTRF) binding test.

The purchased kit (CisBio, #64CUS000C-1) contained the reagents needed for experiments, such as PD-1, PD-L1, anti-tagl-Eu, Anti-tag2-XL665, Dilute Buffer and Detection Buffer.

### Steps of experiment

1. The compound was prepared with 100% DMSO to 10 concentrations with a concentration gradient of 3 times.
2. A solution of the compound in DMSO was added into Dilute Buffer, mixed uniformly, and then transferred into 96-well plate.
3. PD-L1 was diluted with Dilute Buffer, and then added into the above 96-well plate.
4. PD-1 was diluted with Dilute Buffer, and then added into the above 96-well plate and incubated at room temperature for 30 minutes.
5. A part of anti-tagl-Eu and a part of Anti-tag2-XL665 were added into Detection Buffer, mixed uniformly and then transferred into the above 96-well plate.
6. The mixed solution in this 96-well plate was incubated at room temperature for 1 hour to 24 hours.
7. HTRF values were read with Envision.

### Experimental results

The biological activity of the compounds of the present disclosure was determined by the above test, and the tested results are shown as below (Table 1):

**Table 1 IC₅₀ values of some compounds of the present disclosure binding to PD-1/PD-L1**

| Compounds | IC₅₀ (µM) | Compounds | IC₅₀ (µM) |
|---|---|---|---|
| **1** | 0.0061 | **2** | 0.0044 |
| **3** | 0.0032 | **4** | 0.0043 |
| **5** | 0.0023 | **6** | 0.0028 |
| **7** | 0.0026 | **8** | 0.510 |
| **9** | 0.0075 | **10** | 0.014 |
| **11** | 0.0087 | **12** | 0.0047 |

## Claims

1. A diphenyl-like compound represented by general formula **I,** a pharmaceutically acceptable salt, tautomer, mesomer, racemate, stereoisomer, or prodrug thereof: 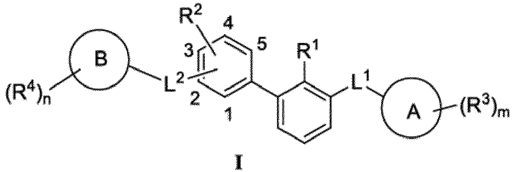 wherein,
ring A and ring B are independently aromatic ring or heteroaromatic ring;
L¹ is a chemical bond, alkynyl, -C(R⁵)=C(R⁶)- or -CR⁷R⁸-CR⁹R¹⁰-, substituted or unsubstituted cycloalkyl, substituted or unsubstituted heterocycloalkyl, substituted or unsubstituted aryl or substituted or unsubstituted heteroaryl; L² is a chemical bond, alkynyl, - C(R⁵)=C(R⁶)- or -CR⁷R⁸-CR⁹R¹⁰-, substituted or unsubstituted cycloalkyl, substituted or unsubstituted heterocycloalkyl, substituted or unsubstituted aryl or substituted or unsubstituted heteroaryl, or absent;
R⁵, R⁶, R⁷, R⁸, R⁹ and R¹⁰ are independently hydrogen, deuterium, halogen, cyano, or substituted or unsubstituted alkyl respectively;
R¹ and R² are independently deuterium, halogen, cyano, or substituted or unsubstituted alkyl;
each R³ and each R⁴ are independently hydrogen, deuterium, hydroxyl, -SR¹¹, -NR¹²R¹³, halogen, cyano, substituted or unsubstituted alkyl, substituted or unsubstituted alkoxy, -CONH₂, -COR¹⁴, -COOR¹⁵ or -OCOR¹⁶ ; R¹¹, R¹² and R¹³ are independently hydrogen, C₁-C₄ alkyl, substituted C₁-C₄ alkyl or -COR^{a}, R^{a} is hydrogen, hydroxyl, C₁-C₄ alkyl or C₁-C₄ alkoxy;
R¹⁴, R¹⁵ and R¹⁶ are independently hydrogen, C₁-C₄ alkyl or substituted C₁-C₄ alkyl; among R¹¹, R¹², R¹³, R¹⁴, R¹⁵ and R¹⁶, the substituted in the substituted C₁-C₄ alkyl refers to being substituted with one or more of C₆-C₁₄ aryl, substituted C₆-C₁₄ aryl, C₁-C₁₀ heteroaryl and substituted C₁-C₁₀ heteroaryl;
substituents in the substituted cycloalkyl, the substituted heterocycloalkyl, the substituted aryl, the substituted heteroaryl in L¹ and L², the substituted alkyl in R¹ and R², the substituted alkyl or the substituted alkoxy in each R³ and each R⁴ are selected from one or more of halogen, cyano, C₁-C₄ alkyl, hydroxyl, 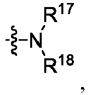 C₆-C₁₄ aryl, substituted C₆-C₁₄ aryl, C₁-C₁₀ heteroaryl, substituted C₁-C₁₀ heteroaryl, C₁-C₄ alkoxy, C₁-C₄ carboxyl, C₁-C₄ ester group and C₁-C₄ amido; in 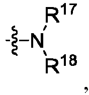
R¹⁷ and R¹⁸ are independently hydrogen, substituted or unsubstituted C₁-C₄ alkyl, substituted or unsubstituted C₆-C₁₄ aryl, substituted or unsubstituted C₃-C₆ cycloalkyl, or substituted or unsubstituted C₁-C₄ alkoxy; or R¹⁷, R¹⁸ together with the nitrogen atom to which they are attached form a substituted or unsubstituted 5 to 7-membered heterocycle; in the heterocycle, heteroatom is N, or N and O, the number of heteroatom is 1 to 4; each R¹⁷ and each R¹⁸ are identical or different;
substituents in the substituted C₁-C₄ alkyl, the substituted C₆-C₁₄ aryl, the substituted C₃-C₆ cycloalkyl, the substituted C₁-C₄ alkoxy and the substituted 5 to 7-membered heterocycle in R¹⁷ and R¹⁸ are selected from one or more of halogen, cyano, C₁-C₄ alkyl, substituted C₁-C₄ alkyl, C₆-C₁₄ aryl, substituted C₆-C₁₄ aryl, C₁-C₁₀ heteroaryl, substituted C₁-C₁₀ heteroaryl, hydroxyl, 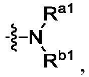 C₁-C₄ alkoxy, C₁-C₄ carboxyl, C₁-C₄ ester group and C₁-C₄ amido;
in R¹⁷ and R¹⁸, when substituents in the substituted C₁-C₄ alkyl, the substituted C₆-C₁₄ aryl, the substituted C₃-C₆ cycloalkyl, the substituted C₁-C₄ alkoxy and the substituted 5 to 7-membered heterocycle are substituted C₁-C₄ alkyl, in the substituents, substituents in the substituted C₁-C₄ alkyl are selected from one or more of halogen, cyano, C₁-C₄ alkyl, C₆-C₁₄ aryl, substituted C₆-C₁₄ aryl, C₁-C₁₀ heteroaryl, substituted C₁-C₁₀ heteroaryl, hydroxyl, 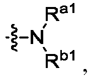 C₁-C₄ alkoxy, C₁-C₄ carboxyl, C₁-C₄ ester group and C₁-C₄ amido; in 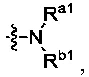 R^{a1} and R^{b1} are independently hydrogen, C₁-C₄ alkyl or 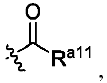 R^{a11} is C₁-C₄ alkyl;
all the above C₁-C₁₀ heteroaryl refer to C₁-C₁₀ heteroaryl in which heteroatom is selected from N, O and S and the number of heteroatom is 1 to 4;
substituents in all the above substituted C₆-C₁₄ aryl and substituted C₁-C₁₀ heteroaryl are selected from one or more of cyano, halogen, hydroxyl, C₁-C₄ alkyl and C₁-C₄ alkoxy;
when substituents are more than one, the substituents are identical or different;
m is 1, 2 or 3;
n is 1, 2 or 3.

2. The diphenyl-like compound represented by general formula I, a pharmaceutically acceptable salt, tautomer, mesomer, racemate, stereoisomer, or prodrug thereof as defined in claim 1, wherein
the aromatic ring is C₆-C₂₀ aromatic ring, preferably C₆-C₁₄ aromatic ring, more preferably C₆-C₁₀ aromatic ring, most preferably benzene, naphthalene, tetrahydronaphthalene, 2,3-dihydroindene, diphenyl, phenanthrene, anthracene or acenaphthene;
and/or, the heteroaromatic ring refers to C₁-C₁₀ heteroaromatic ring in which heteroatom is selected from O, N and S and the number of heteroatom is 1, 2, 3 or 4, preferably C₁-C₈ heteroaromatic ring in which heteroatom is selected from O, N and S and the number of heteroatom is 1, 2, 3 or 4, more preferably C₁-C₆ heteroaromatic ring in which heteroatom is selected from O, N and S and the number of heteroatom is 1, 2, 3 or 4, most preferably, acridine, carbazole, cinnoline, carboline, quinoxaline, imidazole, pyrazole, pyrrole, indole, indoline, benzotriazole, benzimidazole, furan, thiophene, isothiazole, benzothiophene, dihydrobenzothiophene, benzofuran, isobenzofuran, benzoxazole, benzofurazan, benzopyrazole, quinoline, isoindoline, isoquinoline, oxazole, oxadiazole, isoxazole, indole, pyrazine, pyridopyridine, tetrazolopyridine, pyridazine, pyridine, naphthyridine, pyrimidine, pyrrole, tetrazole, thiadiazole, thiazole, thiophene, triazole, quinazoline, tetrahydroquinoline, dihydrobenzimidazole, dihydrobenzofuran, dihydrobenzoxazole or dihydroquinoline;
and/or, the cycloalkyl is C₃-C₂₀ cycloalkyl, preferably C₃-C₁₀ cycloalkyl, more preferably C₃-C₆ cycloalkyl, most preferably, cyclopropyl, cyclobutyl, cyclopentyl, cyclohexyl, cycloheptyl, cyclooctyl, cyclodecyl and cyclododecyl or cyclohexenyl;
and/or, the heterocycloalkyl refers to C₂-C₁₀ non-aromatic ring in which heteroatom is selected from O, N and S and the number of heteroatom is 1, 2, 3 or 4, preferably C₂-C₈ heterocycloalkyl in which heteroatom is selected from O, N and S and the number of heteroatom is 1, 2, 3 or 4, further preferably C₂-C₆ heterocycloalkyl in which heteroatom is selected from O, N and S and the number of heteroatom is 1, 2, 3 or 4, most preferably, tetrahydropyranyl, azetidinyl, 1,4-dioxanyl, piperazinyl, piperidinyl, pyrrolidinyl, morpholinyl, thiomorpholinyl, dihydrofuryl, dihydroimidazolyl, indolinyl, dihydroisoxazolyl, dihydroisothiazolyl, dihydrooxadiazolyl, dihydrooxazolyl, dihydropyrazinyl, dihydropyrazolyl, dihydropyridyl, dihydropyrimidinyl, dihydropyrrolyl, dihydroquinolyl, dihydrotetrazolyl, dihydrothiadiazolyl, dihydrothiazolyl, dihydrothiophenyl, dihydrotriazolyl, dihydroazetidinyl, methylenedioxybenzoyl, tetrahydrofuryl, tetrahydrothiophenyl or N-oxides thereof;
and/or, the aryl is C₆-C₂₀ aryl, preferably C₆-C₁₄ aryl, more preferably C₆-C₁₀ aryl, most preferably phenyl, naphthyl, tetrahydronaphthyl, 2,3-dihydroindenyl, xenyl, phenanthryl, anthryl or acenaphthyl,
and/or, the heteroaryl refers to C₁-C₁₀ heteroaryl in which heteroatom is selected from O, N and S and the number of heteroatom is 1, 2, 3 or 4, further preferably C₁-C₈ heteroaryl in which heteroatom is selected from O, N and S and the number of heteroatom is 1, 2, 3 or 4, more preferably C₁-C₆ heteroaryl in which heteroatom is selected from O, N and S and the number of heteroatom is 1, 2, 3 or 4, most preferably, benzimidazolyl, benzofuryl, benzofurazanyl, benzopyrazolyl, benzotriazolyl, benzothiophenyl, benzoxazolyl, carbazolyl, carbolinyl, cinnolinyl, furyl, imidazolyl, indolinyl, indolyl, indazolyl, isobenzofuryl, isoindolinyl, isoquinolyl, isothiazolyl, isooxazolyl, naphthyridinyl, oxadiazolyl, oxazolyl, oxazoline, isoxazoline, oxetanyl, pyranyl, pyrazinyl, pyrazolyl, pyridazinyl, pyridopyridyl, pyridazinyl, pyridyl, pyrimidinyl, pyrrolyl, quinazolyl, quinolyl, quinoxalinyl, tetrazolyl, tetrazolopyridyl, thiadiazolyl, thiazolyl, thiophenyl or triazolyl;
and/or, the halogen is fluorine, chlorine, bromine or iodine;
and/or, the alkyl refers to branched and linear saturated aliphatic hydrocarbonyl comprising 1 to 20 carbon atoms, preferably 1 to 10 carbon atoms, more preferably 1 to 8 carbon atoms, most preferably, methyl, ethyl, n-propyl, isopropyl, n-butyl, tert-butyl, isobutyl, pentyl, hexyl, heptyl, octyl, nonyl, decyl, 4,4-dimethylpentyl, 2,2,4-trimethylpentyl, undecyl, dodecyl and various isomers thereof;
and/or, the alkoxy represents cyclic or non-cyclic alkyl linked via oxygen bridge with the recited carbon atom number, preferably C₁-C₄ alkoxy, more preferably methoxy, ethoxy, n-propoxy, isopropoxy or tert-butoxy;
and/or, the 5 to 7-membered heterocycle refers to 5 to 7-membered heterocycle in which heteroatom is selected from O, N and S, the number of heteroatom is 1, 2, 3 or 4 and the number of carbon atom is 1, 2, 3, 4, 5 or 6, preferably azetidinyl, piperazinyl, piperidinyl, pyrrolidinyl, morpholinyl, thiomorpholinyl, dihydroimidazolyl, indolinyl, dihydroisoxazolyl, dihydroisothiazolyl, dihydrooxadiazolyl, dihydrooxazolyl, dihydropyrazinyl, dihydropyrazolyl, dihydropyridyl, dihydropyrimidinyl, dihydropyrrolyl, dihydroquinolyl, dihydrotetrazolyl, dihydrothiadiazolyl, dihydrothiazolyl, dihydrotriazolyl or dihydroazetidinyl.

3. The diphenyl-like compound represented by general formula **I,** a pharmaceutically acceptable salt, tautomer, mesomer, racemate, stereoisomer, or prodrug thereof as defined in claim 1 or 2, wherein
L¹ is alkynyl, -C(R⁵)=C(R⁶)-, -CR⁷R⁸-CR⁹R¹⁰-, substituted or unsubstituted cycloalkyl, substituted or unsubstituted heterocycloalkyl, substituted or unsubstituted aryl, or substituted or unsubstituted heteroaryl, preferably alkynyl, -C(R⁵)=C(R⁶)- or -CR⁷R⁸-CR⁹R¹⁰-, more preferably -C(R⁵)=C(R⁶)-, most preferably -CH=CH-;
and/or, L² is alkynyl, -C(R⁵)=C(R⁶)-, -CR⁷R⁸-CR⁹R¹⁰-, substituted or unsubstituted cycloalkyl, substituted or unsubstituted heterocycloalkyl, substituted or unsubstituted aryl, or substituted or unsubstituted heteroaryl or absent, preferably alkynyl, -C(R⁵)=C(R⁶)-, -CR⁷R⁸-CR⁹R¹⁰- or absent, more preferably -C(R⁵)=C(R⁶)- or absent, most preferably -CH=CH- or absent;
and/or, R⁵, R⁶, R⁷, R⁸, R⁹ and R¹⁰ are independently hydrogen or deuterium respectively; and/or, R¹ is halogen, cyano, substituted or unsubstituted alkyl, the alkyl is preferably C₁-C₄ alkyl, more preferably methyl; substituents in the substituted alkyl are preferably halogen or hydroxy;
and/or, R² is deuterium, halogen, cyano, substituted or unsubstituted alkyl, the alkyl is preferably C₁-C₄ alkyl, more preferably methyl, ethyl, n-propyl, isopropyl, n-butyl, isobutyl or tert-butyl, substituents in the substituted alkyl are preferably one or more of halogen, cyano, C₁-C₄ alkyl, hydroxyl, C₁-C₄ alkoxy, C₁-C₄ carboxyl, C₁-C₄ ester group and C₁-C₄ amido;
and/or, R³ and R⁴ are independently deuterium, halogen, cyano, -SR¹¹, -NR¹²R¹³, substituted or unsubstituted alkyl, or substituted or unsubstituted alkoxy; preferably are independently deuterium, halogen, cyano, -SR¹¹, substituted or unsubstituted alkyl, or substituted or unsubstituted alkoxy.

4. The diphenyl-like compound represented by general formula I, a pharmaceutically acceptable salt, tautomer, mesomer, racemate, stereoisomer, or prodrug thereof as defined in at least one of claims 1 to 3, wherein
R¹ and R² are independently halogen, alkyl, or alkyl substituted with halogen;
or, R³ and R⁴ are halogen;
or, R³ and R⁴ are substituted or unsubstituted alkyl, substituents in the substituted alkyl are substituted with one or more of halogen, cyano, hydroxyl, 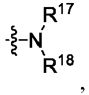 C₆-C₁₄ aryl, substituted C₆-C₁₄ aryl, C₁-C₁₀ heteroaryl, substituted C₁-C₁₀ heteroaryl, C₁-C₄ alkoxy and C₁-C₄ carboxyl, preferably are substituted with one or more of halogen, 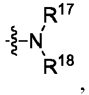
substituted C₆-C₁₄ aryl and substituted C₁-C₁₀ heteroaryl, when substituents are more than one, the substituents are identical or different,
or, R³ and R⁴ are substituted or unsubstituted alkoxy, substituents in the substituted alkoxy are substituted with one or more of halogen, cyano, hydroxyl, 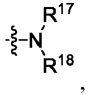 C₆-C₁₄ aryl, substituted C₆-C₁₄ aryl, C₁-C₁₀ heteroaryl, substituted C₁-C₁₀ heteroaryl and C₁-C₄ alkoxy, preferably are substituted with one or more of C₁-C₁₀ heteroaryl, substituted C₁-C₁₀ heteroaryl and C₁-C₄ alkoxy, most preferably are substituted with C₁-C₄ alkoxy; when substituents are more than one, the substituents are identical or different.

5. The diphenyl-like compound represented by general formula **I,** a pharmaceutically acceptable salt, tautomer, mesomer, racemate, stereoisomer, or prodrug thereof as defined in at least one of claims 1 to 4, wherein
R¹ and R² are independently halogen, C₁-C₄ alkyl, or C₁-C₄ alkyl substituted with one or more of F, Cl, Br and I (for example, -CH₂F, -CHF₂ or -CF₃); or, R³ and R⁴ are alkyl substituted with halogen, the alkyl substituted with halogen is C₁-C₄ alkyl preferably substituted with one or more of F, Cl, Br and I, preferably -CF₃,
or, R³ and R⁴ are alkyl substituted with 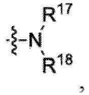 the alkyl substituted with 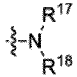 is preferably C₁-C₄ alkyl substituted with 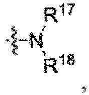 the C₁-C₄ alkyl substituted with 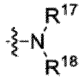 is preferably 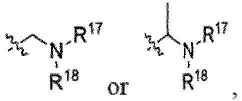 wherein, one of R¹⁷ and R¹⁸ is H, the other one is alkyl substituted with one or more of C₁-C₄ alkoxy, hydroxy and carboxyl, when R³ and R⁴ are alkyl substituted with 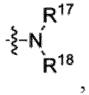 the alkyl substituted with 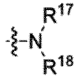 is preferably 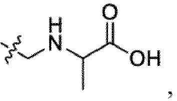 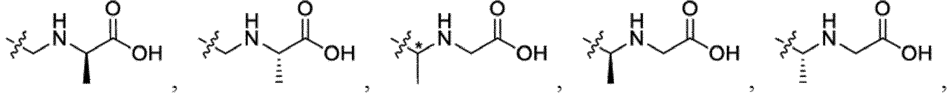 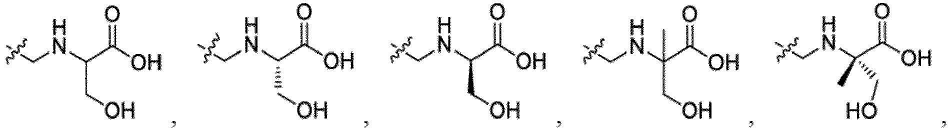 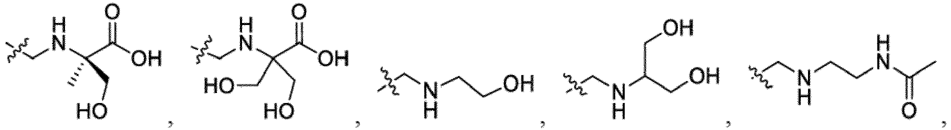 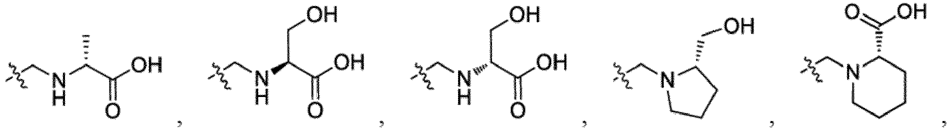 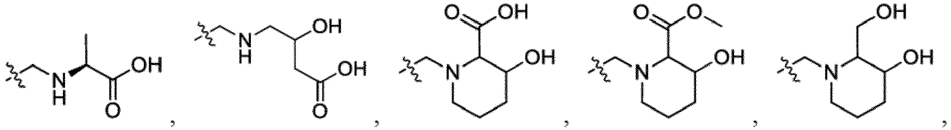 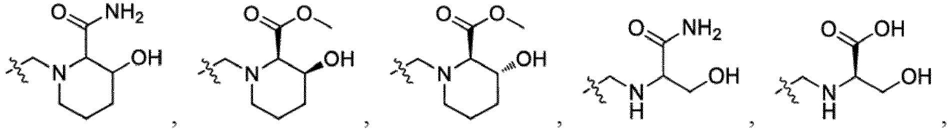 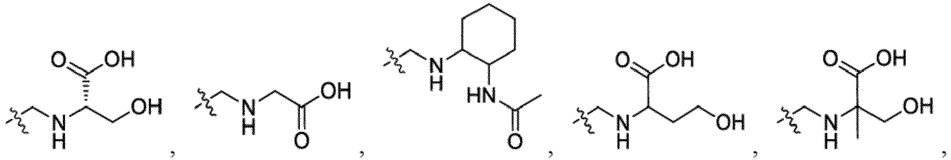 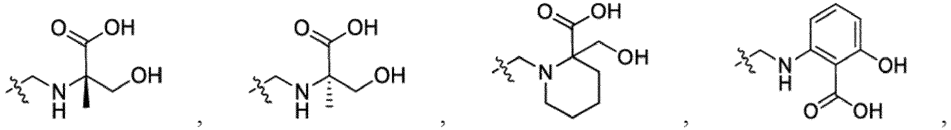 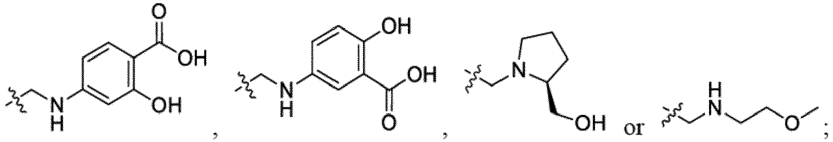
or, R³ and R⁴ is alkyl substituted with substituted C₆-C₁₄ aryl, preferably 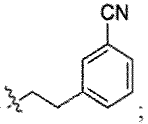
or, R³ and R⁴ is alkyl substituted with substituted C₁-C₁₀ heteroaryl, preferably 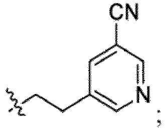
or, when R³ is substituted or unsubstituted alkyl, R³ is at meta-position or para-position of the atom linked with L¹ on ring A;
or, when R⁴ is substituted or unsubstituted alkyl, R⁴ is at meta-position or para-position of the atom linked with L² on ring B;
or, when R³ is substituted or unsubstituted alkyl, then 0, 1 or 2 additional substituents can be present on ring A, when 1 additional substituent is present, the additional substituent is at para-position, meta-position or ortho-position of the substituted or unsubstituted alkyl;
or, when R⁴ is substituted or unsubstituted alkyl, then 0, 1 or 2 additional substituents can be present on ring B, when 1 additional substituent is present, the additional substituent is at para-position, meta-position or ortho-position of the substituted or unsubstituted alkyl;
or, R³ and R⁴ are substituted or unsubstituted alkoxy, substituents in the substituted alkoxy are substituted with one or more of halogen, cyano, hydroxyl, 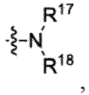 C₆-C₁₄ aryl, substituted C₆-C₁₄ aryl, C₁-C₁₀ heteroaryl, substituted C₁-C₁₀ heteroaryl and C₁-C₄ alkoxy; preferably R³ and R⁴ are substituted alkoxy, substituents in the substituted alkoxy are substituted with one or more of C₁-C₁₀ heteroaryl, substituted C₁-C₁₀ heteroaryl and C₁-C₄ alkoxy, when substituents are more than one, the substituents are identical or different; the substituted alkoxy is preferably 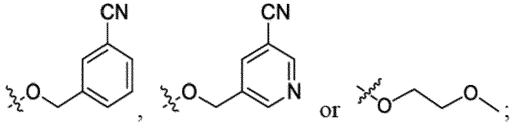
or, when R³ is substituted or unsubstituted alkoxy, R³ is at ortho-position or meta-position of the atom linked with L¹ on ring A;
or, when R⁴ is substituted or unsubstituted alkoxy, R⁴ is at ortho-position or meta-position of the atom linked with L² on ring B.

6. The diphenyl-like compound represented by general formula **I,** a pharmaceutically acceptable salt, tautomer, mesomer, racemate, stereoisomer, or prodrug thereof as defined in at least one of claims 1 to 5, wherein
group 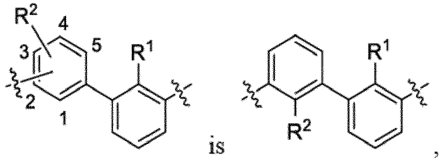 wherein the definitions of R¹ and R² are the same as defined in claim 1 or 2; group 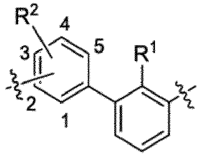 is preferably 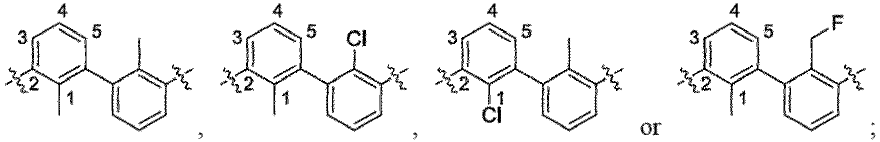 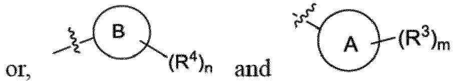 are independently 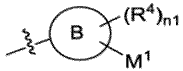 and 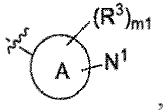 wherein M¹ and N¹ are alkyl substituted with 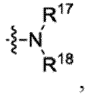 or one of M¹ and N¹ is alkyl substituted with 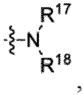 the other one is substituted alkoxy; wherein the definitions of R¹⁷, R¹⁸, R³ and R⁴ are the same as defined in any one of claims 1 to 5, n1 and m1 are independently 0, 1 or 2;
preferably, M¹ and N¹ are 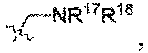 or one of M¹ and N¹ is 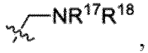 the other one is alkoxy substituted with one or more of C₁-C₄ alkoxy, C₁-C₁₀ heteroaryl and substituted C₁-C₁₀ heteroaryl; R³ and R⁴ are hydrogen, halogen, alkyl, alkyl substituted with halogen, alkoxy or substituted alkoxy, substituents in the substituted alkoxy preferably are substituted with one or more of C₁-C₄ alkoxy, C₁-C₁₀ heteroaryl and substituted C₁-C₁₀ heteroaryl; the definitions of R¹⁷ and R¹⁸ are the same as defined in any one of claims 1 to 5;
more preferably, M¹ and N¹ are 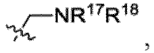 or one of M¹ and N¹ is 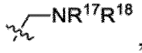 the other one is alkoxy substituted with C₁-C₄ alkoxy; R³ and R⁴ preferably are halogen, alkyl, alkyl substituted with halogen, alkoxy or alkoxy substituted with C₁-C₄ alkoxy; the definitions of R¹⁷ and R¹⁸ are the same as defined in any one of claims 1 to 5.

7. The diphenyl-like compound represented by general formula I, a pharmaceutically acceptable salt, tautomer, mesomer, racemate, stereoisomer, or prodrug thereof as defined in claim 6, wherein 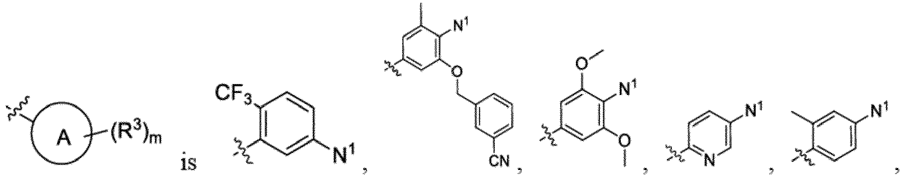 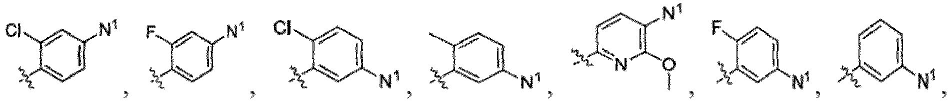 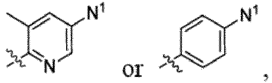 wherein N¹, R¹⁷ and R¹⁸ are the same as those defined in claim 6; 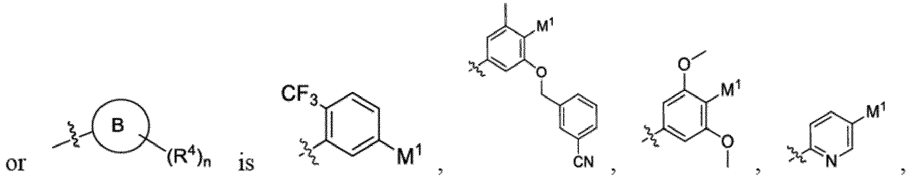 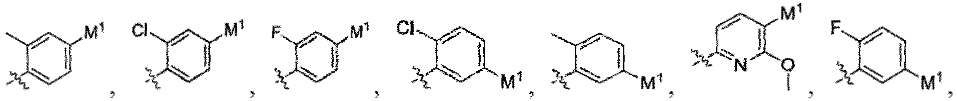 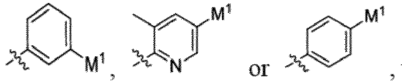 wherein the definitions of M¹, R¹⁷ and R¹⁸ are the same as defined in claim 6.

8. The diphenyl-like compound represented by general formula I, a pharmaceutically acceptable salt, tautomer, mesomer, racemate, stereoisomer, or prodrug thereof as defined in at least one of claims 1 to 7, wherein 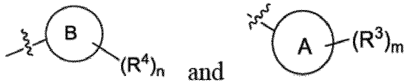 are independently 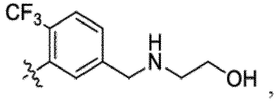 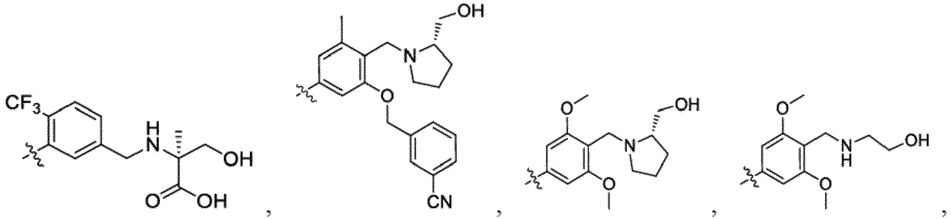 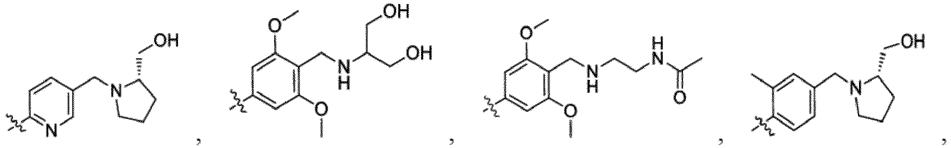 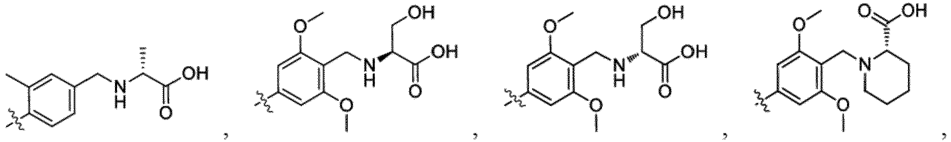 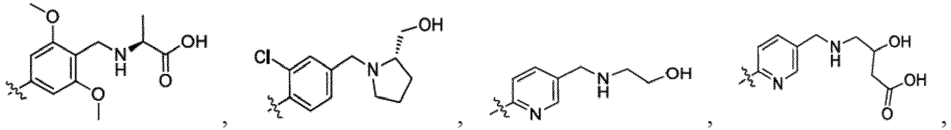 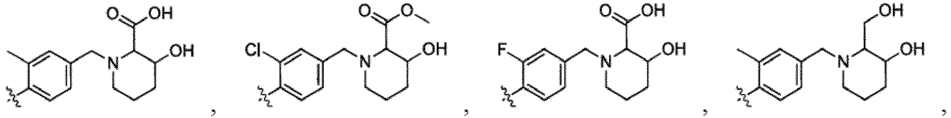 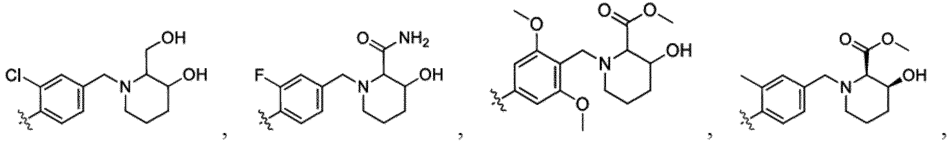 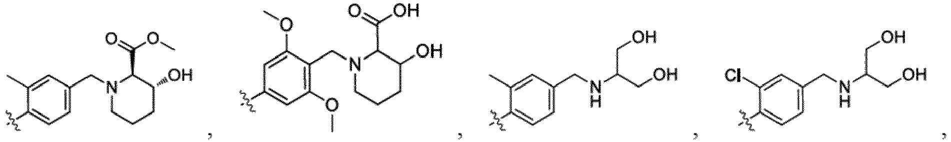 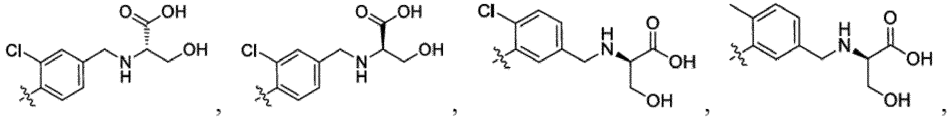 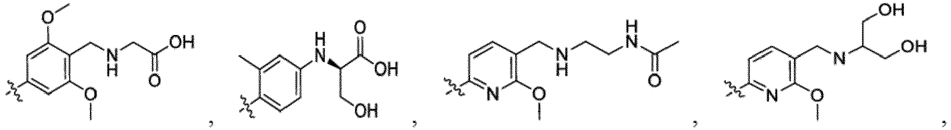 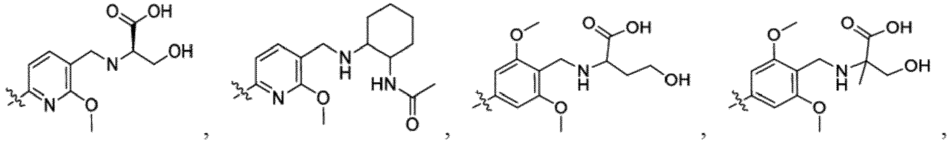 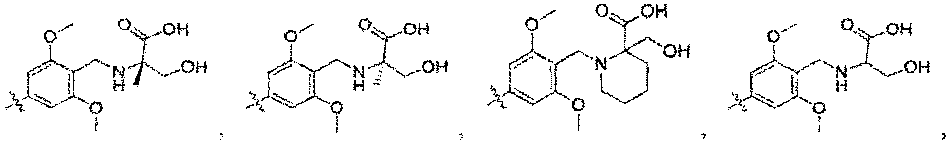 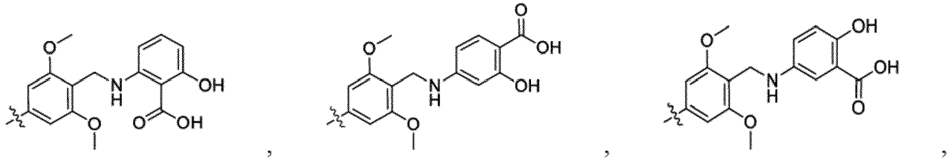 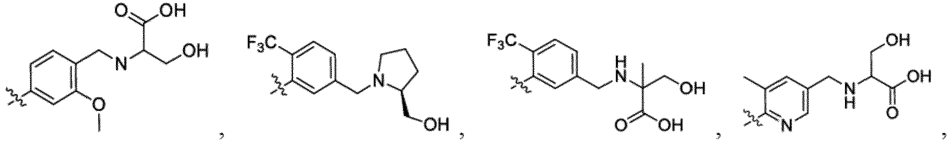 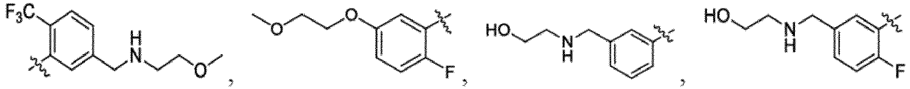 or 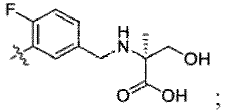 and/or, 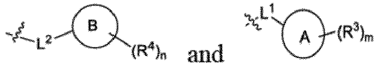 are independently

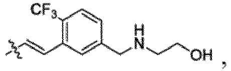

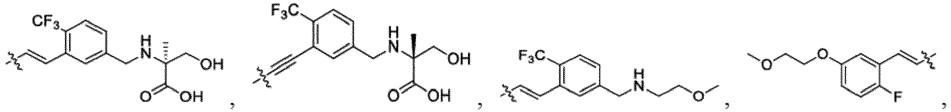

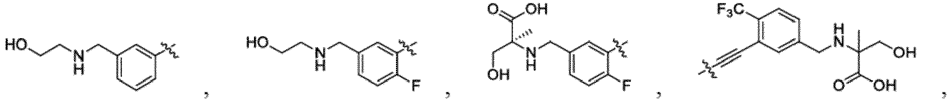

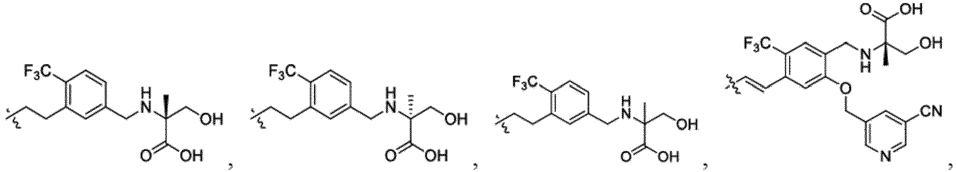

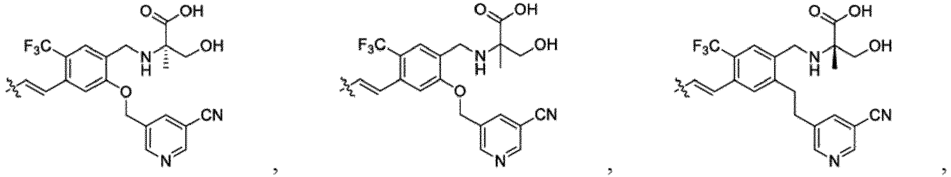

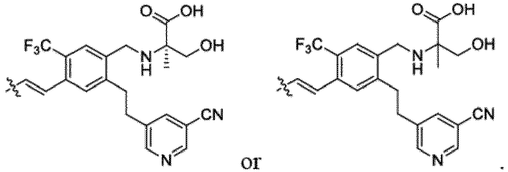

9. The diphenyl-like compound represented by general formula **I,** a pharmaceutically acceptable salt, tautomer, mesomer, racemate, stereoisomer, or prodrug thereof as defined in at least one of claims 1 to 8, wherein the diphenyl-like compound represented by general formula **I** is selected from any one compound of: 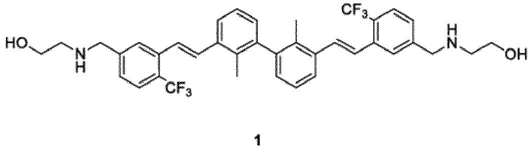 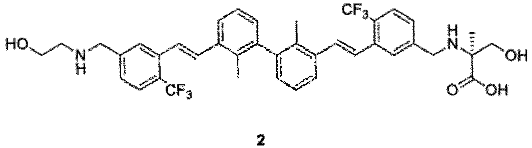 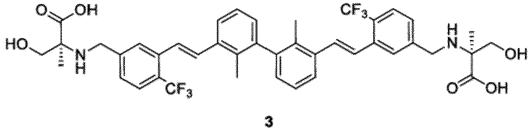 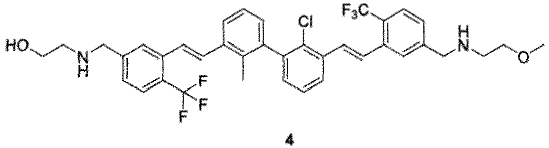 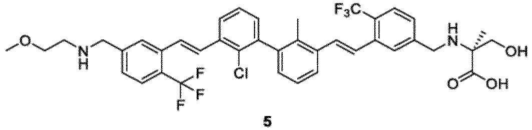 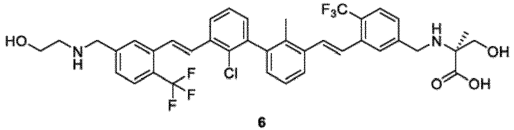 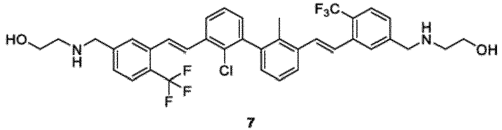 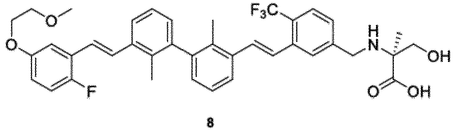 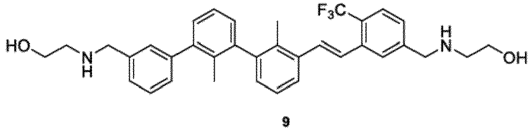 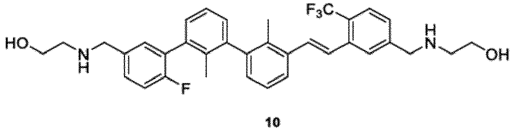 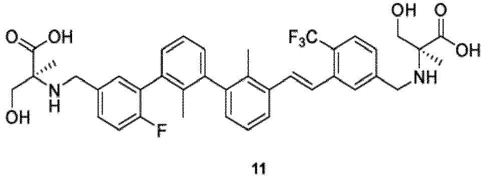 and

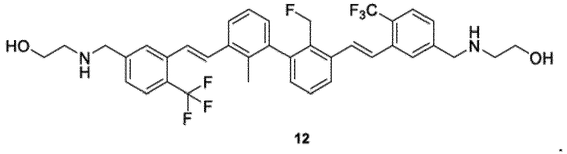

10. The diphenyl-like compound represented by general formula **I,** a pharmaceutically acceptable salt, tautomer, mesomer, racemate, stereoisomer, or prodrug thereof as defined in at least one of claims 1 to 9, wherein the diphenyl-like compound represented by general formula **I** is a diphenyl-like compound represented by general formula **I-A** or **II:** 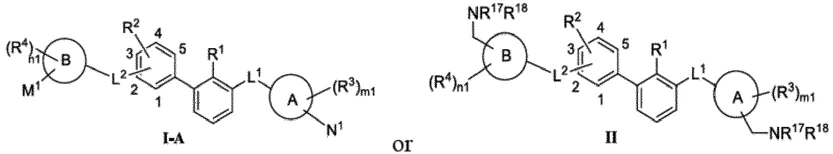 wherein, the definitions of ring A, ring B, L¹, L², R¹, R², R³, R⁴, M¹, N¹, R¹⁷ and R¹⁸ are the same as defined in any one of claims 1 to 9, nl is 0, 1 or 2, m1 is 0, 1 or 2.

11. A preparation method of the diphenyl-like compound represented by general formula **I-A** or **II** in claim 10, wherein
in the compound represented by general formula **I-A,** when -NH- or -COOH is contained in M¹ and N¹, it is prepared by using a method comprising a step of: deprotecting a compound represented by general formula **II-F** as shown below, to obtain the diphenyl-like compound represented by general formula **I-A,** 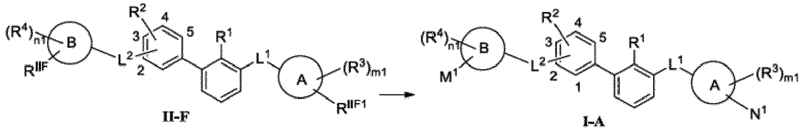
wherein the definitions of ring A, ring B, L¹, L², R¹, R², R³, R⁴, M¹ and N¹ are the same as defined in claim 10, n1 is 0, 1 or 2, m1 is 0, 1 or 2, R^{IIF} is a group containing amino or carboxyl protecting group corresponding to M¹, R^{IIF1} is identical to N¹; or R^{IIF} is identical to M¹, R^{IIF1} is a group containing amino or carboxyl protecting group corresponding to N¹; or R^{IIF} is a group containing amino or carboxyl protecting group corresponding to M¹, R^{IIF1} is a group containing amino or carboxyl protecting group corresponding to N¹;
the preparation method of the diphenyl-like compound of general formula **II** employs any one method of:
(1) method 1 comprising a step of: reacting a compound represented by general formula **II-A** with a compound **II-A1** as shown below, to obtain the diphenyl-like compound represented by general formula **II,** 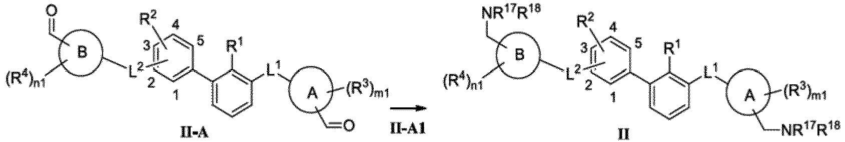 wherein the structure of the compound **II-A1** is as follows: 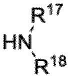 or an acid salt thereof, the definitions of ring A, ring B, L¹, L², R¹, R², R³, R⁴, R¹⁷ and R¹⁸ are the same as defined in claim 10, nl is 0, 1 or 2, m1 is 0, 1 or 2; in this method, 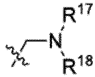 in ring A and ring B are identical;
(2) method 2 comprising a step of: reacting a compound represented by general formula **II-B** with a compound **II-B1** as shown below, to obtain the diphenyl-like compound represented by general formula **II,** 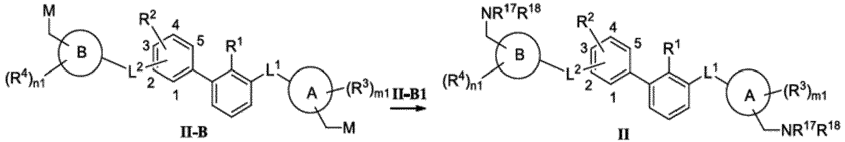 wherein the structure of the compound **II-B1** is as follows: 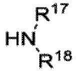 or an acid salt thereof, the definitions of ring A, ring B, L¹, L², R¹, R², R³, R⁴, R¹⁷ and R¹⁸ are the same as defined in claim 10, n1 is 0, 1 or 2, m1 is 0, 1 or 2, M is halogen; in this method, 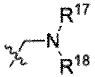 in ring A and ring B are identical;
(3) method 3 comprising a step of: reacting a compound represented by general formula **II-C** with a compound **II-C1** as shown below, to obtain the diphenyl-like compound represented by general formula **II,** 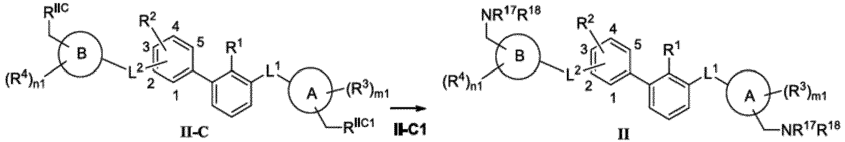 wherein the structure of the compound **II-C1** is as follows: 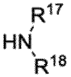 or an acid salt thereof, the definitions of ring A, ring B, L¹, L², R¹, R², R³, R⁴, R¹⁷ and R¹⁸ are the same as defined in claim 10, n1 is 0, 1 or 2, m1 is 0, 1 or 2; one of R^{IIC} and R^{IIC1} is 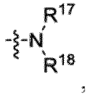 the other one is 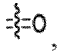 in this method, 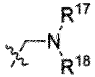 in ring A and ring B are identical or different;
(4) method 4 comprising a step of: reacting a compound represented by general formula **II-D** with a compound **II-D1** as shown below, to obtain the diphenyl-like compound represented by general formula **II,** 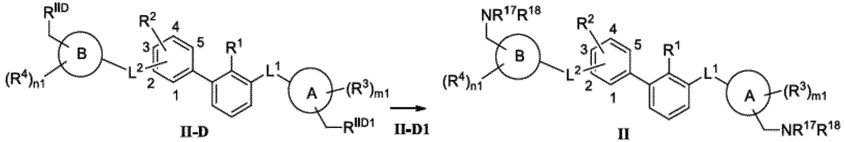 wherein the structure of the compound **II-D1** is as follows: 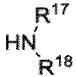 or an acid salt thereof, the definitions of ring A, ring B, L¹, L², R¹, R², R³, R⁴, R¹⁷ and R¹⁸ are the same as defined in claim 10, n1 is 0, 1 or 2, m1 is 0, 1 or 2; one of R^{IID} and R^{IID1} is 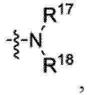 the other one is halogen, in this method, 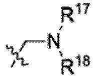 in ring A and ring B are identical or different;
(5) method 5 comprising a step of: deprotecting a compound represented by general formula **II-E** as shown below, to obtain the diphenyl-like compound represented by general formula **II,** R¹⁷ or R¹⁸ in the compound represented by general formula **II** containing carboxyl; 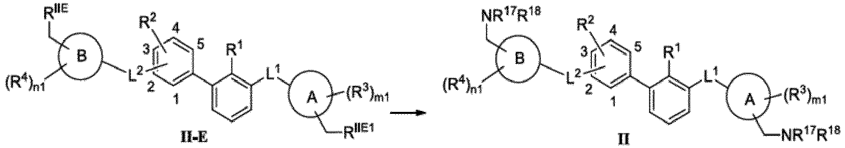 wherein the definitions of ring A, ring B, L¹, L², R¹, R², R³, R⁴, R¹⁷ and R¹⁸ are the same as defined in claim 10, nl is 0, 1 or 2, m1 is 0, 1 or 2, R^{IIE} and R^{IIE1} are 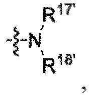 each R^{17'} and each R^{18'} are identical or different, and at least one contains carboxyl protecting group, R^{17'} and R^{18'} free of carboxyl protecting group are the same as the corresponding R¹⁷ and R¹⁸ in general formula **II** respectively; in this method, 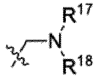 in ring A and ring B are identical or different.

12. A compound represented by general formula **II-A, II-B, II-C, II-D, II-E** and **II-F:** 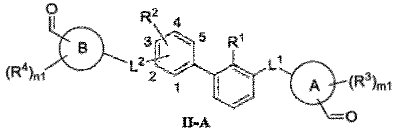 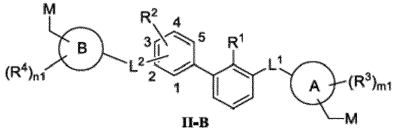 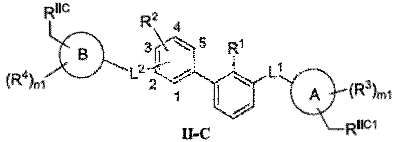 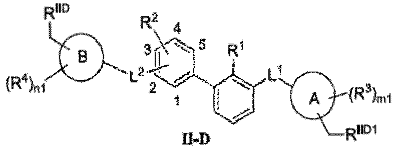 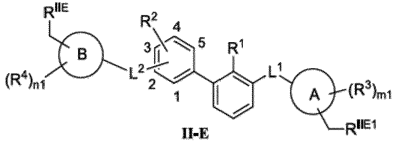 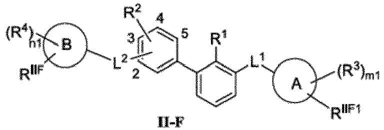 the definitions of ring A, ring B, L¹, L², R¹, R², R³, R⁴, M¹, N¹, R¹⁷ and R¹⁸ are the same as defined in at least one of claims 1 to 9, nl is 0, 1 or 2, m1 is 0, 1 or 2; M is halogen, one of R^{IIC} and R^{IIC1} is 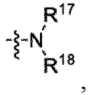 the other one is 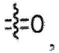 one of R^{IID} and R^{IID1} is 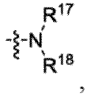 the other one is halogen, R^{IIE} and R^{IIE1} are 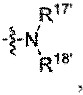 each R^{17'} and each R^{18'} are identical or different, and at least one contains carboxyl protecting group, R^{17'} and R^{18'} free of carboxyl protecting group are the same as the corresponding R¹⁷ and R¹⁸ in general formula **II** respectively; R^{IIF} is a group containing amino or carboxyl protecting group corresponding to M¹, R^{IIF1} is identical to N¹; or R^{IIF} is identical to M¹, R^{IIF1} is a group containing amino or carboxyl protecting group corresponding to N¹; or R^{IIF} is a group containing amino or carboxyl protecting group corresponding to M¹, R^{IIF1} is a group containing amino or carboxyl protecting group corresponding to N¹.

13. A compound as shown below: 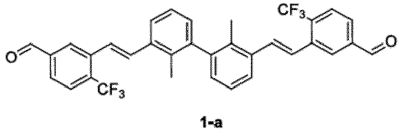 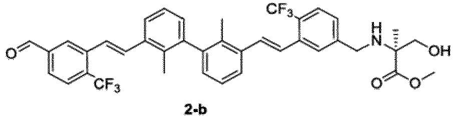 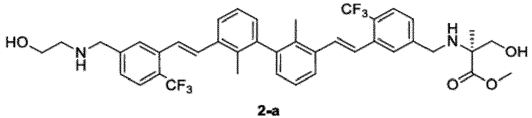 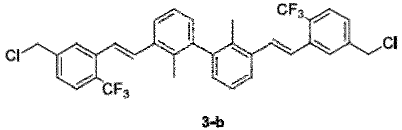 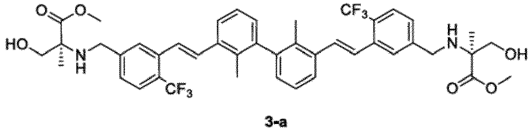 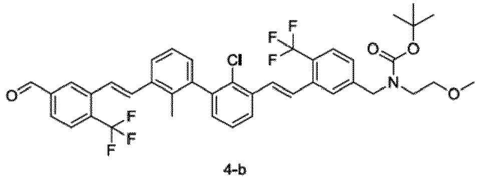 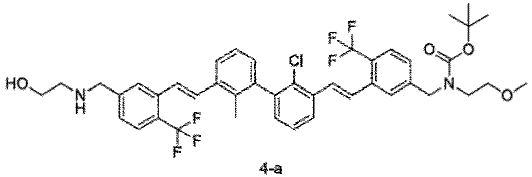 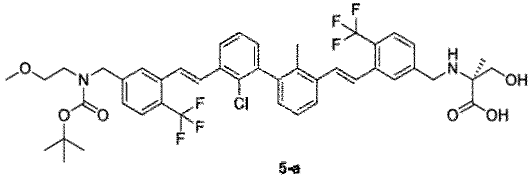 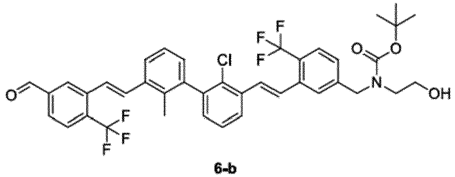 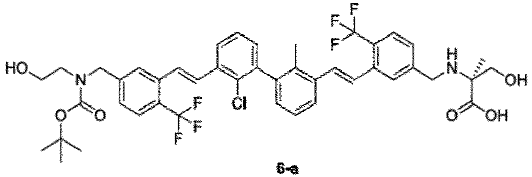 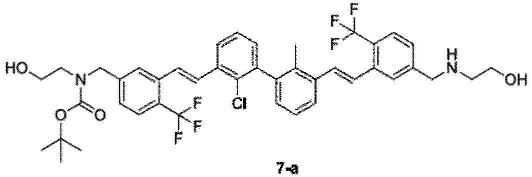 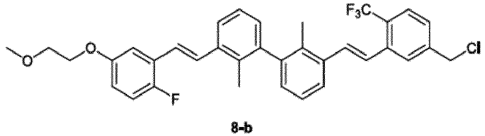 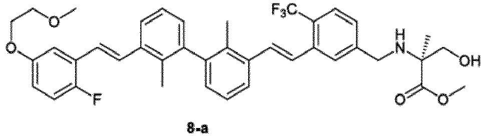 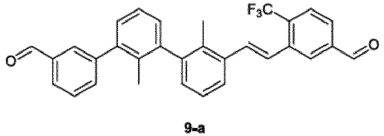 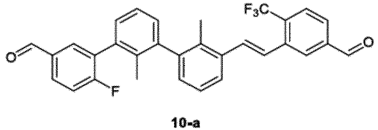 and

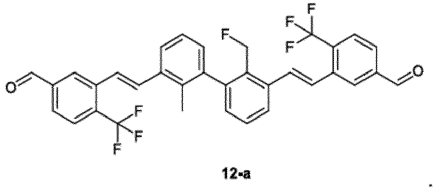

14. Use of the diphenyl-like compound of general formula **I,** a pharmaceutically acceptable salt, tautomer, mesomer, racemate, stereoisomer, or prodrug thereof as defined in at least one of claims 1 to 9 in the preparation of a PD-1 inhibitor and/or PD-L1 inhibitor.

15. Use of one or more of the diphenyl-like compound represented by general formula **I,** a pharmaceutically acceptable salt, tautomer, mesomer, racemate, stereoisomer, metabolite, metabolic precursor and prodrug thereof as defined in at least one of claims 1 to 9 in the preparation of a medicament for preventing, alleviating or treating cancer, infection, autoimmune disease or related diseases thereof, wherein the cancer is preferably one or more of lung cancer, esophageal cancer, gastric cancer, colorectal cancer, liver cancer, nasopharyngeal cancer, brain tumor, breast cancer, cervical cancer, blood cancer and bone cancer.

16. A pharmaceutical composition comprising a therapeutically and/or prophylactically effective amount of the diphenyl-like compound represented by general formula **I,** a pharmaceutically acceptable salt, tautomer, mesomer, racemate, stereoisomer, metabolite, metabolic precursor or prodrug thereof as defined in at least one of claims 1 to 9, and a pharmaceutically acceptable carrier and/or diluent.
